# EUROPEAN PATENT APPLICATION

(11) **EP 1 500 650 A1**
(43) Date of publication of application: **26.01.2005**
(21) Application number: 03723201.4
(22) Date of filing: 24.04.2003
(51) Int. Cl.: C07D 213/40, C07D 213/61, C07D 405/12, C07D 409/12, A01N 43/40

(54) **PYRIDINE COMPOUNDS OR SALTS THEREOF AND HERBICIDES CONTAINING THE SAME**

(30) Priority: 26.04.2002 JP 2002125603
(71) Applicant: ISHIHARA SANGYO KAISHA, LTD., Osaka-shi, Osaka 550-0002 (JP)
(72) Inventor: KOYANAGI, Toru, Ishihara Sangyo Kaisha, Ltd., Kusatsu-shi, Shiga 525-0025 (JP); KIKUGAWA, Hiroshi, Ishihara Sangyo Kaisha, Ltd., Kusatsu-shi, Shiga 525-0025 (JP); MIYASHITA, Seiko, Ishihara Sangyo Kaisha, Ltd., Kusatsu-shi, Shiga 525-0025 (JP); NAGAYAMA, Souichiro, Ishihara Sangyo Kaisha, Ltd., Kusatsu-shi, Shiga 525-0025 (JP); SANO, Makiko, Ishihara Sangyo Kaisha, Ltd., Kusatsu-shi, Shiga 525-0025 (JP); HISAMATSU, Akihiro, Ishihara Sangyo Kaisha, Ltd., Kusatsu-shi, Shiga 525-0025 (JP)
(74) Representative: Ward, David Ian
(86) International application number: PCT/JP2003/005284
(87) International publication number: WO 2003/091217

(57) **Abstract**

A pyridine compound represented by formula (I): 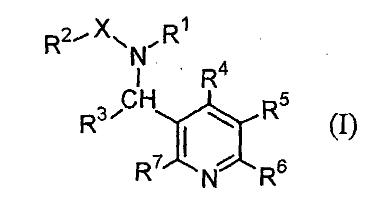 wherein R¹ represents hydrogen or alkyl which may be substituted; R² represents alkyl which may be substituted, alkenyl which may be substituted, alkynyl which may be substituted, cycloalkyl which may be substituted, *etc*.; R³ represents alkyl which may be substituted, *etc*.; R⁴ represents hydrogen, alkyl, haloalkyl, halogen, -OR⁸, or -SR⁸; R⁵, R⁶ and R⁷ each represents hydrogen, halogen, or alkyl; R⁸ represents alkyl which may be substituted, alkenyl which may be substituted, alkynyl which may be substituted, or cycloalkyl which may be substituted; and X represents CO, CS, or SO₂, or a salt thereof

## Description

### TECHNICAL FIELD

The present invention relates to novel pyridine compounds useful as active ingredients of herbicides.

### BACKGROUND ART

JP-A-9-328471 discloses 4-difluorohalogenoalkyl-3-substituted pyridine derivatives but does not disclose specific compound similar to pyridine compounds of formula (I) mentioned below. Moreover, WO 01/17975 discloses pyrimidine derivatives, which are however different in chemical structure from the pyridine compounds of formula (I) to be mentioned below.

### DISCLOSURE OF THE INVENTION

As a result of various investigations for finding out more excellent herbicides, the present inventors have found that a specific pyridine compound has excellent herbicidal activity, and accomplished the present invention.

Namely, the present invention relates to a pyridine compound represented by formula (I): 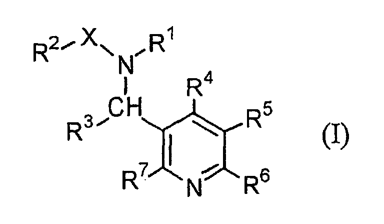 wherein R¹ represents hydrogen or alkyl which may be substituted; R² represents alkyl which may be substituted, alkenyl which may be substituted, alkynyl which may be substituted, cycloalkyl which may be substituted, cycloalkenyl which may be substituted, alkoxy which may be substituted, alkylthio which may be substituted, mono- or di-alkylamino which may be substituted, phenylamino which may be substituted, cyclic alkylamino which may be substituted, aryl which may be substituted, or a cyclic ether group which may be substituted; R³ represents alkyl which may be substituted, cycloalkyl which may be substituted, aryl which may be substituted, or heteroaryl which may be substituted; R⁴ represents hydrogen, alkyl, haloalkyl, halogen, -OR⁸, or -SR⁸; R⁵, R⁶ and R⁷ each represents hydrogen, halogen, or alkyl; R⁸ represents alkyl which may be substituted, alkenyl which may be substituted, alkynyl which may be substituted, or cycloalkyl which may be substituted; and X represents CO, CS, or SO₂, and wherein R⁴ is not chlorodifluoromethyl, bromodifluoromethyl, nor iododifluoromethyl, or a salt thereof, a process for producing the same, and a herbicide comprising the same.

Examples of the substituent for the alkyl which may be substituted, the alkenyl which may be substituted, the alkynyl which may be substituted, the cycloalkyl which may be substituted, the cycloalkenyl which may be substituted, the alkoxy which may be substituted, the alkylthio which may be substituted, the mono- or di-alkylamino which may be substituted, the cyclic alkylamino which may be substituted and the cyclic ether group which may be substituted, which are contained in R¹, R², R³ or R⁸ in the above formula (I), include halogen, alkyl, alkoxy, alkylthio, dialkylamino, trimethylsiliyl, cycloalkyl, cycloalkenyl, alkylcarbonyl, alkoxycarbonyl, cyclic ether, aryl which may be further substituted, aryloxy which may be further substituted, arylthio which may be further substituted, heteroaryl which may be further substituted, and the like. The number of substitution of these substituents may be 1 or 2 or more. When the number is 2 or more, the substituents are the same or different from each other. When a group is substituted with 2 or more alkyls, these alkyls may be combined with each other to form a carbon ring. Moreover, among these substituents, the aryl which may be further substituted, the aryloxy which may be further substituted, the arylthio which may be further substituted, and the heteroaryl which may be further substituted may be substituted with halogen, alkyl, haloalkyl, alkoxy, alkylthio, alkoxycarbonyl, nitro, cyano, trimethylsilyl, alkoxyimino, phenyl or the like.

Examples of the substituent for the phenylamino which may be substituted, the aryl which may be substituted, and the heteroaryl which may be substituted, which are contained in R² or R³ in the above formula (I), include halogen, alkyl, haloalkyl, alkoxy, alkylthio, alkoxycarbonyl, nitro, cyano, trimethylsilyl, alkoxyimino, phenyl, and the like. The number of substitution of these substituents may be I or 2 or more. When the number is 2 or more, the substituents are the same or different from each other. Moreover, among these substituents, the phenyl may be substituted with halogen, alkyl, haloalkyl, alkoxy, alkylthio, alkoxycarbonyl, nitro, cyano, alkoxyimino, trimethylsilyl or the like.

The above alkyl or alkyl moiety may be linear or branched one having 1 to 8 carbon atoms, and examples include methyl, ethyl, *n*-propyl, isopropyl, *n*-butyl, *sec*-butyl, *tert*-butyl, pentyl, hexyl, and the like.

The above alkenyl or alkenyl moiety may be linear or branched one having 2 to 8 carbon atoms, and examples include vinyl, propenyl, isopropenyl, butenyl, pentenyl, hexenyl, and the like.

The above alkynyl or alkynyl moiety may be linear or branched one having 2 to 8 carbon atoms, and examples include ethynyl, propynyl, butynyl, isobutynyl, pentynyl, hexynyl, and the like.

The above cycloalkyl may be one having 3 to 6 carbon atoms, and examples include cyclopropyl, cyclobutyl, cyclopentyl, cyclohexyl, and the like. In addition, it may be one condensed with a benzene ring such as indanyl.

The above cycloalkenyl may be one having 4 to 6 carbon atoms, and examples include cyclobutenyl, cyclopentenyl, cyclohexenyl, and the like. In addition, it may be one condensed with a benzene ring such as indenyl.

The above cyclic alkylamino may be one having 2 to 6 carbon atoms, and examples include aziridinyl, azetidinyl, pyrrolidinyl, piperidinyl, and the like.

The above aryl includes phenyl, naphthyl, and the like. In addition, it may be one condensed with a cycloalkane such as indanyl.

The above heteroaryl is 5-membered or 6-membered aryl containing 1 to 3 heteroatoms selected from oxygen, sulfur and nitrogen, or the aryl condensed with a benzene ring, and examples include thienyl, furanyl, thiazolyl, isothiazolyl, oxazolyl, isoxazolyl, imidazolyl, pyrazolyl, triazolyl, pyridyl, pyridazinyl, pyrimidinyl, pyrazinyl, benzothienyl, benzofuranyl, indolyl, benzothiazolyl, benzisothiazolyl, benzoxazolyl, benzisoxazolyl, benzimidazolyl, indazolyl, quinolyl, isoquinolyl, phthalazinyl, quinazolinyl, quinoxalinyl, and the like.

The above halogen includes each atom of fluorine, chlorine, bromine and iodine. The number of halogens contained in the above haloalkyl may be 1 or 2 or more. When the number is 2 or more, the halogens are the same or different from each other. Moreover, substitution position of the halogen(s) may be any position(s).

The above cyclic ether may be one having 2 to 4 carbon atoms, and examples include epoxy, tetrahydrofuryl, 1,3-dioxolanyl, 1,3-dioxanyl, and the like.

The pyridine compound of formula (I) is capable of forming a salt. The salt may be any one so long as it is agriculturally acceptable, and examples include alkali metal salts such as sodium salt and potassium salt; alkaline earth metal salts such as magnesium salt and calcium salt; ammonium salts such as dimethylamine salt and triethylamine salt; and the like.

Optical isomers or geometrical isomers may be present in the pyridine compounds of formula (I) and the present invention includes both of respective isomers and isomeric mixtures.

Among the pyridine compounds of formula (I), the compound wherein X is CO can be produced according to Process [A]: 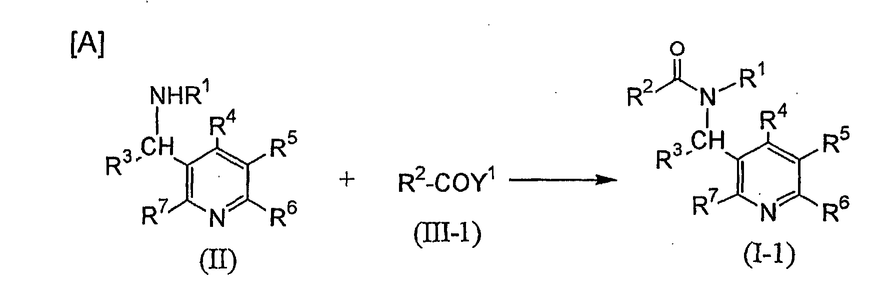 wherein R¹, R², R³, R⁴, R⁵, R⁶ and R⁷ have the same meanings as described above; and Y¹ represents OH, chlorine, bromine, or R²COO.

Namely, the pyridine compound of formula (I-1) can be produced by reacting an amine derivative of formula (II) with a carboxylic acid derivative of formula (III-1).

The above reaction is carried out in the presence of a base. As the base, any of inorganic bases and organic bases can be employed. The inorganic bases include alkali metal carbonates such as sodium carbonate and potassium carbonate; alkali metal hydroxides such as sodium hydroxide and potassium hydroxide; alkali metal hydrides such as sodium hydride and potassium hydride; and the like. The organic salts include organolithium compounds such as *n*-butyllithium and phenyllithium; tertiary amines such as triethylamine; pyridine; and the like. When Y¹ is OH, the above reaction is carried out in the presence of a condensing agent. The condensing agent includes *N*,*N'*-dicyclohexylcarbodiimide, *N*,*N'*-carbonyldiimidazole, and the like.

The above reaction is carried out in a solvent. The solvent may be any one so long as it is inert to the reaction, and examples include polar aprotic solvents such as acetonitrile, *N*,*N*-dimethylformamide and dimethylsulfoxide; ethers such as diethyl ether, tetrahydrofuran and dioxane; halogenated hydrocarbons such as dichloromethane and chloroform; aromatic hydrocarbons such as benzene and toluene; and the like. One or two or more of them can be optionally selected.

Moreover, the above reaction is carried out under inert gas atmosphere, if necessary. As the inert gas, for example, nitrogen, helium or argon is used.

The reaction temperature is usually from -20°C to 120°C, preferably from -10°C to 50°C. The reaction time is preferably from 30 minutes to 48 hours.

Among the pyridine compounds of formula (I), the compound wherein X is SO₂ can be produced according to Process [B]: 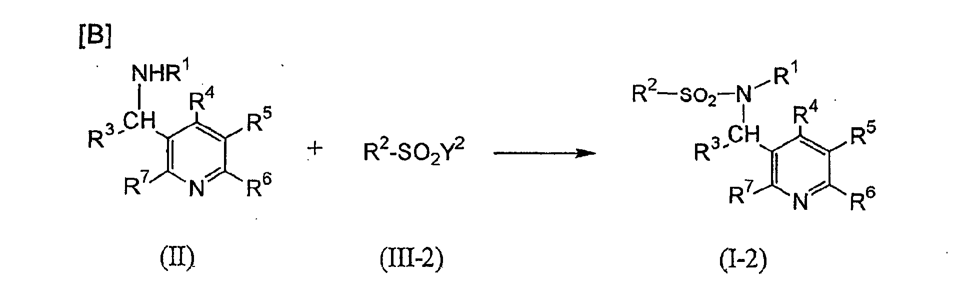 wherein R¹, R², R³, R⁴, R⁵, R⁶ and R⁷ have the same meanings as described above; and Y² represents chlorine, bromine, or R²SO₂O.

Namely, the pyridine compound of formula (1-2) can be produced by reacting the amine derivative of formula (II) with a sulfonic acid derivative of formula (III-2).

The above reaction is carried out in a similar manner to the above Process [A].

Among the pyridine compounds of formula (I), the compound wherein X is CS can be produced according to Process [C]: 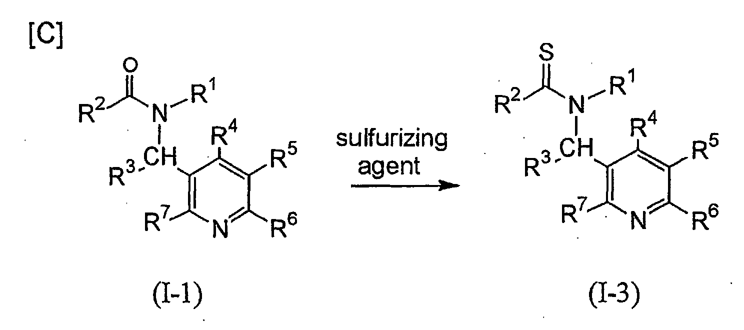 wherein R¹, R², R³, R⁴, R⁵, R⁶ and R⁷ have the same meanings as described above.

Namely, the pyridine compound of formula (I-3) can be produced by reacting the compound of formula (I-1) with a sulfurizing agent. Examples of the sulfurizing agent include phosphorus pentasulfide, Lawesson's reagent [2,4-bis(4-methoxyphenyl)-1,3-dithia-2,4-diphosphetane-2,4-disulfide], and the like.

The above reaction is carried out in a solvent. The solvent may be any one so long as it is inert to the reaction, and examples include aromatic hydrocarbons such as toluene and xylene; and the like.

The reaction temperature is usually from 80°C to 150°C, preferably from 110°C to 130°C. The reaction time is preferably from 30 minutes to 24 hours.

The amine derivatives of formula (II) include novel compounds, which can be, for example, produced by the following Processes [D] to [L]: 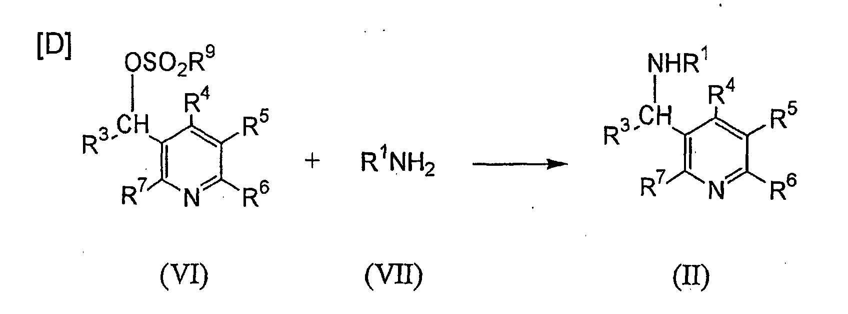 wherein R¹, R³, R⁴, R⁵, R⁶ and R⁷ have the same meanings as described above; and R⁹ represents C₁-C₆ alkyl or phenyl which may be substituted with C₁-C₆ alkyl.

Namely, the amine derivative of formula (II) can be produced by reacting a sulfonic acid ester of formula (VI) with an amine of formula (VII) according to process [D].

The above reaction is carried out in a solvent. The solvent may be any one so long as it is inert to the reaction, and examples include alcohols such as methanol and ethanol; ethers such as tetrahydrofuran and dioxane; polar aprotic solvents such as acetonitrile and *N*,*N*-dimethylformamide; water; mixtures of water and organic solvents listed here; and the like.

Moreover, the above reaction is carried out under inert gas atmosphere, if necessary. As the inert gas, for example, nitrogen, helium or argon is used.

The reaction temperature is usually from -20°C to 150°C, preferably from -10°C to 120°C. The reaction time is preferably from 30 minutes to 48 hours.

The sulfonic acid ester of formula (VI) can be produced by reacting an alcohol derivative of formula (IV) with a sulfonic acid chloride of formula (V) according to Process [E]: 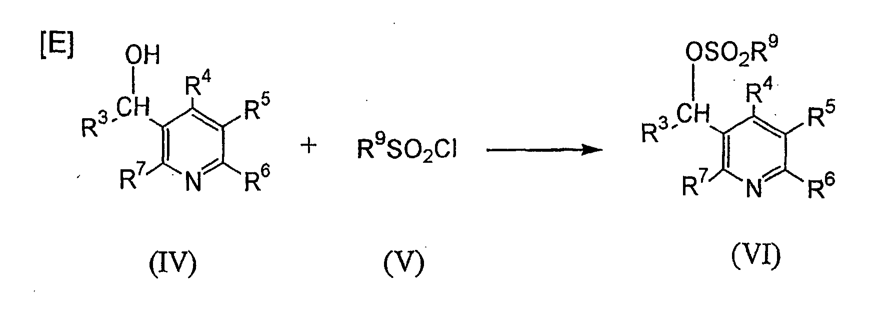 wherein R³, R⁴, R⁵, R⁶, R⁷ and R⁹ have the same meanings as described above.

The above reaction is carried out in the presence of a base. The base includes ones similar to those exemplified in the description of the above Process [A]. Preferably, organic bases such as pyridine and triethylamine are suitable. Moreover, pyridine can play two roles of a solvent and a base when used in large excess.

The above reaction is carried out in a solvent. The solvent may be any one so long as it is inert to the reaction and includes ones similar to those exemplified in the description of the above Process [A].

Moreover, the above reaction is carried out under inert gas atmosphere, if necessary. As the inert gas, for example, nitrogen, helium or argon is used.

The reaction temperature is usually from -78°C to 120°C, preferably from -20°C to 50°C. The reaction time is preferably from 30 minutes to 48 hours.

Among the amine derivatives of formula (II), the compound wherein R¹ is especially hydrogen can be produced according to Process [F]: 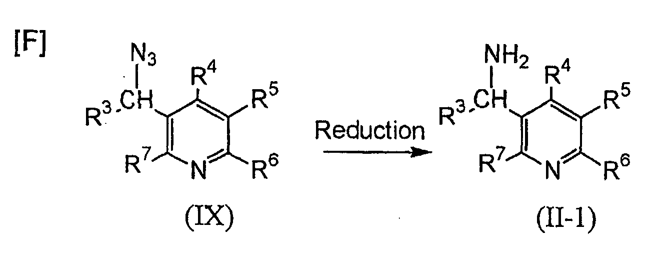 wherein R³, R⁴, R⁵, R⁶ and R⁷ have the same meanings as described above.

Namely, the amine derivative of formula (II-1) can be produced by reducing an azide compound of formula (IX).

As the reduction reaction, suitable is a hydrogenative catalytic reduction using a catalyst such as palladium-carbon, platinum-carbon or Raney nickel.

The above reaction is carried out in a solvent. The solvent may be any one so long as it is inert to the reaction, and examples include alcohols such as methanol and ethanol; cyclic ethers such as tetrahydrofuran and dioxane; and the like.

The reaction is carried out under hydrogen gas of atmospheric pressure to about 5 atm. The reaction temperature is usually from -20°C to 100°C, preferably from 0°C to 50°C. The reaction time is preferably from 30 minutes to 48 hours.

The above azide compound of formula (IX) can be produced by reacting the sulfonic acid ester derivative of formula (VI) with an alkali metal azide of formula (VIII) according to Process [G]: 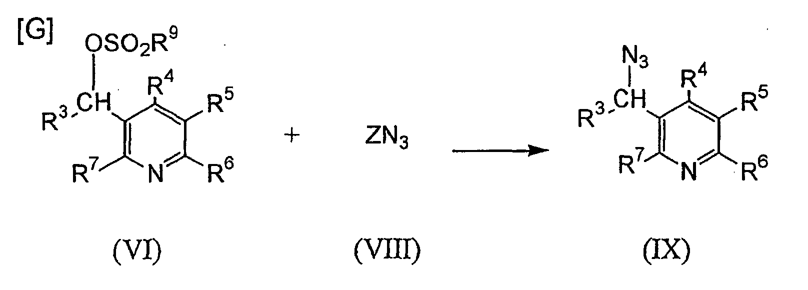 wherein R³, R⁴, R⁵, R⁶, R⁷ and R⁹ have the same meanings as described above; and Z represents an alkali metal such as sodium or potassium.

The above reaction is carried out in a solvent. The solvent may be any one so long as it is inert to the reaction, and examples include polar aprotic solvents such as acetonitrile, *N*,*N*-dimethylformamide and dimethylsulfoxide; cyclic ethers such as tetrahydrofuran and dioxane; halogenated hydrocarbons such as dichloromethane and chloroform; and the like.

Moreover, the above reaction is carried out under inert gas atmosphere, if necessary. As the inert gas, for example, nitrogen, helium or argon is used.

The reaction temperature is usually from -20°C to 150°C, preferably from -10°C to 120°C. The reaction time is preferably from 30 minutes to 24 hours.

Among the amine derivatives of formula (II), the compound wherein R¹ is especially alkyl which may be substituted can be produced according to Process [H]: 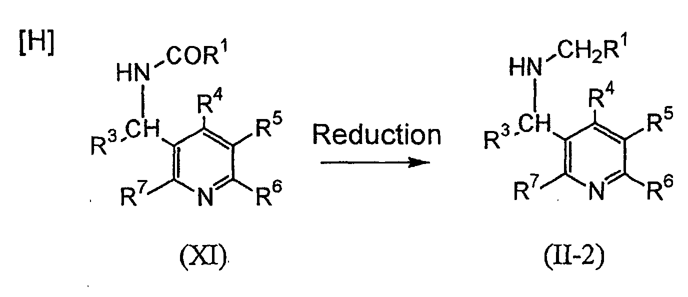 wherein R¹, R³, R⁴, R⁵, R⁶ and R⁷ have the same meanings as described above.

Namely, the amine derivative of formula (II-2) can be produced by reducing a carboxylic acid amide of formula (XI).

The reduction reaction is carried out using a reducing agent such as lithium aluminum hydride, sodium borohydride or a borane complex.

The above reaction is carried out in a solvent. The solvent may be any one so long as it is inert to the reaction, and examples include ethers such as tetrahydrofuran and diethyl ether; aromatic hydrocarbons such as toluene and benzene; and the like.

Moreover, the above reaction is carried out under inert gas atmosphere, if necessary. As the inert gas, for example, nitrogen, helium or argon is used.

The reaction temperature is usually from -20°C to 150°C, preferably from -10°C to 120°C. The reaction time is preferably from 30 minutes to 48 hours.

The above carboxylic acid amide of formula (XI) can be produced by reacting the amine derivative of formula (II-1) with a carboxylic acid derivative of formula (X) according to Process [I]: 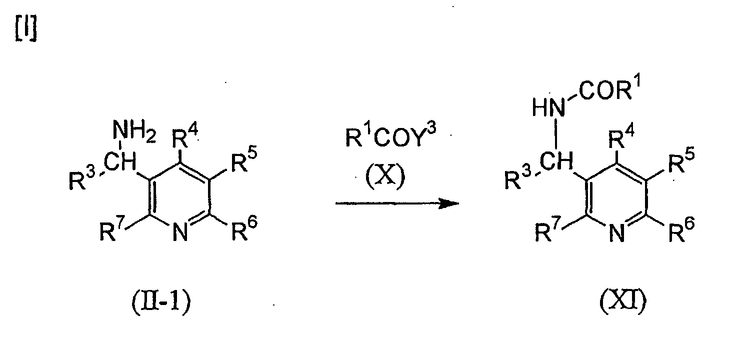 wherein R¹, R³, R⁴, R⁵, R⁶ and R⁷ have the same meanings as described above; and Y³ represents OH, chlorine, or R¹COO.

The above reaction is carried out in the presence of a base or a condensing agent. The base and condensing agent includes ones similar to those exemplified in the description of the above Process [A].

The above reaction is carried out in a solvent. The solvent may be any one so long as it is inert to the reaction and includes ones similar to those exemplified in the description of the above Process [A].

Moreover, the above reaction is carried out under inert gas atmosphere, if necessary. As the inert gas, for example, nitrogen, helium or argon is used.

The reaction temperature is usually from -20°C to 120°C, preferably from -10°C to 50°C. The reaction time is preferably from 30 minutes to 48 hours.

Furthermore, the amine derivatives of formula (II) can be produced according to Process [J]: 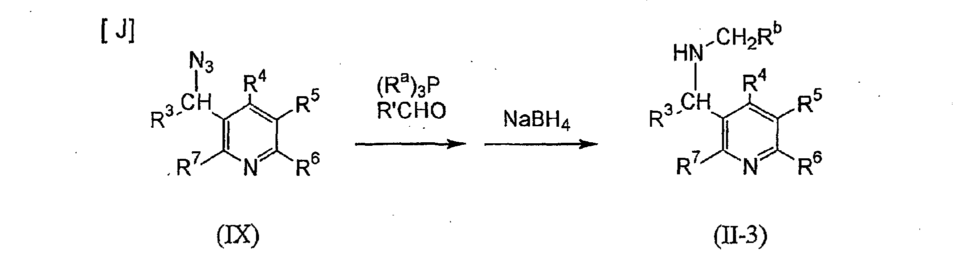 wherein R³, R⁴, R⁵, R⁶ and R⁷ have the same meanings as described above; R^{a} represents alkyl or phenyl; and R^{b} represents hydrogen or C₁-C₆ alkyl.

Namely, the amine derivative of formula (II-3) can be produced by reducing a phosphonium ylide, which is formed in a reaction solution by reacting the azide compound of formula (IX) with a phosphine such as trimethylphosphine or triphenylphosphine and an aldehyde, with sodium borohydride.

The above reaction is carried out in a solvent. The solvent may be any one so long as it is inert to the reaction, and examples include ethers such as tetrahydrofuran and diethyl ether; aromatic hydrocarbons such as toluene and benzene; halogenated hydrocarbons such as dichloromethane and chloroform; alcohols such as methanol and ethanol; and the like.

Moreover, the above reaction is carried out under inert gas atmosphere, if necessary. As the inert gas, for example, nitrogen, helium or argon is used.

The reaction temperature is usually from -20°C to 100°C, preferably from -10°C to 60°C. The reaction time is preferably from 30 minutes to 48 hours.

Furthermore, the amine derivatives of formula (II) can be produced according to Process [K]: 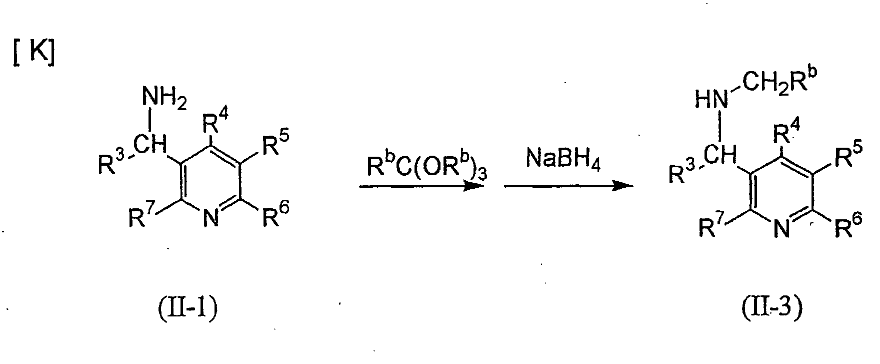 wherein R³, R⁴, R⁵, R⁶, R⁷ and R^{b} have the same meanings as described above.

Namely, the amine derivative of formula (II-3) can be produced by reducing alkoxyimine, which is formed by reacting the amine derivative of formula (II-1) with an orthoester such as methyl orthoformate or ethyl orthoacetate, with sodium borohydride.

The alkoxyimine is formed in the presence of an acid catalyst such as trifluoroacetic acid or acetic anhydride. No solvent is used in the reaction. Moreover, the reaction is carried out under inert gas atmosphere, if necessary. As the inert gas, for example, nitrogen, helium or argon is used.

The reaction temperature is usually from 50°C to 200°C, preferably from 80°C to 180°C. The reaction time is preferably from 30 minutes to 12 hours.

The reduction reaction of the alkoxyimine is carried out in a solvent. As the solvent, alcohol such as methanol or ethanol is used.

The reaction temperature is usually from -20°C to 120°C, preferably from 0°C to 80°C. The reaction time is preferably from 30 minutes to 12 hours.

Furthermore, the alcohol derivatives of formula (IV) can be produced according to Process [L]: 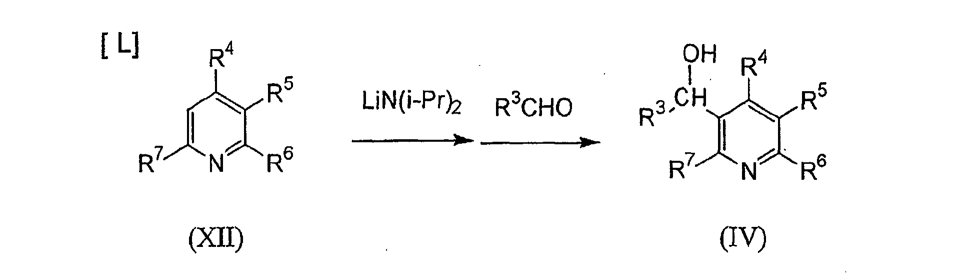 wherein R³, R⁴, R⁵, R⁶ and R⁷ have the same meanings as described above.

Namely, the alcohol derivative of formula (IV) can be obtained by reacting a pyridine derivative of formula (XII) with lithium diisopropylamide and subsequently reacting the product with an aldehyde.

The above reaction is carried out in a solvent. The solvent may be any one so long as it is inert to the reaction, and examples include ethers such as tetrahydrofuran and diethyl ether.

Moreover, the above reaction is carried out under inert gas atmosphere, if necessary. As the inert gas, for example, nitrogen, helium or argon is used.

The reaction temperature is usually from -100°C to 60°C, preferably from -85°C to 30°C. The reaction time is preferably from 30 minutes to 48 hours.

The pyridine compound of formula (I) can be also produced according to Processes [M] to [N]: 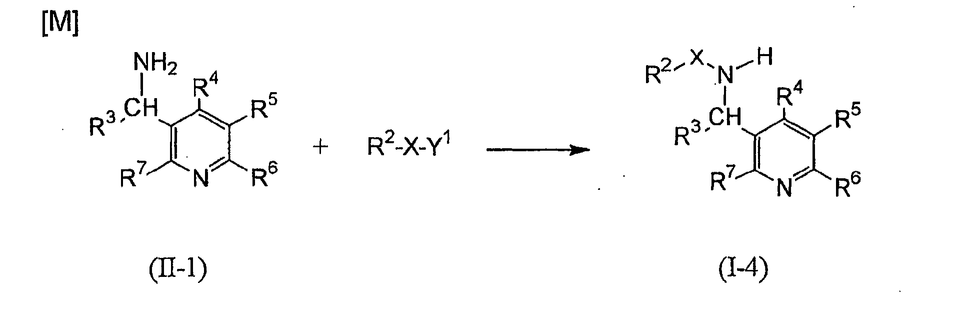 wherein R², R³, R⁴, R⁵, R⁶, R⁷, X and Y¹ have the same meanings as described above.

The above reaction is carried out in a similar manner to the above Process [A]. 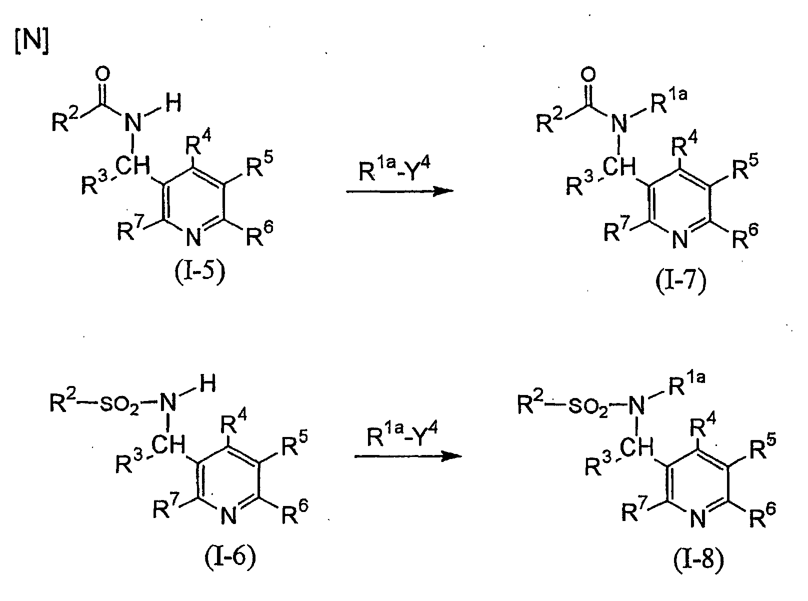 wherein R², R³, R⁴, R⁵, R⁶ and R⁷ have the same meanings as described above, R^{1a} represents alkyl which may be substituted; Y⁴ represents chlorine, bromine, iodine, -OSO₂R⁹, or -OSO₃R⁹; and R⁹ has the same meaning as described above.

The above reaction is carried out in the presence of a base. As the base, any of inorganic bases and organic bases can be employed. The inorganic bases include alkali metal hydroxides such as sodium hydroxide and potassium hydroxide; alkali metal hydrides such as sodium hydride and potassium hydride; and the like. The organic salts include organolithium compounds such as *n*-butyllithium and phenyllithium; tertiary amines such as triethylamine; pyridine; and the like.

The above reaction is carried out in a solvent. The solvent may be any one so long as it is inert to the reaction, and examples include polar aprotic solvents such as acetonitrile, *N*,*N*-dimethylformamide and dimethylsulfoxide; ethers such as diethyl ether, tetrahydrofuran and dioxane; aromatic hydrocarbons such as benzene and toluene; and the like. One or two or more of them can be optionally selected.

Moreover, the above reaction is carried out under inert gas atmosphere, if necessary. As the inert gas, for example, nitrogen, helium or argon is used.

The reaction temperature is usually from -100°C to 120°C, preferably from -80°C to 50°C. The reaction time is preferably from 30 minutes to 48 hours.

The pyridine compounds of formula (I) (hereinafter also referred to as "the compounds of the present invention") exhibit each an excellent herbicidal effect when used as active ingredients of herbicides. It can be widely applied to crop lands such as paddy field, upland farms, orchards, and mulberry; and non-crop lands such as forests, farm roads, playgrounds, and factory sites, and the applied method can be appropriately selected from soil treatment application, foliar application, water application, and the like.

The compound of the present invention can control noxious weeds, for example, grasses (or gramineae) such as barnyardgrass (*Echinochloa crus-galli* L.), carbgrass (*Digitaria sanguinalis* L.), greenfoxtail (*Setaria viridis* L.), giant foxtail (*Setaria faberi* Herrm.), goosegrass (*Eleusine indica* L.), wild oat (*Avena fatua L.),* johnsongrass (*Sorghum halepense* L.), quackgrass (*Agropyron repens L.*), alexandergrass (*Brachiaria plantaginea*), paragrass (*Panicum purpurascens*), sprangletop (*Leptochloa chinensis*), red sprangletop (*Leptochloa panicea*), annual bluegrass (*Poa annua* L.), and black grass (*Alopecurus myosuroides* Huds.); sedges (or Cyperaceae) such as rice flatsedge (*Cyperus iria* L.), purple nutsedge (*Cyperus rotundus* L.), yellow nutsedge (*Cyperus esculentus* L.), japanese bulrush (*Scirpus juncoides*), flatsedge (*Cyperus serotinus*), small-flower umbrellaplant (*Cyperus difformis*), slender spikerush (*Eleocharis acicularis*), and water chestnut (*Eleocharis kuroguwai*); alismataceae such as japanese ribbon waparo (*Sagittaria pygmaea*), arrow-head (*Sagittaria trifolia*), and narrowleaf waterplantain (*Alisma canaliculatum*); pontederiaceae such as monochoria (*Monochoria vaginalis*) and monochoria species (*Monochoria korsakowii*); scrophulariaceae such as false pimpernel (*Lindernia pyxidaria*) and abunome (*Dopatrium junceum*); lythraceae such as toothcup (*Rotala india*) and red stem (*Ammannia multiflora*); broadleaves such as velvetleaf (*Abutilon theophrasti* MEDIC.), tall morningglory (*Ipomoea purpurea* L.), common lambsquarters (*Chenopodium album* L.), prickly sida (*Sida spinosa* L.), common purslane (*Portulaca oleracea* L.), slender amaranth (*Amaranthus viridis* L.), redroot pigweed (*Amaranthus retroflexus* L.), sicklepod (*Cassia obtusifolia* L.), black nightshade (*Solanum nigrum* L.), pale smartweed (*Polygonum lapathifolium L.),* common chickweed (*Stellaria media* L.), common cocklebur (*Xanthium strumarium L.*), flexuous bittercress (*Cardamine flexuosa* WITH.), henbit (*Lamium amplexicaule L.*), common ragweed (*Ambrosia elatior* L.), catchweed (*Galium spurium* L.), field bindweed (*Calystegia arvensis* L.), jimsonweed (*Datura stramonium*), thistle (*Breea setosa* (BIEB.) KITAM.), and threeseeded copperleaf (*Acalypha australis* L.); and the like. Therefore, it can be effectively used for selectively controlling noxious weeds or nonselectively controlling noxious weeds in cultivation of useful crops such as corn (*Zea mays* L.), soybean (*Glycine max* Merr.), cotton (*Gossypium* spp.), wheat (*Triticum* spp.), rice (*Oryza sativa* L.), barley (*Hordeum vulgare* L.), oat (*Avena sativa* L.), sorgo (*Sorghum bicolor* Moench), rape (*Brassica napus* L.), sunflower (*Helianthus annuus* L.), sugar beet (*Beta vulgaris* L.), sugar cane (*Saccharum officinarum* L.), japanese lawngrass (*Zoysia japonica stend*), peanut (*Arachis hypogaea* L.), flax (*Linum usitatissimum* L.), tobacco (*Nicotiana tabacum* L.), and coffee (*Coffea* spp.). Particularly, the compound of the present invention is effectively used for selectively controlling noxious weeds in cultivation of corn, soybean, cotton, wheat, rice, rape, sunflower, sugar beet, sugar cane, japanese lawngrass, peanut, flax, tobacco, coffee, and the like, and among these, especially corn, soybean, wheat, rice, and the like.

The herbicidal composition comprising the compound of the present invention is usually formulated by mixing the compound with various agricultural adjuvants and used in the form of a formulation such as a dust, granules, water-dispersible granules, a wettable powder, a water-based suspension concentrate, an oilbased suspension concentrate, water soluble granules (or powder), an emulsifiable concentrate, tablets or capsules. However, so long as it is suitable for the purpose of the present invention, it may be formulated into any type of formulation which is commonly used in this field. Such agricultural adjuvants include solid carriers such as diatomaceous earth, slaked lime, calcium carbonate, talc, white carbon, kaoline, bentonite, a mixture of kaolinite and sericite, clay, sodium carbonate, sodium bicarbonate, mirabilite, zeolite and starch; solvents such as water, toluene, xylene, solvent naphtha, dioxane, acetone, isophorone, methyl isobutyl ketone, chlorobenzene, cyclohexane, dimethylsulfoxide, dimethylformamide, N-methyl-2-pyrrolidone, and alcohol; anionic surfactants and spreaders such as a salt of fatty acid, a benzoate, an alkylsulfosuccinate, a dialkylsulfosuccinate, a polycarboxylate, a salt of alkylsulfuric acid ester, an alkyl suflate, an alkylaryl sulfate, an alkyl diglycol ether sulfate, a salt of alcohol sulfuric acid ester, an alkyl sulfonate, an alkylaryl sulfonate, an aryl sulfonate, a lignin sulfonate, an alkyldiphenyl ether disuflonate, a polystyrene sulfonate, a salt of alkylphosphoric acid ester, an alkylaryl phosphate, a styrylaryl phosphate, a salt of polyoxyethylene alkyl ether sulfuric acid ester, a polyoxyethylene alkylaryl ether sulfate, a salt of polyoxyethylene alkyl ether sulfuric acid ester, a polyoxyethylene alkylaryl ether sulfuric acid ester, a polyoxyethylene alkyl ether phosphate, a salt of polyoxyethylene alkyl aryl phosphoric acid ester, and a salt of a condensate of naphthalene sulfonate with formalin; nonionic surfactants and spreaders such as a sorbitan fatty acid ester, a glycerin fatty acid ester, a fatty acid polyglyceride, a fatty acid alcohol polyglycol ether, acetylene glycol, acetylene alcohol, an oxyalkylene block polymer, a polyoxyethylene alkyl ether, a polyoxyethylene alkylaryl ether, a polyoxyethylene styrylaryl ether, a polyoxyethylene glycol alkyl ether, a polyoxyethylene fatty acid ester, a polyoxyethylene sorbitan fatty acid ester, a polyoxyethylene glycerin fatty acid ester, a polyoxyethylene hydrogenated castor oil, and a polyoxypropylene fatty acid ester; and vegetable and mineral oils such as olive oil, kapok oil, castor oil, palm oil, camellia oil, coconut oil, sesame oil, corn oil, rice bran oil, peanut oil, cottonseed oil, soybean oil, rapeseed oil, linseed oil, tung oil, and liquid paraffins. Such adjuvants may be selected for use among those known in this field, so long as the purpose of the present invention can thereby be accomplished. Further, various additives which are commonly used, such as a filler, a thickener, an anti-settling agent, an anti-freezing agent, a dispersion stabilizer, a phytotoxicity reducing agent, and an anti-mold agent, may also be employed. The weight ratio of the compound of the present invention to the various agricultural adjuvants is usually from 0.1 : 99.9 to 95 : 5, preferably from 0.2 : 99.8 to 85 : 15.

The dose of the herbicidal composition of the present invention cannot generally be defined, since it may vary depending upon the weather conditions, the soil conditions, the type of the formulation, the types of the weeds to be controlled, the season for the application, etc. However, it is usually applied so that the compound of the present invention would be applied in an amount of from 0.5 to 5,000 g/ha, preferably from 1 to 1,000 g/ha, and more preferably from 10 to 500 g/ha. The present invention covers such a method for controlling noxious weeds by application of such a herbicidal composition.

The herbicidal compositions of the present invention may be used in admixture with or in combination with other agricultural chemicals, fertilizers or phytotoxicity-reducing agents. In such a case, they may exhibit even better effects or activities. As other agricultural chemicals, herbicides, fungicides, antibiotics, plant hormones or insecticides may, for example, be mentioned. Especially with a mixed herbicidal composition having the compound of the present invention used in admixture with or in combination with one or more active ingredients of other herbicides, it is possible to improve the herbicidal activities, the season for the application and the range of applicable weed types. Further, the compound of the present invention and an active ingredient of other herbicide may be separately formulated, so that they may be mixed for use at the time of application, or both may be formulated together. The present invention covers such mixed herbicidal compositions.

The blend ratio of the compounds of the present invention with the active ingredients of other herbicides cannot generally be defined, since it varies depending upon the weather conditions, the soil conditions, the type of the formulation, the season for the application, the manner of the application, etc. However, one active ingredient of other herbicide may be incorporated usually in an amount of from 0.001 to 10,000 parts by weight, preferably from 0.01 to 1,000 parts by weight, per part by weight of the compound of the present invention. Further, the total dose of all of the active ingredients is usually from 0.1 to 10,000 g/ha, preferably from 0.2 to 5,000 g/ha, and more preferably from 10 to 3,000 g/ha. The present invention covers a method for controlling noxious weeds by application of such mixed herbicidal compositions.

Examples of active ingredients of other herbicides are shown below (common name; a part thereof is under application for ISO). Even if there is no description, salts, alkylesters, and the like of the compounds, if they exists, are included as a matter of course.
(1) Those which are believed to exhibit herbicidal effects by disturbing hormone activities of plants, for example, phenoxy compounds such as 2,4-D, 2,4-DP, MCPA, MCPB, MCPP, and naproanilide; aromatic carboxylic acid compounds such as 2,3,6-TBA, dicamba, dichlobenil, picloram, and clopyralid; others such as benazolin, quinclorac, quinmerac, diflufenzopyr, thiazopyr, etc.
(2) Those which are believed to exhibit herbicidal effects by inhibiting photosynthesis of plants, for example, urea compounds such as chlorotoluron, diuron, fluometuron, linuron, isoproturon, metobenzuron, and tebuthiuron; triazine compounds such as simazine, atrazine, atratone, simetryn, prometryn, dimethametryn, hexazinone, metribuzin, terbuthylazine, cyanazine, ametryn, cybutryne, triaziflam, and propazine; uracil compounds such as bromacil, lenacil, and terbacil; anilide compounds such as propanil and cypromid; carbamate compounds such as swep, desmedipham, and phenmedipham; 'hydroxybenzonitrile compounds such as bromoxynil, bromoxynil-octanoate, and ioxynil; others such as pyridate, bentazon, amicarbazone, etc.
(3) Quarternary ammonium salt compounds such as paraquat, diquat which are believed to be converted to free radicals by themselves to form active oxygen to thereby exhibit quick herbicidal effects.
(4) Those which are believed to exhibit herbicidal effects by inhibiting chlorophyllbiosynthesis of plants and abnormally accumulating a photosensitizing peroxide substance in the plant body, for example, diphenyl ether compuonds such as nitrofen, chlomethoxyfen, bifenox, acifluorfen-sodium, fomesafen, oxyfluorfen, lactofen, and ethoxyfen-ethyl; cyclic imide compounds such as chlorphthalim, flumioxazin, flumiclorac-pentyl, and fluthiacet-methyl; others such as oxadiargyl, oxadiazon, sulfentrazone, carfentrazone-ethyl, thidiazimin, pentoxazone, azafenidin, pyraflufen-ethyl, benzfendizone, butafenacil, metobenzuron, cinidon-ethyl, flupoxam, fluazolate, profluazol, pyrachlonil, *etc*.
(5) Those which are believed to exhibit herbicidal effects characterized by whitening activities by inhibiting chromogenesis of plants such as carotenoids, for example, pyridazinone comopunds such as norflurazon and metflurazon; pyrazole compunds such as pyrazolate, pyrazoxyfen and benzofenap; others such as amitrol, fluridone, flurtamone, diflufenican, methoxyphenone, clomazone, sulcotrione, mesotrione, isoxaflutole, difenzoquat, isoxachlortole, benzobicyclone, picolinofen, beflubutamid, *etc*.
(6) Those which exhibit herbicidal effects specifically to gramineous plants, for example, aryloxyphenoxypropionic acid compunds such as diclofop-methyl, flamprop-M-methyl, pyriphenop-sodium, fluazifop-butyl, haloxyfop-methyl, quizalofop-ethyl, cyhalofop-butyl, and fenoxaprop-ethyl; cyclohexanedione compounds such as alloxydim-sodium, clethodim, sethoxydim, tralkoxydim, butroxydim, tepraloxydim, caloxydim, and clefoxydim; *etc*.
(7) Those which are believed to exhibit herbicidal effects by inhibiting an amino acid biosynthesis of plants, for example, sulfonylurea compounds such as chlorimuron-ethyl, sulfometuron-methyl, primisulfuron-methyl, bensulfuron-methyl, chlorsulfuron, metsulfuron-methyl, cinosulfuron, pyrazosulfuron-ethyl, azimsulfuron, flazasulfuron, rimsulfuron, nicosulfuron, imazosulfuron, cyclosulfamuron, prosulfuron, flupyrsulfuron, trisulfuron-methyl, halosulfuron-methyl, thifensulfuron-methyl, ethoxysulfuron, oxasulfuron, ethametsulfuron, flupyrsulfuron, iodosulfuron, sulfosulfuron, tritosulfuron, foramsulfuron, and trifloxysulfuron; triazolopyrimidine compunds such as flumetsulam, metosulam, diclosulam, cloransulam-methyl, florasulam, metosulfam, and penoxsulam; imidazolynone compounds such as imazapyr, imazethapyr, imazaquin, imazamox, imazameth, imazamethabenz, and imazapic; pyrimidinylsalicylic acid compunds such as pyrithiobac-sodium, bispyribac-sodium, pyriminobac-methyl, pyribenzoxim, and pyriftalid; sulfonylaminocarbonyltriazolinone compunds such as flucarbazone and procarbazone-sodium; others such as glyphosate-ammonium, glyphosate-isopropylamine, glufosinate-ammonium, bialaphosl, *etc.*
(8) Those which are believed to exhibit herbicidal effects by inhibiting cell division of plant cell, for example, dinitroaniline compounds such as trifluralin, oryzalin, nitralin, pendimethalin, and ethalfluralin; organic phosphorus compounds such as amiprofos-methyl, butamifos, anilofos, and piperophos; phenylcarbamate compounds such as chlorpropham and barban; cumylamine compounds such as daimuron, cumyluron, and bromobutide; others such as asulam, dithiopyr, thiazopyr, *etc.*
(9) Those which are believed to exhibit herbicidal effects by inhibiting protein biosynthesis or lipid biosynthesis of plants, for example, thiocarbamate compounds such as EPTC, butylate, molinate, dimepiperate, fluazolate, esprocarb, thiobencarb, pyributicarb, and triallate; chloroacetamide compounds such as alachlor, butachlor, pretilachlor, metolachlor, S-metolachlor, thenylchlor, pethoxamid, dimethenamid, acetochlor, and propachlor; others such as etobenzanid, mefenacet, flufenacet, tridiphane, cafenstrole, fentrazamide, oxaziclomefone, indanofan, *etc.*
(10) Those which are believed to exhibit herbicidal effects by infection on the plant bodies, for example, *Xanthomonas campestris*, *Epicoccosurus nematosurus*, *Exserohilum monoseras*, *Drechsrela monoceras*, *etc*.

In addition, as shown in Test Examples to be mentioned below, the compounds of the present invention include those having safety toward crops such as corn, soybean, wheat and rice and exhibiting selectivity allowing satisfactory control of weeds. However, when the compounds of the present invention are used in the cultivation of the above crops, mixed use or combined use thereof with one or two or more of the following compounds among the active ingredient compounds of other herbicides mentioned above sometimes affords a synergistic effect.

Cultivation of rice: 2,4-D, MCPA, MCPB, naproanilide, dichlobenil, quinclorac, simetryn, prometryn, dimethametryn, propanil, swep, bentazon, nitrofen, chlomethoxyfen, bifenox, oxadiargyl, oxadiazon, sulfentrazone, carfentrazone-ethyl, pentoxazone, pyrazolate, pyrazoxyfen, benzofenap, methoxyphenone, cyhalofop-butyl, fenoxaprop-ethyl, bensulfuron-methyl, cinosulfuron, pyrazosulfuron-ethyl, azimsulfuron, imazosulfuron, cyclosulfamuron, ethoxysulfuron, penoxsulam, bispyribac-sodium, pyriminobac-methyl, anilofos, piperophos, daimuron, cumyluron, bromobutide, dithiopyr, molinate, dimepiperate, esprocarb, thiobencarb, pyributicarb, thenylchlor, pretilachlor, butachlor, etobenzanid, mefenacet, flufenacet, cafenstrole, fentrazamide, oxaziclomefone, indanofan, benzobicyclone, pyribenzoxim, triaziflam, clefoxydim, pyrachlonil, pyriftalid.

Cultivation of soybean: 2,4-D, linuron, metribuzin, cyanazine, bentazon, paraquat, acifluorfen-sodium, fomesafen, lactofen, ethoxyfen-ethyl, flumiclorac-pentyl, flumioxazin, fluthiacet-methyl, sulfentrazone, norflurazon, clomazone, fluazifop-butyl, quizalofop-ethyl, fenoxaprop-ethyl, haloxyfop-methyl, clethodim, sethoxydim, butroxydim, tepraloxydim, chlorimuron-ethyl, thifensulfuron-methyl, oxasulfuron, flumetsulam, cloransulam-methyl, diclosulam, imazapyr, imazethapyr, imazaquin, imazamox, imazapic, trifluralin, pendimethalin, ethalfluralin, alachlor, pethoxamid, metolachlor, S -metolachlor, acetochlor, dimethenamid, flufenacet.

Cultivation of corn: 2,4-D, MCPA, dicamba, clopyralid, benazolin, diflufenzopyr, diuron, linuron, metobenzuron, simazine, atrazine, atratone, metribuzin, terbuthylazine, cyanazine, ametryn, cypromid, bromoxynil, bromoxynil-octanoate, pyridate, bentazon, paraquat, oxyfluorfen, flumiclorac-pentyl, fluthiacet-methyl, fluridone, sulcotrione, mesotrione, isoxaflutole, carfentrazone-ethyl, primisulfuron-methyl, rimsulfuron, nicosulfuron, prosulfuron, halosulfuron-methyl, thifensulfuron-methyl, flumetsulam, metosulam, imazethapyr, glyphosate-ammonium, glyphosate-isopropylamine, glufosinate-ammonium, trifluralin, pendimethalin, EPTC, butylate, alachlor, pethoxamid, metolachlor, S-metolachlor, acetochlor, propachlor, dimethenamid, tridiphane, florasulam, metobenzuron, metosulfam, oxasulfuron, tepraloxydim.

Cultivation of wheat: MCPB, dichlobenil, quinmerac, chlorotoluron, linuron, isoproturon, prometryn, bromoxynil, bromoxynil-octanoate, pyridate, bifenox, carfentrazone-ethyl, thidiazimin, pyraflufen-ethyl, flurtamone, diflufenican, sulcotrione, diclofop-methyl, flamprop-M-methyl, tralkoxydim, chlorsulfuron, metsulfuron-methyl, prosulfuron, halosulfuron-methyl, flumetsulam, metosulam, pendimethalin, barban, imazamethabenz, cinidon-ethyl, ethoxyfen-ethyl, florasulam, fluazolate, flupoxam, iodosulfuron, metosulfam, pyribenzoxim, sulfosulfuron, tralkoxydim, procarbazone-sodium, picolinofen, cyclosulfamuron, ethoxysulfuron, imazamox.

Preferred embodiments of the present invention are as follows. However, the present invention is not limited thereto.
(1) A pyridine compound of the above formula (I), wherein the above substituent for the alkyl which may be substituted, the alkenyl which may be substituted, the alkynyl which may be substituted, the cycloalkyl which may be substituted, cycloalkenyl which may be substituted, the alkoxy which may be substituted, the alkylthio which may be substituted, the mono- or di-alkylamino which may be substituted, the cyclic alkylamino which may be substituted and the cyclic ether group which may be substituted is at least one selected from the group consisting of halogen, alkyl, alkoxy, alkylthio, dialkylamino, trimethylsiliyl, cycloalkyl, cycloalkenyl, alkylcarbonyl, alkoxycarbonyl, cyclic ether, aryl which may be further substituted, aryloxy which may be further substituted, arylthio which may be further substituted, and heteroaryl which may be further substituted, and wherein the above substituent for the phenylamino which may be substituted, aryl which may be substituted and heteroaryl which may be substituted is at least one selected from the group consisting of halogen, alkyl, haloalkyl, alkoxy, alkylthio, alkoxycarbonyl, nitro, cyano, trimethylsilyl, alkoxyimino, and phenyl which may be further substituted, or a salt thereof; a herbicide comprising it as an active ingredient; and a process for controlling noxious weeds by applying an effective amount thereof.
(2) A pyridine compound of the above formula (I), wherein the above cyclic alkylamino is aziridinyl, azetidinyl, pyrrolidinyl or piperidinyl; the aryl is phenyl, naphthyl, or indanyl; the cyclic ether is epoxy, tetrahydrofuryl, 1,3-dioxolanyl, or 1,3-dioxanyl; the heteroaryl is a 5-membered or 6-membered aryl containing 1 to 3 heteroatoms selected from oxygen, sulfur and nitrogen, or the aryl condensed with a benzene ring, or a salt thereof; a herbicide comprising it as an active ingredient; and a process for controlling noxious weeds by applying an effective amount thereof.
(3) A pyridine compound of the above formula (I), wherein the above heteroaryl is thienyl, furanyl, thiazolyl, isothiazolyl, oxazolyl, isoxazolyl, imidazolyl, pyrazolyl, triazolyl, pyridyl, pyridazinyl, pyrimidinyl, pyrazinyl, benzothienyl, benzofuranyl, indolyl, benzothiazolyl, benzisothiazolyl, benzoxazolyl, benzisoxazolyl, benzimidazolyl, indazolyl, quinolyl, isoquinolyl, phthalazinyl, quinazolinyl, or quinoxalinyl or a salt thereof; a herbicide comprising it as an active ingredient; and a process for controlling noxious weeds by applying an effective amount thereof.
(4) A pyridine compound of the above formula (I), wherein R⁴ is hydrogen, alkyl, trifluoromethyl, halogen, -OR⁸, or -SR⁸, or a salt thereof; a herbicide comprising it as an active ingredient; and a process for controlling noxious weeds by applying an effective amount thereof.
(5) A pyridine compound of the above formula (I), wherein the above formula (I) is formula (Ia): 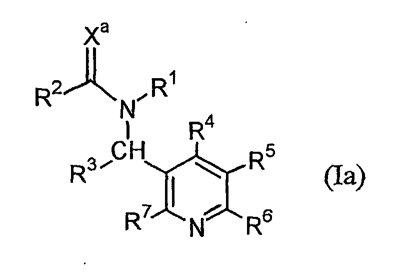
wherein R¹ represents hydrogen or alkyl which may be substituted; R² represents alkyl which may be substituted, alkenyl which may be substituted, alkynyl which may be substituted, cycloalkyl which may be substituted, alkoxy which may be substituted, alkylthio which may be substituted, mono- or di-alkylamino which may be substituted, phenylamino which may be substituted, or cyclic alkylamino which may be substituted; R³ represents alkyl which may be substituted, cycloalkyl which may be substituted, aryl which may be substituted, or heteroaryl which may be substituted; R⁴ represents alkyl, haloalkyl, -OR⁸, or -SR⁸; R⁵, R⁶ and R⁷ each represents hydrogen, halogen, or alkyl; R⁸ represents alkyl which may be substituted, alkenyl which may be substituted, alkynyl which may be substituted, or cycloalkyl which may be substituted; and X^{a} represents oxygen or sulfur, wherein R⁴ is not chlorodifluoromethyl, bromodifluoromethyl, or iododifluoromethyl, or a salt thereof; a herbicide comprising it as an active ingredient; and a process for controlling noxious weeds by applying an effective amount thereof.

### Best Mode for Carrying Out the Invention

Synthetic methods of the compounds of the present invention are explained below with reference to Examples. Also, synthetic methods of synthetic intermediates of the compounds of the present invention are also described.

### Example 1: Synthesis 'of N-methyl-N-[2-methyl-1-(4-trifluoromethylpyridin-3-yl)propyl]phenylacetamide (Compound No. I-1)

To 5 ml of acetonitrile were added 0.184 g of 1-methylamino-2-methyl-1-(4-trifluoromethylpyridin-3-yl)propane and 0.115 g of potassium carbonate, followed by stirring at room temperature for 30 minutes to form a solution. Thereto was added 0.129 g of phenylacetyl chloride, followed by continuously stirring at room temperature for another 14 hours. After completion of the reaction, the reaction solution was extracted with added 50 ml of water and 100 ml of ethyl acetate. The resulting organic layer was washed with water and saturated brine and then dried over anhydrous magnesium sulfate. The ethyl acetate was removed by evaporation under reduced pressure and the residue was purified by silica gel column chromatography (developing solvent: *n*-hexane/ethyl acetate = 1/1) to obtain 0.15 g (yield 54%) of the desired product as colorless crystals (melting point 71.2°C).

### Example 2: Synthesis of N-ethyl-N-[2-methyl-1-(4-trifluoromethylpyridin-3-yl)propyl]2,6-difluorophenylacetamide (Compound No. I-30)

To 20 ml of acetonitrile were added 0.20 g of 1-ethylamino-2-methyl-1-(4-trifluoromethylpyridin-3-yl)propane and 0.15 g of potassium carbonate, followed by stirring at room temperature for 30 minutes to form a solution. Thereto was added 0.20 g of 2,6-difluorophenylacetyl chloride, followed by continuously stirring at room temperature for another 9 hours. After completion of the reaction, the reaction solution was extracted with added 50 ml of water and 100 ml of ethyl acetate. The resulting organic layer was washed with water and saturated brine and then dried over anhydrous magnesium sulfate. The ethyl acetate was removed by evaporation under reduced pressure and the residue was purified by silica gel column chromatography (developing solvent: *n*-hexane/ethyl acetate = 1/1) to obtain 0.16 g (yield 49%) of the desired product as colorless crystals (melting point 121.9°C).

### Example 3: Synthesis of N-methyl-N-[2-methyl-1-(4-trifluoromethylpyridin-3-yl)propyl]phenylsulfonamide (Compound No. I-117)

To 10 ml of acetonitrile were added 0.226 g of 1-methylamino-2-methyl-1-(4-trifluoromethylpyridin-3-yl)propane and 0.18 g of potassium carbonate, followed by stirring at room temperature for 30 minutes to form a solution. Thereto was added 0.22 g of benzenesulfonyl chloride, followed by continuously stirring at room temperature for another 12 hours. After completion of the reaction, the reaction solution was extracted with added 50 ml of water and 100 ml of ethyl acetate. The resulting organic layer was washed with water and saturated brine and then dried over anhydrous magnesium sulfate. The ethyl acetate was removed by evaporation under reduced pressure and the residue was purified by silica gel column chromatography (developing solvent: *n*-hexane/ethyl acetate = 1/1) to obtain 0.18 g (yield 50%) of the desired product as an oily substance.

### Example 4: Synthesis of N-methyl-N-[2-methyl-1-(4-trifluoromethylpyridin-3-yl)propyl] 2-phenoxypropionamide (Compound No. I-119)

To 10 ml of acetonitrile were added 0.23 g of 1-methylamino-2-methyl-1-(4-trifluoromethylpyridin-3-yl)propane and 0.18 g of potassium carbonate, followed by stirring at room temperature for 30 minutes to form a solution. Thereto was added 0.24 g of 2-phenoxypropionyl chloride, followed by continuously stirring at room temperature for another 10 hours. After completion of the reaction, the reaction solution was extracted with added 50 ml of water and 100 ml of ethyl acetate. The resulting organic layer was washed with water and saturated brine and then dried over anhydrous magnesium sulfate. The ethyl acetate was removed by evaporation under reduced pressure and the residue was purified by silica gel column chromatography (developing solvent: *n*-hexane/ethyl acetate = 3/2) to obtain 0.156 g (yield 41 %) of the amorphous desired product.

### Example 5: Synthesis of N-methyl-N-[2-methyl-1-(4-trifluoromethylpyridin-3-yl)propyl] 3-phenoxypropenamide (Compound No. I-166)

To a tetrahydrofuran (10 ml) solution of 0.30 g of 1-methylamino-2-methyl-1-(4-trifluoromethylpyridin-3-yl)propane were added dropwise 0.36 g of cinnamoyl chloride and 0.29 g of triethylamine at 0°C. After stirring at room temperature for 7 hours, water was added to the reaction solution, followed by extraction with ethyl acetate. The resulting organic layer was washed with water and saturated brine and then dried over anhydrous magnesium sulfate. The ethyl acetate was removed by evaporation under reduced pressure and the residue was purified by silica gel column chromatography (developing solvent: *n*-hexane/ethyl acetate = 1/1) to obtain 73 mg (yield 15%) of the oily desired product.

### Example 6: Synthesis of N-methyl-N-[2-methyl-1-(4-trifluoromethylpyridin-3-yl)propyl]butanamide (Compound No. I-189)

To a tetrahydrofuran (7 ml) solution of 0.17 g of 1-methylamino-2-methyl-1-(4-trifluoromethylpyridin-3-yl)propane were added dropwise 0.11 g of butyryl chloride and 0.12 g of triethylamine at 0°C. After stirring at room temperature for 18 hours, the reaction solution was extracted with added 50 ml of water and 100 ml of ethyl acetate. The resulting organic layer was washed with water and saturated saline solution and then dried over anhydrous magnesium sulfate. The ethyl acetate was removed by evaporation under reduced pressure and the residue was purified by silica gel column chromatography (developing solvent: *n*-hexane/ethyl acetate = 1/2) to obtain 0.19 g (yield 86%) of the oily desired product.

### Example 7: Synthesis of 1-(2-chloro-4-trifluoromethylpyridin-3-yl)-2-methyl-1-propanol

A tetrahydrofuran (200 ml) solution of 21.7 g of diisopropylamine was cooled to -70°C and 114 ml of *n*-butyllithium was gradually added dropwise thereto. After continuously stirring at the same temperature for 15 minutes, 30 g of 2-chloro-4-trifluoromethylpyridine was added dropwise, followed by stirring for 1 hour. Then, a tetrahydrofuran (30 ml) solution of 11.9 g of isobutyraldehyde was added dropwise, followed by continuously stirring at the same temperature for 2 hours. After completion of the reaction, the reaction solution was extracted with added an aqueous ammonium chloride solution and ethyl acetate. The organic layer was washed with water and saturated brine and then dried over anhydrous magnesium sulfate. The ethyl acetate was removed by evaporation under reduced pressure and the residue was purified by silica gel column chromatography (developing solvent: *n*-hexane/ethyl acetate = 4/1) to obtain 33 g (yield 79%) of the desired product as an oily substance.

### Example 8: Synthesis of 1-(4-trifluoromethylpyridin-3-yl)-2-methyl-1-propanol

In 300 ml of methanol were dissolved 33 g of 1-(2-chloro-4-trifluoromethylpyridin-3-yl)-2-methyl-1-propanol obtained in the above Example 7 and 23.5 g of triethylamine, and thereto was added 3.0 g of a 10% palladium-carbon catalyst (50% hydrate). The suspension was stirred at room temperature for 8 hours under hydrogen gas atmosphere of 2 atm. After separation of insoluble matter by filtration, the solvent was removed by evaporation under reduced pressure and the residue was extracted with added ethyl acetate and water. The organic layer was washed with water and saturated saline solution and then dried over anhydrous magnesium sulfate. The ethyl acetate was removed by evaporation under reduced pressure and the residue was purified by silica gel column chromatography (developing solvent: n-hexane/ethyl acetate = 1/1) to obtain 22 g (yield 77%) of the desired product as a light yellow liquid.

### Example 9: Synthesis of 2-methyl-1-(4-trifluoromethylpyridin-3-yl)-1-propyl methanesulfonate

In 50 ml of pyridine was dissolved 5 g of 1-(4-trifluoromethylpyridin-3-yl)-2-methyl-1-propanol obtained in the above Example 8, and then 3.1 g of methanesulfonyl chloride was added dropwise at room temperature. After stirring of the solution at room temperature for 8 hours, the pyridine was removed by evaporation under reduced pressure and the residue was extracted with added ethyl acetate and water. The organic layer was washed with water and saturated saline solution and then dried over anhydrous magnesium sulfate. The ethyl acetate was removed by evaporation under reduced pressure and the residue was purified by silica gel column chromatography (developing solvent: *n*-hexane/ethyl acetate = 1/1) to obtain 5.8 g (yield 86%) of the desired product as a white amorphous solid.

### Example 10: Synthesis of 1-methylamino-2-methyl-1-(4-trifluoromethylpyridin-3-yl)-propane

Two grams of 2-methyl-1-(4-trifluoromethylpyridin-3-yl)-1-propyl methanesulfonate obtained in the above Example 9 was reacted with 30 ml of a 40% methylamine methanol solution in an autoclave at 100°C for 8 hours. After removal of the solvent by evaporation, the residue was purified by silica gel column chromatography (developing solvent: ethyl acetate/methanol = 9/1) to obtain 0.2 g (yield 13%) of the desired product as a light yellow liquid.

### Example 11: Synthesis of 1-azido-2-methyl-1-(4-trifluoromethylpyridin-3-yl)-propane

To 100 ml of a dimethylsulfoxide solution of 10 g of 2-methyl-1-(4-trifluoromethylpyridin-3-yl)-1-propyl methanesulfonate synthesized according to the above Example 9 was added 4.4 g of sodium azide at room temperature, followed by heating to 70°C and continuously stirring for 4 hours. The reaction liquid was cooled to room temperature and then extracted with added water and ether. The organic layer was washed with water and saturated brine and then dried over anhydrous magnesium sulfate. The solvent was removed by evaporation to obtain 8 g (yield 97%) of the desired product as a yellow liquid.

### Example 12: Synthesis of 1-methylamino-2-methyl-1-(4-trifluoromethylpyridin-3-yl)-propane

To 80 ml of a dichloromethane solution of 3g of 1-azido-2-methyl-1-(4-trifluoromethylpyridin-3-yl)-propane obtained in Example 11 was added dropwise 16.6 ml of a 1M tetrahydrofuran solution of trimethylphosphine at room temperature, followed by stirring for 2 hours. Then, 1.87 g of paraformaldehyde was gradually added and the whole was stirred for 8 hours. The reaction solution was cooled to 0°C and 50 ml of an ethanol solution of 2.33 g of sodium borohydride was added dropwise, followed by stirring at the same temperature for 1 hour. The reaction solution was neutralized with an aqueous saturated sodium hydrogen carbonate solution and then extracted with added dichloromethane. The organic layer was washed with water and saturated brine and then dried over anhydrous magnesium sulfate. After removal of the solvent by evaporation, the residue was purified by silica gel column chromatography (developing solvent: ethyl acetate) to obtain 1.4 g (yield 49%) of the desired product as a light yellow liquid.

### Example 13: Synthesis of 1-amino-2-methyl-1-(4-trifluoromethylpyridin-3-yl)-propane

In 18 ml of methanol was dissolved 1.38 g of 1-azido-2-methyl-1-(4-trifluoromethylpyridin-3-yl)-propane obtained in the above Example 11, and thereto was added 0.36 g of a 5% palladium-carbon catalyst. The suspension was stirred at room temperature for 4 hours under hydrogen gas atmosphere of 2 atm. After separation of insoluble matter by filtration, the solvent was removed by evaporation under reduced pressure and the residue was purified by silica gel column chromatography (developing solvent: *n*-hexane/ethyl acetate = 1/1) to obtain 1.0 g (yield 81%) of the desired product as a solid.

### Example 14: Synthesis of 1-methylamino-2-methyl-1-(4-trifluoromethylpyridin-3-yl)-propane

To 0.5 g of 1-amino-2-methyl-1-(4-trifluoromethylpyridin-3-yl)-propane obtained in the above Example 13 were added 5.5 ml of triethyl orthoformate and one drop of trifluoroacetic acid, followed by heating at 150°C for 4 hours. The reaction solution was concentrated and 7 ml of ethanol was added to the residue. To the resulting solution was added 0.16 g of sodium borohydride under ice cooling. After completion of the addition, the resulting mixture was heated under reflux until gas evolution ceased. The reaction mixture was concentrated under reduced pressure and then the residue was poured into ice-water, followed by three times of extraction with dichloromethane and drying over magnesium sulfate. After removal of the dichloromethane by evaporation under reduced pressure, the residue was purified by silica gel column chromatography (developing solvent: ethyl acetate) to obtain 87 mg (yield 16%) of the oily desired product.

Next, representative examples of the pyridine compounds of the above formula (I) are shown in Table 1. These compounds can be produced in accordance with the above synthetic examples or the above Processes [A] to [L]. Also, in the tables, Me represents methyl, Et represents ethyl, (n)Pr represents *n*-propyl, (i)Pr represents isopropyl, (n)Bu represents *n*-butyl, (i)Bu represents isobutyl, (s)Bu represents *sec*-butyl (i.e., 1-methylpropyl), (t)Bu represents *tert*-butyl, (n)Pe represents *n*-pentyl, (n)Hex represents *n*-hexyl, (n)Oc represents *n*-octyl, MeO- represents methoxy, MeS- represents methylthio, EtO- represents ethoxy, (i)BuO- represents isobutyloxy, Ph represents phenyl, PhCH₂- represents benzyl, and PhCH(CH₃)-represents α-methylbenzyl. Moreover, in the tables, 3-Pe represents pentan-3-yl (i.e., 1-ethylpropyl), 4-I-PhCH₂- represents 4-iodobenzyl, 2,4-di-Cl-PhCH₂- represents 2,4-dichlorobenzyl, 2,3,6-tri-F-PhCH₂- represents 2,3,6-trifluorobenzyl, and 2,3,4,5,6-penta-F-PhOCH₂- represents 2,3,4,5,6-pentafluorophenoxymethyl. These definitions may similarly apply to other similar descriptions.

The "oil" in the tables represents an oily substance. Moreover, Compound Nos. 1-221 and 1-222 are diastereomers for each other.

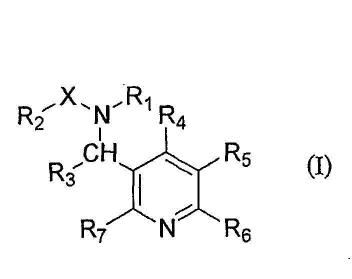

**TABLE 1**

| Comp. No. | X | R¹ | R² | R³ | R⁴ | R⁵ | R⁶ | R⁷ | Properties (mp:°C) |
|---|---|---|---|---|---|---|---|---|---|
| I-1 | C=O | Me | PhCH₂- | (i)Pr | CF₃ | H | H | H | 71.2 |
| I-2 | C=O | Me | 4-I-PhCH₂- | (i)Pr | CF₃ | H | H | H | 91.2 |
| I-3 | C=O | H | PhCH₂- | (i)Pr | CF₃ | H | H | H | 132.8 |
| I-4 | C=O | H | 2,4-di-Cl-PhCH₂- | (i)Pr | CF₃ | H | H | H | 168.4 |
| I-5 | C=O | H | 2,6-di-F-PhCH₂- | (i)Pr | CF₃ | H | H | H | 175.6 |
| I-6 | C=O | H | 4-Ph-PhCH₂- | (i)Pr | CF₃ | H | H | H | 157.1 |
| I-7 | C=O | H | 4-CF₃-PhCH₂- | (i)Pr | CF₃ | H | H | H | 139.0 |
| I-8 | C=O | H | 4-Br-PhCH₂- | (i)Pr | CF₃ | H | H | H | 166.8 |
| I-9 | C=O | Me | 4-Ph-PhCH₂- | (i)Pr | CF₃ | H | H | H | 105.8 |
| I-10 | C=O | Me | 4-Br-PhCH₂- | (i)Pr | CF₃ | H | H | H | 147.2 |
| I-11 | C=O | H | PhCH(CH₃)- | (i)Pr | CF₃ | H | H | H | 112.3 |
| I-12 | C=O | Me | PhCH(CH₃)- | (i)Pr | CF₃ | H | H | H | 123.1 |
| I-13 | C=O | Me | 2,6-di-F-PhCH₂- | (i)Pr | CF₃ | H | H | H | 136.3 |
| I-14 | C=O | Me | 2,4-di-Cl-PhCH₂- | (i)Pr | CF₃ | H | H | H | 158.7 |
| I-15 | C=O | Me | 2-Br-PhCH₂- | (i)Pr | CF₃ | H | H | H | 121.4 |
| I-16 | C=O | Me | 3-Br-PhCH₂- | (i)Pr | CF₃ | H | H | H | 96.3 |
| I-17 | C=O | Me | 2,3,6-tri-F-PhCH₂- | (i)Pr | CF₃ | H | H | H | 129.2 |
| I-18 | C=O | Me | 2,4-di-F-PhCH₂- | (i)Pr | CF₃ | H | H | H | 127.5 |
| I-19 | C=O | Me | 2,3-di-F-PhCH₂- | (i)Pr | CF₃ | H | H | H | 125.1 |
| I-20 | C=O | Me | 3-F-PhCH₂- | (i)Pr | CF₃ | H | H | H | 97.7 |
| I-21 | C=O | Me | 4-F-PhCH₂- | (i)Pr | CF₃ | H | H | H | 74.6 |
| I-22 | C=O | Me | PhCH₂- | (i)Pr | Cl | H | H | H | oil |
| I-23 | C=O | Me | 4-Cl-PhCH₂- | (i)Pr | CF₃ | H | H | H | oil |
| I-24 | C=O | Et | PhCH₂- | (i)Pr | CF₃ | H | H | H | 93.5 |
| I-25 | C=O | Me | 2-F-PhCH₂- | (i)Pr | CF₃ | H | H | H | 102.7 |
| I-26 | C=O | Me | 2-Cl-PhCH₂- | (i)Pr | CF₃ | H | H | H | 129.6 |
| I-27 | C=O | Me | 2,6-di-Cl-PhCH₂- | (i)Pr | CF₃ | H | H | H | 149.4 |
| I-28 | C=O | Me | 3,4-di-Cl-PhCH₂- | (i)Pr | CF₃ | H | H | H | 138.6 |
| I-29 | C=O | Me | 2-F-3-CF₃-PhCH₂- | (i)Pr | CF₃ | H | H | H | 117.4 |
| I-30 | C=O | Et | 2,6-di-F-PhCH₂- | (i)Pr | CF₃ | H | H | H | 121.9 |
| I-31 | C=O | Me | 4-CF₃-PhCH₂- | (i)Pr | CF₃ | H | H | H | 104.0 |
| I-32 | C=O | Me | 4-Cl-PhNH- | (i)Pr | CF₃ | H | H | H | 177.7 |
| I-33 | C=O | Me | 2-Cl-6-F-PhCH₂- | (i)Pr | CF₃ | H | H | H | 119.8 |
| I-34 | C=O | Me | 2-F-6-CF₃-PhCH₂- | (i)Pr | CF₃ | H | H | H | 139.2 |
| I-35 | C=O | Me | 2-MeO-PhCH₂- | (i)Pr | CF₃ | H | H | H | 81.5 |
| I-36 | C=O | Me | 2-Me-PhCH₂- | (i)Pr | CF₃ | H | H | H | 102.1 |
| I-37 | C=O | Me | 4-MeS-PhCH₂- | (i)Pr | CF₃ | H | H | H | oil |
| I-38 | C=O | Me | 4-MeO-PhCH₂- | (i)Pr | CF₃ | H | H | H | 114.5 |
| I-39 | C=O | Me | 2-NO₂-PhCH₂- | (i)Pr | CF₃ | H | H | H | 155.9 |
| I-40 | C=O | Me | 3-CF₃-PhCH₂- | (i)Pr | CF₃ | H | H | H | 83.2 |
| I-41 | C=O | Me | 2-CF₃-PhCH₂- | (i)Pr | CF₃ | H | H | H | 127.9 |
| I-42 | C=O | H | Et | 1-naphthyl | CF₃ | H | H | H | 217-218 |
| I-43 | C=O | Me | Et | 1-naphthyl | CF₃ | H | H | H | 136-137 |
| I-44 | C=O | Et | Et | 1-naphthyl | CF₃ | H | H | H | 50-51 |
| I-45 | C=O | H | MeO- | 1-naphthyl | CF₃ | H | H | H | 63-64 |
| I-46 | C=O | Me | MeO- | 1-naphthyl | CF₃ | H | H | H | oil |
| I-47 | C=O | Me | PhCH₂- | 1-naphthyl | CF₃ | H | H | H | 43-45 |
| I-48 | C=O | Me | 2-thienyl-CH₂- | 1-naphthyl | CF₃ | H | H | H | 109-110 |
| I-49 | C=O | H | (n)Pr | 1-naphthyl | CF₃ | H | H | H | 212-213 |
| I-50 | C=O | Me | (n)Pr | 1-naphthyl | CF₃ | H | H | H | oil |
| I-51 | C=O | H | (i)BuO- | 1-naphthyl | CF₃ | H | H | H | 42-43 |
| I-52 | C=O | Me | (i)BuO- | 1-naphthyl | CF₃ | H | H | H | oil |
| I-53 | C=O | H | EtO- | 1-naphthyl | CF₃ | H | H | H | 133-134 |
| I-54 | C=O | Me | EtO- | 1-naphthyl | CF₃ | H | H | H | 93-94 |
| I-55 | C=S | Me | PhCH₂- | (i)Pr | CF₃ | H | H | H | |
| I-56 | C=O | Me | 1-naphthyl-CH₂- | (i)Pr | CF₃ | H | H | H | 148.1 |
| I-57 | C=O | Et | 1-naphthyl-CH₂- | (i)Pr | CF₃ | H | H | H | 130.0 |
| I-58 | C=O | Me | 2-naphthyl-CH₂- | (i)Pr | CF₃ | H | H | H | 86.1 |
| I-59 | C=O | Me | 2-naphthyl-CH₂- | (i)Pr | Cl | H | H | H | sticky |
| I-60 | C=O | Me | 3,4-di-F-PhCH₂- | (i)Pr | CF₃ | H | H | H | 123.3 |
| I-61 | C=O | Me | 3-CF₃-4-F-PhCH₂- | (i)Pr | CF₃ | H | H | H | 94.4 |
| I-62 | C=O | Me | 3-F-4-CF₃-PhCH₂- | (i)Pr | CF₃ | H | H | H | 130.5 |
| I-63 | C=O | Me | 1-naphthyl- | (i)Pr | CF₃ | H | H | H | 135.9 |
| I-64 | C=O | Me | 4-Cl-Ph-NH- | (i)Pr | CF₃ | H | H | H | 177.7 |
| I-65 | C=O | Me | 3-F-PhCH₂- | (i)Pr | Cl | H | H | H | oil |
| I-66 | C=O | Me | 2-F-PhCH₂- | (i)Pr | Cl | H | H | H | oil |
| I-67 | C=O | Me | 4-F-PhCH₂- | (i)Pr | Cl | H | H | H | oil |
| I-68 | C=O | Me | 3-CF₃-PhCH₂- | (i)Pr | Cl | H | H | H | oil |
| I-69 | C=O | Me | 4-CF₃-PhCH₂- | (i)Pr | Cl | H | H | H | oil |
| I-70 | C=O | Me | 2,6-di-F-PhCH₂- | (i)Pr | Cl | H | H | H | 137.8 |
| I-71 | C=O | Me | 2,4-di-F-PhCH₂- | (i)Pr | Cl | H | H | H | oil |
| I-72 | C=O | Me | 2,3-di-F-PhCH₂- | (i)Pr | Cl | H | H | H | oil |
| I-73 | C=O | Me | 3,4-di-F-PhCH₂- | (i)Pr | Cl | H | H | H | oil |
| I-74 | C=O | Me | 2-F-3-CF₃-PhCH₂- | (i)Pr | Cl | H | H | H | oil |
| I-75 | C=O | Et | 3-CF₃-PhCH₂- | (i)Pr | CF₃ | H | H | H | 135.7 |
| I-76 | C=O | Et | 2-F-3-CF₃-PhCH₂- | (i)Pr | CF₃ | H | H | H | 107.2 |
| I-77 | C=O | Et | 2,3-di-F-PhCH₂- | (i)Pr | CF₃ | H | H | H | 170.2 |
| I-78 | C=O | Et | 2-F-PhCH₂- | (i)Pr | CF₃ | H | H | H | 53.1 |
| I-79 | C=O | Et | 4-CF₃-PhCH₂- | (i)Pr | CF₃ | H | H | H | 70.5 |
| I-80 | C=O | Me | 3,4-di-Cl-PhCH₂- | (i)Pr | Cl | H | H | H | oil |
| I-81 | C=O | Me | 2-Cl-PhCH₂- | (i)Pr | Cl | H | H | H | 136.9 |
| I-82 | C=O | Me | 4-Cl-PhCH₂- | (i)Pr | Cl | H | H | H | oil |
| I-83 | C=O | Me | 2-Me-PhCH₂- | (i)Pr | Cl | H | H | H | 125.3 |
| I-84 | C=O | Me | Ph-O-CH₂- | (i)Pr | Cl | H | H | H | 81.1 |
| I-85 | C=O | Me | 2,5-di-F-PhCH₂- | (i)Pr | CF₃ | H | H | H | 104.6 |
| I-86 | C=O | Me | PhCH₂- | (i)Pr | SMe | H | H | H | oil |
| I-87 | C=O | Me | 3-Me-PhCH₂- | (i)Pr | CF₃ | H | H | H | oil |
| I-88 | C=O | Me | 3-Me-PhCH₂- | (i)Pr | Cl | H | H | H | oil |
| I-89 | C=O | Me | 2,3,6-tri-F-PhCH₂- | (i)Pr | Cl | H | H | H | 117.1 |
| I-90 | C=O | Me | 4-MeCO₂-PhCH₂- | (i)Pr | CF₃ | H | H | H | oil |
| I-91 | C=O | Me | 3,5-di-Cl-PhCH₂- | (i)Pr | CF₃ | H | H | H | 154.0 |
| I-92 | C=O | Me | PhCH₂- | (i)Pr | H | H | H | Cl | oil |
| I-93 | C=O | Me | PhCH₂- | (i)Pr | H | H | H | H | oil |
| I-94 | C=O | Me | 4-Me-PhCH₂- | (i)Pr | CF₃ | H | H | H | oil |
| I-95 | C=O | Me | 4-Et-PhCH₂- | (i)Pr | CF₃ | H | H | H | oil |
| I-96 | C=O | Me | 2-F-5-CF₃-PhCH₂- | (i)Pr | CF₃ | H | H | H | 110.4 |
| I-97 | C=O | Me | 3-F-5-CF₃-PhCH₂- | (i)Pr | CF₃ | H | H | H | 142.2 |
| I-98 | C=O | Et | 2,5-di-F-PhCH₂- | (i)Pr | CF₃ | H | H | H | 128.2 |
| I-99 | C=O | Et | 4-F-PhCH₂- | (i)Pr | CF₃ | H | H | H | 88.4 |
| I-100 | C=O | Et | 4-Cl-PhCH₂- | (i)Pr | CF₃ | H | H | H | 105.9 |
| I-101 | C=O | Et | 4-Me-PhCH₂- | (i)Pr | CF₃ | H | H | H | oil |
| I-102 | C=O | Et | PhCH₂- | (i)Pr | Cl | H | H | H | oil |
| I-103 | C=O | Me | 4-(i)Pr-PhCH₂- | (i)Pr | CF₃ | H | H | H | oil |
| I-104 | C=O | Et | 4-(i)Pr-PhCH₂- | (i)Pr | CF₃ | H | H | H | 92.7 |
| I-105 | C=O | Me | 2-F-4-CF₃-PhCH₂- | (i)Pr | CF₃ | H | H | H | 151.3 |
| I-106 | C=O | Me | 3,5-di-Me-PhCH₂- | (i)Pr | CF₃ | H | H | H | oil |
| I-107 | C=O | Me | 2,5-di-Me-PhCH₂- | (i)Pr | CF₃ | H | H | H | 146.6 |
| I-108 | C=O | Me | 2-F-5-Me-PhCH₂- | (i)Pr | CF₃ | H | H | H | 137.1 |
| I-109 | C=O | Me | 2-F-4-Me-PhCH₂- | (i)Pr | CF₃ | H | H | H | oil |
| I-110 | C=O | Me | PhCH₂- | (i)Pr | Br | H | H | H | oil |
| I-111 | C=O | Me | PhCH₂-O- | (i)Pr | CF₃ | H | H | H | oil |
| I-112 | C=S | Me | Me-NH- | (i)Pr | CF₃ | H | H | H | 148.8 |
| I-113 | C=O | Me | PhCH₂-S- | (i)Pr | CF₃ | H | H | H | oil |
| I-114 | C=O | Me | 4-CN-PhCH₂- | (i)Pr | CF₃ | H | H | H | 108.3 |
| I-115 | C=O | H | Ph | (i)Pr | CF₃ | H | H | H | 124.8 |
| I-116 | C=O | Me | 2-Me-4-F-PhCH₂- | (i)Pr | CF₃ | H | H | H | 114.3 |
| I-117 | SO₂ | Me | Ph | (i)Pr | CF₃ | H | H | H | oil |
| I-118 | C=O | Me | cyclopropyl | Ph | CF₃ | H | H | H | |
| I-119 | C=O | Me | Ph-O-CH(CH₃)- | (i)Pr | CF₃ | H | H | H | amorphous |
| I-120 | C=O | Me | 2-tetrahydrofuryl | (i)Pr | CF₃ | H | H | H | 69-70 |
| I-121 | C=O | H | MeNH- | Ph | CF₃ | H | H | H | 211-212 |
| I-122 | C=O | Me | 4-MeO-N=CH-PhCH₂- | (i)Pr | CF₃ | H | H | H | oil |
| I-123 | C=O | Me | PhCH₂- | (i)Pr | H | H | CF₃ | H | oil |
| I-124 | SO₂ | Me | 4-F-Ph | (i)Pr | CF₃ | H | H | H | oil |
| I-125 | SO₂ | Me | 4-Cl-Ph | (i)Pr | CF₃ | H | H | H | oil |
| I-126 | SO₂ | Me | 2-Cl-Ph | (i)Pr | CF₃ | H | H | H | 56.7 |
| I-127 | SO₂ | Me | 2,4-di-F-Ph | (i)Pr | CF₃ | H | H | H | 131.2 |
| I-128 | C=O | Me | 4-CHF₂O-PhCH₂- | (i)Pr | CF₃ | H | H | H | oil |
| I-129 | C=O | Me | 3-F-4-Br-PhCH₂- | (i)Pr | CF₃ | H | H | H | 130.1 |
| I-130 | SO₂ | Me | 4-Me-Ph | (i)Pr | CF₃ | H | H | H | amorphous |
| I-131 | C=O | Me | 4-Me-Ph-O-CH(CH₃)- | (i)Pr | CF₃ | H | H | H | 85.3 |
| I-132 | C=O | Me | 4-Br-Ph-O-CH(CH₃)- | (i)Pr | CF₃ | H | H | H | amorphous |
| I-133 | C=O | Me | 4-F-Ph-O-CH(CH₃)- | (i)Pr | CF₃ | H | H | H | amorphous |
| I-134 | SO₂ | Me | PhCH₂- | (i)Pr | CF₃ | H | H | H | oil |
| I-135 | SO₂ | Me | Ph-CH=CH- | (i)Pr | CF₃ | H | H | H | oil |
| I-136 | SO₂ | Me | 4-Br-Ph | (i)Pr | CF₃ | H | H | H | amorphous |
| I-137 | C=O | Me | 2-F-4-Br-PhCH₂- | (i)Pr | CF₃ | H | H | H | 130.0 |
| I-138 | C=O | Me | 2,4-di-F-Ph-O-CH(CH₃)- | (i)Pr | CF₃ | H | H | H | amorphous |
| I-139 | C=O | Me | 4-Cl-Ph-O-CH(CH₃)- | (i)Pr | CF₃ | H | H | H | oil |
| I-140 | C=O | Me | Ph-S-CH(CH₃)- | (i)Pr | CF₃ | H | H | H | oil |
| I-141 | C=O | Me | 4-Cl-Fh-S-CH(CH₃)- | (i)Pr | CF₃ | H | H | H | oil |
| I-142 | SO₂ | Me | 4-Cl-Ph-CH=CH- | (i)Pr | CF₃ | H | H | H | oil |
| I-143 | C=O | Me | Ph-CF₂- | (i)Pr | CF₃ | H | H | H | oil |
| I-144 | SO₂ | Me | 4-MeO-Ph- | (i)Pr | CF₃ | H | H | H | oil |
| I-145 | SO₂ | Me | 4-(t)Bu-Ph- | (i)Pr | CF₃ | H | H | H | oil |
| I-146 | SO₂ | Me | 4-CF₃-Ph- | (i)Pr | CF₃ | H | H | H | oil |
| I-147 | C=O | Me | Ph-O-CF₂- | (i)Pr | CF₃ | H | H | H | oil |
| I-148 | SO₂ | Et | 4-Me-Ph- | (i)Pr | CF₃ | H | H | H | 96.7 |
| I-149 | C=O | Me | 3,4-di-Cl-Ph-O-CH(CH₃)- | (i)Pr | CF₃ | H | H | H | 82.1 |
| I-150 | C=O | Me | 2-F-4-Cl-Ph-O-CH(CH₃)- | (i)Pr | CF₃ | H | H | H | 91.9 |
| I-151 | C=O | Me | 2-F-4-Br-Ph-O-CH(CH₃)- | (i)Pr | CF₃ | H | H | H | 120.0 |
| I-152 | C=O | Me | 3,4-di-Me-Ph-O-CH(CH₃)- | (i)Pr | CF₃ | H | H | H | 118.6 |
| I-153 | C=O | Me | 4-Et-Ph-O-CH(CH₃)- | (i)Pr | CF₃ | H | H | H | oil |
| I-154 | C=O | Me | 3-F-4-Cl-Ph-O-CH(CH₃)- | (i)Pr | CF₃ | H | H | H | oil |
| I-155 | C=O | Me | 4-CF₃-Ph-O-CH(CH₃)- | (i)Pr | CF₃ | H | H | H | oil |
| I-156 | SO₂ | Me | 4-Et-Ph- | (i)Pr | CF₃ | H | H | H | oil |
| I-157 | SO₂ | Me | 3,4-di-Cl-Ph- | (i)Pr | CF₃ | H | H | H | oil |
| I-158 | C=O | Me | Ph-S-CF₂- | (i)Pr | CF₃ | H | H | H | oil |
| I-159 | C=O | Me | 4-F-Ph-O-CF₂- | (i)Pr | CF₃ | H | H | H | 130.7 |
| I-160 | C=O | Me | 4-F-Ph-CF₂- | (i)Pr | CF₃ | H | H | H | oil |
| I-161 | C=O | Me | 4-Me-Ph-CF₂- | (i)Pr | CF₃ | H | H | H | 133.5 |
| I-162 | C=O | Me | 4-MeO-Ph-CF₂- | (i)Pr | CF₃ | H | H | H | oil |
| I-163 | C=O | Me | Et | (i)Pr | CF₃ | H | H | H | 42 |
| I-164 | C=O | Me | cyclohexyl-CH₂- | (i)Pr | CF₃ | H | H | H | oil |
| I-165 | C=O | Me | cyclopentyl-CH₂- | (i)Pr | CF₃ | H | H | H | oil |
| I-166 | C=O | Me | Ph-CH=CH- | (i)Pr | CF₃ | H | H | H | oil |
| I-167 | C=O | H | Ph-CH=CH- | (i)Pr | CF₃ | H | H | H | 203-204 |
| I-168 | C=O | Me | Cl-CH₂- | (i)Pr | CF₃ | H | H | H | oil |
| I-169 | C=O | Me | Ph-CH₂CH₂- | (i)Pr | CF₃ | H | H | H | oil |
| I-170 | C=O | Me | 3-thienyl-CH₂- | (i)Pr | CF₃ | H | H | H | oil |
| I-171 | C=O | Me | 4-Cl-Ph-CH=CH- | (i)Pr | CF₃ | H | H | H | oil |
| I-172 | C=O | Me | 4-F-Ph-CH=CH- | (i)Pr | CF₃ | H | H | H | oil |
| I-173 | C=O | Me | 2-thienyl-CH=CH- | (i)Pr | CF₃ | H | H | H | 127-128 |
| I-174 | C=O | Me | Ph(Me)C=CH- | (i)Pr | CF₃ | H | H | H | oil |
| I-175 | C=O | Me | cyclohexyl-CH=CH- | (i)Pr | CF₃ | H | H | H | oil |
| I-176 | C=O | Me | cyclopentylidenemethyl | (i)Pr | CF₃ | H | H | H | oil |
| I-177 | C=O | Me | cyclohexylidenemethyl | (i)Pr | CF₃ | H | H | H | oil |
| I-178 | C=O | Me | 4-Br-Ph-CH=CH- | (i)Pr | CF₃ | H | H | H | oil |
| I-179 | C=O | Me | 3-Br-Ph-CH=CH- | (i)Pr | CF₃ | H | H | H | oil |
| I-180 | C=O | Me | Me-CH=CH- | (i)Pr | CF₃ | H | H | H | oil |
| I-181 | C=O | Me | 4-Me-Ph-CH=CH- | (i)Pr | CF₃ | H | H | H | oil |
| I-182 | C=O | Me | 4-MeO-Ph-CH=CH- | (i)Pr | CF₃ | H | H | H | oil |
| I-183 | C=O | Me | 2-cyclopentenyl-CH₂- | (i)Pr | CF₃ | H | H | H | oil |
| I-184 | C=O | Me | 3-pyridyl-CH=CH- | (i)Pr | CF₃ | H | H | H | 107-108 |
| I-185 | C=O | Me | Ph-C=C- | (i)Pr | CF₃ | H | H | H | oil |
| I-186 | C=O | Me | 4-CF₃-Ph-CH=CH- | (i)Pr | CF₃ | H | H | H | oil |
| I-187 | C=O | Me | 1-naphthyl-CH=CH- | (i)Pr | CF₃ | H | H | H | 144-145 |
| I-188 | C=O | Me | 2-naphthyl-CH=CH- | (i)Pr | CF₃ | H | H | H | 139-140 |
| I-189 | C=O | Me | (n)Pr | (i)Pr | CF₃ | H | H | H | oil |
| I-190 | C=O | Me | CH₂=CH- | (i)Pr | CF₃ | H | H | H | oil |
| I-191 | C=O | Me | indan-2-yl-CH₂- | (i)Pr | CF₃ | H | H | H | oil |
| I-192 | C=O | Me | benzothiophen-3-yl-CH₂- | (i)Pr | CF₃ | H | H | H | 117-118 |
| I-193 | C=O | Me | (i)Bu | (i)Pr | CF₃ | H | H | H | 49-50 |
| I-194 | C=O | Me | (CH₃)₃CCH₂- | (i)Pr | CF₃ | H | H | H | oil |
| I-195 | C=O | Me | MeO-CH₂- | (i)Pr | CF₃ | H | H | H | oil |
| I-196 | C=O | Me | (CH₃)₃CCH(Br)- | (i)Pr | CF₃ | H | H | H | 138-142 |
| I-197 | C=O | Me | (CH₃)₂CHCH(Br)- | (i)Pr | CF₃ | H | H | H | 113-120 |
| I-198 | C=O | Me | (i)Pr | (i)Pr | CF₃ | H | H | H | 84 |
| I-199 | C=O | Me | (n)Bu | (i)Pr | CF₃ | H | H | H | oil |
| I-200 | C=O | Me | Br(CH₂)₃- | (i)Pr | CF₃ | H | H | H | oil |
| I-201 | C=O | Me | Br(CH₂)₂- | (i)Pr | CF₃ | H | H | H | oil |
| I-202 | C=O | Me | CH₃CH₂CH(Br)- | (i)Pr | CF₃ | H | H | H | 129-130 |
| I-203 | C=O | Me | (n)Pr-O- | (i)Pr | CF₃ | H | H | H | oil |
| I-204 | C=O | Me | cyclopropyl | (i)Pr | CF₃ | H | H | H | 88-89 |
| I-205 | C=O | Me | Me₂C=CH- | (i)Pr | CF₃ | H | H | H | 65-66 |
| I-206 | C=O | Me | Ph-CH=C(Me)- | (i)Pr | CF₃ | H | H | H | oil |
| I-207 | C=O | Me | Ph-CH=CF- | (i)Pr | CF₃ | H | H | H | 86-87 |
| I-208 | C=O | Me | Me | (i)Pr | CF₃ | H | H | H | oil |
| I-209 | C=O | Me | CF₃ | (i)Pr | CF₃ | H | H | H | 80-81 |
| I-210 | C=O | Me | Cl-CH₂CH₂CH₂- | (i)Pr | CF₃ | H | H | H | oil |
| I-211 | C=O | Me | CH₂=CHCH₂CH₂- | (i)Pr | CF₃ | H | H | H | oil |
| I-212 | C=O | Me | cyclopentyl | (i)Pr | CF₃ | H | H | H | 105-106 |
| I-213 | C=O | Me | CH₂=C(Me)- | (i)Pr | CF₃ | H | H | H | oil |
| I-214 | C=O | Me | CH₃(CH₂)₂CH(Me)- | (i)Pr | CF₃ | H | H | H | oil |
| I-215 | C=O | Me | CH₃CH₂CH(Me)- | (i)Pr | CF₃ | H | H | H | 60-64 |
| I-216 | C=O | Me | (t)Bu | (i)Pr | CF₃ | H | H | H | 104-105 |
| I-217 | C=O | Me | CH₃CH(CF₃)CH₂- | (i)Pr | CF₃ | H | H | H | oil |
| I-218 | C=O | Me | (n)Oc | (i)Pr | CF₃ | H | H | H | |
| I-219 | C=O | Me | CF₃C(Me)C=CH- | (i)Pr | CF₃ | H | H | H | |
| I-220 | C=O | Me | Et₂CH- | (i)Pr | CF₃ | H | H | H | 67-68 |
| I-221 | C=O | Me | 2,2-di-Cl-1-Me-cyclopropyl | (i)Pr | CF₃ | H | H | H | oil |
| I-222 | C=O | Me | 2,2-di-Cl-1-Me-cyclopropyl | (i)Pr | CF₃ | H | H | H | 99-101 |
| I-223 | C=O | Me | (i) Pr | Ph | CF₃ | H | H | H | oil |
| I-224 | C=O | Me | (n) Pr | Ph | CF₃ | H | H | H | |
| I-225 | C=O | Me | PhCH₂- | Ph | CF₃ | H | H | H | |
| I-226 | C=O | Me | Ph-CH=CH- | Ph | CF₃ | H | H | H | |
| I-227 | C=O | Me | (i)Pr-O- | Ph | CF₃ | H | H | H | |
| I-228 | C=O | Me | (i)Bu | Ph | CF₃ | H | H | H | oil |
| I-229 | C=O | Me | Ph-O-CH₂- | (i)Pr | CF₃ | H | H | H | 120 |
| I-230 | C=O | Me | Ph-NH- | (i)Pr | CF₃ | H | H | H | 152 |
| I-231 | C=S | Me | Ph-NH- | (i)Pr | CF₃ | H | H | H | oil |
| I-232 | C=O | Me | PhN(CH₃)- | (i)Pr | CF₃ | H | H | H | oil |
| I-233 | C=O | Me | PhN(CH₂CH₃)- | (i)Pr | CF₃ | H | H | H | oil |
| I-234 | C=O | Me | 2-Me-4-Cl-PhOCH₂- | (i)Pr | CF₃ | H | H | H | oil |
| I-235 | C=O | Me | 2-Cl-PhOCH₂- | (i)Pr | CF₃ | H | H | H | oil |
| I-236 | C=O | Me | 2,4-di-Cl-PhOCH₂- | (i)Pr | CF₃ | H | H | H | oil |
| I-237 | C=O | Me | 4-NO₂-PhOCH₂- | (i)Pr | CF₃ | H | H | H | 105 |
| I-238 | C=O | Me | 3-Me-PhOCH₂- | (i)Pr | CF₃ | H | H | H | 100 |
| I-239 | C=O | Me | 4-Me-PhOCH₂- | (i)Pr | CF₃ | H | H | H | 113 |
| I-240 | C=O | Me | 2-Me-PhOCH₂- | (i)Pr | CF₃ | H | H | H | 113 |
| I-241 | C=O | Me | 4-F-PhOCH₂- | (i)Pr | CF₃ | H | H | H | oil |
| I-242 | C=O | Me | 3-F-PhOCH₂- | (i)Pr | CF₃ | H | H | H | 118 |
| I-243 | C=O | Me | 2-F-PhOCH₂- | (i)Pr | CF₃ | H | H | H | 119 |
| I-244 | C=O | Me | 4-MeO-PhOCH₂- | (i)Pr | CF₃ | H | H | H | oil |
| I-245 | C=O | Me | 2,5-di-F-PhOCH₂- | (i)Pr | CF₃ | H | H | H | 125 |
| I-246 | C=O | Me | 2,4-di-F-PhOCH₂- | (i)Pr | CF₃ | H | H | H | 112 |
| I-247 | C=O | Me | 3-Cl-PhOCH₂- | (i)Pr | CF₃ | H | H | H | oil |
| I-248 | C=O | Me | 4-Cl-PhOCH₂- | (i)Pr | CF₃ | H | H | H | oil |
| I-249 | C=O | Me | 4-Et-PhOCH₂- | (i)Pr | CF₃ | H | H | H | oil |
| I-250 | C=O | Me | 4-(t)Bu-PhOCH₂- | (i)Pr | Cl | H | H | H | oil |
| I-251 | C=O | Me | 4-MeS-PhOCH₂- | (i)Pr | CF₃ | H | H | H | oil |
| I-252 | C=O | Me | 2,3,4,5,6-penta-F-PhOCH₂- | (i)Pr | CF₃ | H | H | H | 121 |
| I-253 | C=O | Me | 2,4-di-Cl-5-NO₂-PhOCH₂- | (i)Pr | CF₃ | H | H | H | oil |
| I-254 | C=O | Me | 3-CF₃-PhOCH₂- | (i)Pr | CF₃ | H | H | H | oil |
| I-255 | C=O | Me | 4-Br-PhOCH₂- | (i)Pr | CF₃ | H | H | H | oil |
| I-256 | C=O | Me | PhSCH₂- | (i)Pr | CF₃ | H | H | H | oil |
| I-257 | C=O | Me | PhN((i)Pr)- | (i)Pr | CF₃ | H | H | H | oil |
| I-258 | C=O | Me | 2-thienyl-CH₂- | (i)Pr | CF₃ | H | H | H | oil |
| I-259 | C=O | Et | PhOCH₂- | (i)Pr | CF₃ | H | H | H | oil |
| I-260 | C=O | Me | PhCH₂- | (s)Bu | CF₃ | H | H | H | oil |
| I-261 | C=O | Me | PhOCH₂- | (s)Bu | CF₃ | H | H | H | 104 |
| I-262 | C=O | Me | PhCH₂- | 3-Pe | CF₃ | H | H | H | oil |
| I-263 | C=O | Me | PhOCH₂- | 3-Pe | CF₃ | H | H | H | oil |
| I-264 | C=O | H | (i)Pr | Ph | CF₃ | H | H | H | 171-172 |
| I-265 | C=O | H | (i)Bu | Ph | CF₃ | H | H | H | 187-188 |
| I-266 | C=O | Me | (n)Pe | (i)Pr | CF₃ | H | H | H | oil |
| I-267 | C=O | Me | Me₂N- | Ph | CF₃ | H | H | H | oil |
| I-268 | C=O | Me | EtOC(=O)CH₂CH₂- | (i)Pr | CF₃ | H | H | H | oil |
| I-269 | C=O | Me | (n)Hex | (i)Pr | CF₃ | H | H | H | oil |
| I-270 | SO₂ | Me | CF₃CH₂- | (i)Pr | CF₃ | H | H | H | 99.5 |

Also, NMR spectrum data of the pyridine compounds are described in Table 2.

**TABLE 2**

| Comp. No. | ¹H-NMR δppm ( Solvent : CDCl₃ /400MHz) |
|---|---|
| I-22 | 0.87(3H,d), 0.97(3H,d), 2.47-2.56(1H,m), 2.72(3H,s), |
| | 3.68(2H,m), 5.66(1H,d), 7.19-7.31(5H,m), 7.38(1H,d), |
| | 8.39(1H,d), 8.62(1H,s) |
| I-23 | 0.77(3H,d), 0.97(3H,d), 2.56-2.65(1H,m), 2.82(3H,s), |
| | 3.62(2H,m), 5.73(1H,d), 7.12-7.82 (4H,m), 7.58(1H,d), |
| | 8.70(1H,d), 8.98(1H,s) |
| I-37 | 0.78(3H,d), 0.96(3H,d), 2.41(3H,s), 2.56-2.65(1H,m), |
| | 2.82(3H,s), 3.61(2H,m), 5.75(1H,d), 7.11-7.22 (4H,m), |
| | 7.58(1H,d), 8.69(1H,d), 8.97(1H,s) |
| I-46 | 2.71(3H,s), 2.75(3H,s), 3.72(3H,s), 3.79(3H,s), 6.86(1H,d), |
| | 6.97(1H,d), 7.33-7.91(8H,m,8H,m), 8.59(1H,s), 8.72(1H,s), |
| | 8.77-8.79(1H,d,1H,d), rotational isomer mixture |
| I-50 | 0.99(3H,t), 1.71-1.77(2H,m), 2.42(2H,t), 2.79(3H,s), |
| | 6.80(1H,d), 7.32-7.91(8H,m), 8.66(1H,s), 8.77(1H,d) |
| I-52 | 0.69-0.75(3H,d,3H,d), 0.93-0.95(3H,d,3H,d), 1.78-1.99 |
| | (1H,m,1H,m), 2.73(3H,s), 2.74(3H,s), 3.90-3.99 |
| | (2H,m,2H,m), 6.86(1H,d), 6.93(1H,d), 7.33-7.91 |
| | (8H,m,8H,m), 8.66-8.78(1H,s,1H,s,1H,bs,1H,bs), |
| | diastereomer mixture |
| I-59 | 0.85(3H,d), 0.98(3H,d), 2.43-2.54(1H,m), 2.71(3H,s), |
| | 3.84(2H,m), 5.73(1H,d), 7.31-7.79(8H,m), 8.37(1H,d), |
| | 8.60(1H,s) |
| I-65 | 0.88(3H,d), 0.97(3H,d), 2.47-2.57(1H,m), 2.72(3H,s), |
| | 3.65(2H,m), 5.68(1H,d), 6.88-7.27(4H,m), 7.34(1H,d), |
| | 8.39(1H,d), 8.61(1H,s) |
| I-66 | 0.87(3H,d), 0.95(3H,d), 2.51-2.57(1H,m), 2.77(3H,s), |
| | 3.70(2H,m), 5.66(1H,d), 6.98-7.34(4H,m), 7.39(1H,d), |
| | 8.39(1H,d), 8.63(1H,s) |
| I-67 | 0.83(3H,d), 0.96(3H,d), 2.47-2.66(1H,m), 2.83(3H,s), |
| | 3.64(2H,m), 5.67(1H,d), 6.93-7.27(4H,m), 7.33(1H,d), |
| | 8.39(1H,d), 8.61(1H,s) |
| I-68 | 0.85(3H,d), 0.98(3H,d), 2.48-2.57(1H,m), 2.72(3H,s), |
| | 3.70(2H,m), 5.70(1H,d), 7.29-7.52(5H,m), 8.39(1H,d), |
| | 8.61(1H,s) |
| I-69 | 0.86(3H,d), 0.98(3H,d), 2.48-2.57(1H,m), 2.73(3H,s), |
| | 3.73(2H,m), 5.68(1H,d), 7.07-7.66(5H,m), 8.40(1H,d), |
| | 8.62(1H,s) |
| I-71 | 0.89(3H,d), 0.99(3H,d), 2.50-2.64(1H,m), 2.79(3H,s), |
| | 3.65(2H,m), 5.64(1H,d), 6.75-7.26(3H,m), 7.34(1H,d), |
| | 8.40(1H,d), 8.64(1H,s) |
| I-72 | 0.88(3H,d), 0.99(3H,d), 2.49-2.56(1H,m), 2.79(3H,s), |
| | 3.65(2H,m), 5.65(1H,d), 6.95-7.10(3H,m), 7.34(1H,d), |
| | 8.40(1H,d), 8.64(1H,s) |
| I-73 | 0.86(3H,d), 0.97(3H,d), 2.48-2.58(1H,m), 2.73(3H,s), |
| | 3.61(2H,m), 5.66(1H,d), 6.91-7.13(3H,m), 7.34(1H,d), |
| | 8.40(1H,d), 8.62(1H,s) |
| I-74 | 0.86(3H,d), 1.03(3H,d), 2.51-2.63(1H,m), 2.81(3H,s), |
| | 3.68(2H,m), 5.65(1H,d), 7.15-7.50(4H,m), 8.40(1H,d), |
| | 8.64(1H,s) |
| I-80 | 0.86(3H,d), 0.98(3H,d), 2.48-2.59(1H,m), 2.72(3H,s), |
| | 3.60(2H,m), 5.67(1H,d), 7.05-7.40(4H,m), 8.40(1H,d), |
| | 8.62(1H,s) |
| I-82 | 0.84(3H,d), 0.96(3H,d), 2.47-2.56(1H,m), 2.71(3H,s), |
| | 3.65(2H,m), 5.66(1H,d), 7.14-7.28(4H,m), 7.34(1H,d), |
| | 8.39(1H,d), 8.61(1H,s) |
| I-86 | 0.85(3H,d), 0.92(3H,d), 2.42-2.50(1H,m), 2.62(3H,s), |
| | 3.68(2H,m), 5.55(1H,d), 7.03-7.29(6H,m), 8.32(1H,d), |
| | 8.39(1H,s) |
| I-87 | 0.77(3H,d), 0.97(3H,d), 2.27(3H,s), 2.55-2.65(1H,m), |
| | 2.84(3H,s), 3.62(2H,m), 5.76(1H,d), 6.97-7.15(4H,m), |
| | 7.56(1H,d), 8.69(1H,d), 8.96(1H,s) |
| I-88 | 0.85(3H,d), 0.97(3H,d), 2.27(1H,s), 2.48-2.54(1H,m), |
| | 2.69(3H,s), 3.64(2H,m), 5.69(1H,d), 6.97-7.21(4H,m), |
| | 7.33(1H,d), 8.38(1H,d), 8.62(1H,s) |
| I-90 | 0.72(3H,d), 0.91(3H,d), 2.49-2.62(1H,m), 2.74(3H,s), |
| | 3.66(2H,s), 3.82(3H,s), 5.78(1H,d), 7.23(2H,d), |
| | 7.52(1H,d), 7.89(2H,d), 8.64(1H,d), 8.89(1H,s) |
| I-92 | 0.83(3H,d), 0.97(3H,d), 2.52-2.61(1H,m), 2.76(3H,s), |
| | 3.68(2H,m), 5.31(1H,d), 7.20-7.31(6H,m), 7.83(1H,d), |
| | 8.29(1H,m) |
| I-93 | 0.85(3H,d), 0.91(3H,d), 2.31-2.43(1H,m), 2.68(3H,s), |
| | 3.68(2H,s), 5.52(1H,d), 7.16-7.37(5H,m), 7.65(1H,d), |
| | 8.50(1H,bs), 8.60(1H,bs) |
| I-94 | 0.77(3H,d), 0.96(3H,d), 2.31(1H,s), 2.55-2.78(1H,m), |
| | 2.83(3H,s), 3.64(2H,m), 5.76(1H,d), 7.05-7.17(4H,m), |
| | 7.56(1H,d), 8.68(1H,d), 8.96(1H,s) |
| I-95 | 0.75(3H,d), 0.95(3H,d), 1.16(3H,t), 2.55-2.61(3H,q), |
| | 2.77(3H,s), 3.57-3.66(2H,dd), 5.75(1H,d), 7.09(4H,s), |
| | 7.56(1H,d), 8.68(1H,d), 8.95(1H,s) |
| I-101 | 0.72(3H,t), 0.73(3H,d), 0.94(3H,d), 2.28(3H,s), |
| | 2.70-2.84(1H,m), 3.07-3.16(1H,m), 3.33-3.43(1H,m), |
| | 3.57-3.66(2H,m), 5.50(1H,d), 7.07-7.14(4H,dd), |
| | 7.56(1H,d), 8.70(1H,d), 9.18(1H,s) |
| I-103 | 0.78(3H,d), 0.99(3H,d), 1.21(6H,d) 2.56-2.65(1H,m), |
| | 2.79(3H,s), 2.823-2.89(1H,m), 3.62(2H,m), 5.75(1H,d), |
| | 7.10-7.21(4H,m), 7.56(1H,d), 8.69(1H,d), 8.97(1H,s) |
| I-106 | 0.78(3H,d), 0.98(3H,d), 2.23(6H,s), 2.56-2.65(1H,m), |
| | 2.84(3H,s), 3.58(2H,m), 5.76(1H,d), 6.81-6.91(3H,m), |
| | 7.60(1H,d), 8.68(1H,d), 8.98(1H,s) |
| 1-109 | 0.76(3H,d), 0.98(3H,d), 2.27(3H,s), 2.58-2.71(1H,m), |
| | 2.83(3H,s), 3.53-3.66(2H,dd), 5.68(1H,d), 6.80-6.86(2H,dd), |
| | 7.10(1H,t), 7.59(1H,d), 8.68(1H,d), 8.98(1H,s) |
| I-110 | 0.75(3H,d), 1.05(3H,d), 2.59-2.66(1H,m), 2.77(3H,s), |
| | 5.02-5.28(3H,m), 7.25-7.46(5H,m), 7.56(1H,d), 8.70(1H,d), |
| | 8.90(1H,d) |
| I-113 | 0.80(3H,d), 0.96(3H,d), 2.61-2.71(1H,m), 2.80(3H,s), |
| | 4.16(2H,m), 5.62(1H,d), 7.18-7.36 (5H,m), 7.57(1H,d), |
| | 8.71(1H,d), 8.98(1H,s) |
| I-117 | 0.76(3H,d), 0.87(3H,d), 1.00(3H,d), 1.29(3H,d), 2.02- |
| | 2.16(1H,m), 2.57-2.71(1H,m), 3.03(3H,s), 3.44(3H,s), |
| | 5.27(1H,d), 7.45-7.70(5H,m), 8.02(1H,d), 8.82(1H,d), |
| | 8.92(1H,s), 9.03(1H,s), rotational isomer mixture |
| I-119 | 0.71-0.76(3H,dd), 0.84-1.00(3H,dd), 1.49-1.54(3H,dd), |
| | 2.54-2.67(1H,m), 2.88(3H,s), 2.91(3H,s), 4.92-4.98(1H,q), |
| | 5.59-5.61(1H,d), 5.75-5.77(1H,d), 6.76-7.20(5H,m), |
| | 7.56(1H,d), 7.61(1H,d), 8.68(1H,d), 8.71(1H,d), 8.92(1H,s), |
| | 9.00(1H,s), diastereomer mixture |
| I-123 | 0.86(3H,d), 0.92(3H,d), 2.35-2.44(1H,m), 2.70(3H,s), |
| | 3.68(2H,s), 5.55(1H,d), 7.15-7.63(5H,m), 7.62(1H,d), |
| | 7.85(1H,d), 8.69(1H,s) |
| I-124 | 0.70(3H,d), 1.15(3H,d), 2.47-2.53(1H,m), 2.96(3H,s), |
| | 5.10(1H,d), 6.90-7.54(5H,m), 8.67(1H,d), 8.83(1H,s) |
| I-125 | 0.70(3H,d), 1.15(3H,d), 1.56(3H,s), 2.45-2.53(1H,m), |
| | 5.10(1H,d), 6.90-7.46 (4H,m), 7.52(1H,d), 8.75(1H,d), |
| | 8.88(1H,s) |
| I-128 | 0.81(3H,d), 0.98(3H,d), 2.58-2.71(1H,m), 2.85(3H,s), |
| | 3.66(2H,s), 5.76(1H,d), 6.49(1H,t), 7.07-7.22(4H,dd), |
| | 7.66(1H,d), 8.81(1H,d), 9.03(1H,s) |
| I-130 | 0.69(3H,d), 1.13(3H,d), 1.92-2.02(1H,m), 2.32(3H,s), |
| | 2.93(3H,s), 5.13(1H,d), 7.11(2H,d), 7.42(2H,d), 7.76(1H,d), |
| | 8.66(1H,d), 8.90(1H,s) |
| I-132 | 0.76(3H,3H,m), 0.86(3H,d), 1,10(3H,d), 1.53(3H,3H,m), |
| | 2.60-2.64(1H,1H,m), 2.87(3H,s), 2.90(3H,s), |
| | 4.86-4.92(1H,1H,m), 5.63(1H,d), 5.77(1H,d), |
| | 6.66-6.74(2H,2H,d), 7.25-7.29(2H,2H,d), 7.55-7.60(1H,1H,d), |
| | 8.68-8.73(1H,1H,d), 8.91-8.98(1H,1H,s), |
| | diastereomer mixture |
| I-133 | 0.76(3H,3H,m), 0.84(3H,d), 0.99(3H,d), 1.51(3H,3H,m), |
| | 2.56-2.66(1H,1H,m), 2.89(3H,s) 2.91(3H,s), |
| | 4.86-4.91(1H,1H,m), 5.64(1H,d), 5.77(1H,d), |
| | 6.72-6.81(4H,4H,m), 7.55-7.60(1H,1H,d), 8.67-8.73(1H,1H,d), |
| | 8.92-8.99(1H,1H,s), diastereomer mixture |
| I-134 | 0.74(3H,d), 1.08(3H,d), 1.95-2.01(1H,m), 2.20(3H,s) |
| | 3.70(1H,d), 4.32(2H,s), 7.36-7.38(5H,m), 7.40(1H,d), |
| | 8.64(1H,d), 8.97(1H,s) |
| I-135 | 0.72(3H,d), 1.25(3H,d), 2.51-2.57(1H,m), 2.79(3H,s), |
| | 4.07-4.13(1H,m), 5.17(1H,d), 6.31(1H,d), 7.20-7.37 (5H,m), |
| | 7.61(1H,d), 8.71(1H,d), 8.92(1H,s) |
| I-136 | 0.61(3H,d), 1.54(3H,d), 2.45-2.53(1H,m), 2.98(3H,s), |
| | 5.11(1H,d), 7.37-7.48 (4H,m), 7.49(1H,d),8.69(1H,d), |
| | 8.89(1H,s) |
| I-138 | 0.77(3H,3H,m), 0.87(3H,d), 0.94(3H,d), 1.60(3H,3H,m), |
| | 2.60-2.63(1H,1H,m), 2.88(3H,s), 2.91(3H,s), 4.92-4.97 |
| | (1H,1H,m), 5.67(1H,d), 5.77(1H,d), 6.63-6.94(4H,4H,m), |
| | 7.55-7.58(1H,1H,d), 8.69-8.72(1H,1H,d), 8.93-8.98(1H,1H,s), |
| | diastereomer mixture |
| I-139 | 0.75(3H,3H,m), 0.85(3H,d), 0.99(3H,d), 1.53(3H,3H,m), |
| | 2.60-2.66(1H,1H,m), 2.88(3H,s) 2.90(3H,s), 4.86-4.92 |
| | (1H,1H,m), 5.64(1H,d), 5.78(1H,d), 6.70-6.79(2H,2H,m), |
| | 7.09-7.15(2H,2H,m), 7.55-7.59(1H,1H,d), 8.68-8.73 |
| | (1H,1H,d), 8.91-8.98(1H,1H,s), diastereomer mixture |
| I-140 | 0.77(3H,3H,m), 1.00(3H,d), 1.10(3H,d), 1.42(3H,3H,m), |
| | 2.64-2.71(1H,1H,m), 2.91(3H,s), 2.95(3H,s), 3.92-4.12 |
| | (1H,1H,m), 5.57(1H,d), 5.69(1H,d), 7.17-7.61(5H,5H,m), |
| | 7.62-7.66(1H,1H,d), 8.72-8.73(1H,1H,d), 9.01-9.04(1H,1H,s), |
| | diastereomer mixture |
| I-141 | 0.77(3H,3H,m), 0.99(3H,d), 1.05(3H,d), 1.40(3H,3H,m), |
| | 2.64-2.70(1H,1H,m), 2.92(3H,s), 2.94(3H,s), 3.90-4.12 |
| | (1H,1H,m), 5.59(1H,d), 5.64(1H,d), 7.13-7.33(4H,4H,m), |
| | 7.61-7.64(1H,1H,d), 8.73(1H,1H,bd), 9.04(1H,1H,bs), |
| | diastereomer mixture |
| I-142 | 0.73(3H,d), 1.26(3H,d), 2.50-2.62(1H,m), 2.84(3H,s), |
| | 4.12(1H,dd), 5.17(1H,d), 6.31(1H,d), 7.25(4H,dd), |
| | 7.29(1H,d), 8.74(1H,d), 8.95(1H,s) |
| I-143 | 0.83(3H,d), 1.04(3H,d), 2.62-2.70(1H,m), 2.75(3H,s), |
| | 5.80(1H,d), 7.40-7.52(5H,m), 7.63(1H,d), 8.74(1H,d), |
| | 8.95(1H,s) |
| I-144 | 0.71(3H,d), 1.15(3H,d), 2.44-2.50(1H,m), 2.93(3H,s), |
| | 3.80(3H,s), 5.14(1H,d), 6.80(2H,d), 7.48(2H,d), |
| | 7.55(1H,d), 8.67(1H,d), 8.88(1H,s) |
| I-145 | 0.71(3H,d), 1.15(3H,d), 1.61(9H,m), 2.44-2.50(1H,m), |
| | 2.96(3H,s), 5.30(1H,d), 7.35(2H,d), 7.49(2H,d), 7.83(1H,d), |
| | 8.67(1H,d), 8.80(1H,s) |
| I-146 | 0.76(3H,d), 1.20(3H,d), 2.44-2.66(1H,m), 3.04(3H,s), |
| | 5.15(1H,d), 7.63(4H,d,d), 8.04(1H,d), 8.71(1H,d), 8.92(1H,s) |
| I-147 | 0.86(3H,d), 1.05(3H,d), 2.69-2.78(1H,m), 3.05(3H,s), |
| | 5.74(1H,d), 7.18-7.36(5H,m), 7.63(1H,d), 8.76(1H,d), |
| | 9.02(1H,s) |
| I-153 | 0.74(3H,3H,m), 0.83(3H,d), 1.01(3H,d), 1.14(3H,3H,m), |
| | 1.50(3H,3H,m), 2.48-2.51(2H,2H,m), 2.59-2.64(1H,1H,m), |
| | 2.89(3H,s) 2.91(3H,s), 4.88-4.93(1H,1H,m), 5.62(1H,d), |
| | 5.77(1H,d), 6.70-6.78(2H,2H,m), 6.97-7.03(2H,2H,m), |
| | 7.54-7.60(1H,1H,d), 8.66-8.72(1H,1H, d), |
| | 8.92-8.99(1H,1H,bs), diastereomer mixture |
| I-154 | 0.76(3H,3H,m), 0.90(3H,d), 1.00(3H,d), 1.52(3H,3H,m), |
| | 2.64-2.66(1H,1H,m), 2.89(3H,s), 2.91(3H,s), 4.83-4.91 |
| | (1H,1H,m), 5.59(1H,d), 5.74(1H,d), 6.52-7.20(3H,3H,m), |
| | 7.60-7.65(1H,1H,d), 8.71(1H,1H,d), 9.00(1H,1H,s), |
| | diastereomer mixture |
| I-155 | 0.75(3H,3H,m), 0.88(3H,d), 1.00(3H,d), 1.55(3H,3H,m), |
| | 2.62-2.66(1H,1H,m), 2.90(3H,s), 2.93(3H,s), 4.95-5.02 |
| | (1H,1H,m), 5.53(1H,d), 5.74(1H,d), 6.83-7.45(4H,4H,m), |
| | 7.57-7.64(1H,1H,d), 8.67-8.74(1H,1H,d), 8.92-9.04(1H,1H,s), |
| | diastereomer mixture |
| I-156 | 0.68(3H,d), 1.12(3H,d), 2.36-2.56(1H,m), 2.56(2H,q), |
| | 2.92(3H,s), 5.16(1H,d), 7.26(2H,d), 7.44(2H,d), 7.56(1H,d), |
| | 8.66(1H,d), 8.87(1H,s) |
| I-157 | 0.74(3H,d), 1.18(3H,d), 2.44-2.68(1H,m), 3.00(3H,s), |
| | 5.12(1H,d), 7.40(2H,d), 7.52(1H,s), 7.66(1H,d), 8.74(1H,d), |
| | 8.90(1H,s) |
| I-158 | 0.84(3H,d), 1.08(3H,d), 2.64-2.80(1H,m), 2.98(3H,s), |
| | 5.68(1H,d), 7.36-7.40(3H,m), 7.60(2H,d), 7.64(1H,d), |
| | 8.76(1H,d), 8.97(1H,s) |
| I-160 | 0.83(3H,d), 1.03(3H,d), 2.65-2.71(1H,m), 2.79(3H,s), |
| | 5.79(1H,d), 7.12(2H,d), 7.49(2H,d), 7.49(2H,d), |
| | 7.62(1H,d), 8.75(1H,d), 8.95(1H,s) |
| I-162 | 0.84(3H,d), 1.05(3H,d), 2.65-2.67(1H,m), 2.75(3H,s), |
| | 5.81(1H,d), 6.93(2H,d), 7.45(2H,d), 7.63(1H,d), |
| | 8.74(1H,d), 8.95(1H,s) |
| I-164 | 0.81(3H,d), 1.02(3H,d), 0.84-1.86(11H,m), 2.04-2.21(2H,m), |
| | 2.60-2.66(1H,m), 2.79(3H,s), 5.84(1H,d), 7.58(1H,d), 8.71 |
| | (1H,d), 8.99(1H,s) |
| I-165 | 0.81(3H,d), 1.02(3H,d), 1.04-2.37(11H,m), 2.58-2.68(1H,m), |
| | 2.80(3H,s), 5.84(1H,d), 7.58(1H,d), 8.71(1H,d), 8.99(1H,s) |
| I-166 | 0.87(3H,d), 1.097(3H,d), 2.69-2.75(1H,m), 3.00(3H,s), 5.87 |
| | (1H,d), 6.80(1H,d), 7.31-7.745(7H,m), 8.75(1H,d), 9.06(1H,s) |
| I-168 | 0.83(3H,d), 1.07(3H,d), 2.6-2.73(1H,m), 2.91(3H,s), 4.03 |
| | (2H,s), 5.69(1H,d), 7.61(1H,d), 8.75(1H,d), 9.02(1H,s) |
| I-169 | 0.82(3H,d), 1.00(3H,d), 2.49-2.66(3H,m), 2.77(3H,s), |
| | 2.88-3.04(2H,m), 5.79(1H,d), 7.15-7.30(5H,m), 7.59(1H,d), |
| | 8.72(1H,bs), 9.00(1H,s) |
| I-170 | 0.80(3H,d), 0.99(3H,d), 2.59-2.65(1H,m), 2.82(3H,s), |
| | 3.69(2H,s), 5.79(1H,d), 6.98(1H,dd), 7.04(1H,bs), 7.24-7.27 |
| | (1H,m), 7.59(1H,d), 8.72(1H,d), 8.98(1H,s) |
| I-171 | 0.87(3H,d), 1.09(3H,d), 2.69-2.75(1H,m), 2.99(3H,s), 5.85 |
| | (1H,d), 6.77(1H,d), 7.31-7.72(6H,m), 8.74(1H,d), 9.06(1H,s) |
| I-172 | 0.87(3H,d), 1.09(3H,d), 2.65-2.81(1H,m), 2.99(3H,s), |
| | 5.86(1H,d), 6.72(1H,d), 7.42-7.74(6H,m), 8.74(1H,d), |
| | 9.06(1H,s) |
| I-174 | 0.86(3H,d), 1.12(3H,d), 2.26(3H,s), 2.69-2.72(1H,m), |
| | 2.89(3H,s), 5.91(1H,d), 6.21(1H,s), 7.20-7.43(5H,m), |
| | 7.63(1H,d), 8.74(1H,bs), 9.04(1H,s) |
| I-175 | 0.86(3H,d), 1.05(3H,d), 1.08-2.18(11H,m), 2.66-2.72(1H,m), |
| | 2.90(3H,s), 5.75(1H,d), 6.08(1H,d), 6.86(1H,dd), 7.60(1H,d), |
| | 8.71(1H,d), 9.05(1H,s) |
| I-176 | 0.80(3H,d), 1.04(3H,d), 1.82-2.33(6H,m), 2.53-2.68(1H,m), |
| | 2.84(3H,s), 3.13(2H,m), 5.41(1H,bs), 5.76(1H,d), 7.60(1H,d), |
| | 8.72(1H,d), 9.01(1H,s) |
| I-177 | 0.82(3H,d), 1.06(3H,d), 1.55-2.38(10H,m), 2.59-2.72(1H,m), |
| | 2.83(3H,s), 5.65(1H,s), 5.85(1H,d), 7.60(1H,d), 8.72(1H,d), |
| | 9.01(1H,s) |
| I-178 | 0.87(3H,d), 1.09(3H,d), 2.69-2.77(1H,m), 2.99(3H,s), 5.87 |
| | (1H,d), 6.79(1H,d), 7.35-7.66(6H,m), 8.74(1H,d), 9.06(1H,s) |
| I-179 | 0.87(3H,d), 1.09(3H,d), 2.69-2.75(1H,m), 2.99(3H,s), |
| | 5.86(1H,d), 6.78(1H,d), 7.21-7.69(6H,m), 8.74(1H,d), |
| | 9.06(1H,s) |
| I-180 | 0.84(3H,d), 1.05(3H,d), 1.86(3H,d), 2.61-2.74(1H,m), |
| | 2.88(3H,s), 5.79(1H,d), 6.18(1H,d), 6.89-6.94(1H,m), |
| | 7.59(1H,d), 8.72(1H,d), 9.02(1H,s) |
| I-181 | 0.86(3H,d), 1.09(3H,d), 2.35(3H,s), 2.71-2.75(1H,m), |
| | 2.99(3H,s), 5.86(1H,d), 6.75(1H,d), 7.15(2H,d), 7.40(2H,d,), |
| | 7.60(1H,d), 7.69(1H,d), 8.73(1H,d), 9.06(1H,s) |
| I-182 | 0.86(3H,d), 1.09(3H,d), 2.69-2.75(1H,m), 2.99(3H,s), |
| | 3.82(3H,s), 5.87(1H,d), 6.68(1H,d), 6.87(2H,d), 7.405(2H,d), |
| | 7.60(1H,d), 7.70(1H,d), 8.73(1H,d), 9.06(1H,s) |
| I-183 | 0.82(3H,d), 1.03(3H,d), 1.35-1.49(1H,m), 2.11-2.44(5H,m), |
| | 2.55-2.73(1H,m), 2.79(3H,s), 3.17-3.20(1H,m), 5.68-5.73 |
| | (2H,m), 5.86(1H,d), 7.60(1H,d), 8.72(1H,d), 9.00(1H,s) |
| I-185 | 0.84-0.87(3H,d,3H,d), 1.11(3H,d), 1.19(3H,d), 2.68-2.78 |
| | (1H,m,1H,m), 2.87(3H,s), 3.14(3H,s), 5.75-5.80(1H,d,1H,d), |
| | 7.33-7.65(6H,m,6H,m), 8.75(1H,d), 8.79(1H,d), 9.00(1H,s), |
| | 9.05(1H,s), isomer mixture |
| I-186 | 0.88(3H,d), 1.10(3H,d), 2.70-2.76(1H,m), 3.01(3H,s), |
| | 5.88(1H,d), 6.88(1H,d), 7.58-7.79(6H,m), 8.75(1H,d), |
| | 9.07(1H,s) |
| I-I89 | 0.82(3H,d), 0.93(3H,t), 1.03(3H,d), 1.63-1.69(2H,m), |
| | 2.19-2.32(2H,m), 2.62-2.68(1H,m), 2.81(3H,s), 5.82(1H,d), |
| | 7.59(1H,d), 8.72(1H,d), 9.01(1H,s) |
| I-190 | 0.85(3H,d), 1.06(3H,d), 2.65-2.77(1H,m), 3.08(3H,s), |
| | 5.69(1H,dd), 5.81(1H,d), 6.32(1H,dd), 6.47-6.54(1H,dd), |
| | 7.61(1H,d), 8.74(1H,d), 9.05(1H,s) |
| I-191 | 0.84(3H,d), 1.05(3H,d), 2.37-2.67(5H,m), 2.77(3H,s), |
| | 2.95-3.02(1H,m), 3.15-3.22(2H,m), 5.88(1H,d), 7.11-7.18 |
| | (4H,m), 7.60(1H,d), 8.72(1H,d), 8.995(1H,s) |
| I-194 | 0.81(3H,d), 1.03(9H,s), 1.04(3H,d), 2.17-2.26(2H,m), |
| | 2.55-2.66(1H,m), 2.82(3H,s), 5.9(1H,d), 7.61(1H,d), 8.72 |
| | (1H,d), 9.00(1H,s) |
| I-195 | 0.83(3H,d), 1.06(3H,d), 2.68-2.72(1H,m), 2.81(3H,s), |
| | 3.40(3H,s), 4.07(2H,d), 5.72(1H,d), 7.62(1H,d), 8.74(1H,d), |
| | 9.03(1H,s) |
| I-199 | 0.82(3H,d), 0.92(3H,t), 1.03(3H,d), 1.30-1.40(2H,m), 1.56- |
| | 1.66(2H,m), 2.21-2.38(2H,m), 2.62-2.68(1H,m), 2.81(3H,s), |
| | 5.79(1H,d), 7.59(1H,d), 8.72(1H,d), 9.02(1H,s) |
| I-200 | 0.80-0.85(3H,d,3H,d), 0.92-0.99(3H,t,3H,t), 1.04-1.13 |
| | (3H,d,3H,d), 1.97-2.26(2H,m,2H,m), 2.62-2.83(1H,m,1H,m), |
| | 2.89(3H,s), 2.95(3H,s), 4.15(1H,t), 4.28(1H,t), 5.60(1H,d), |
| | 5.92(1H,d), 7.60-7.62(1H,d,1H,d), 8.73-8.76(1H,d,1H,d), |
| | 8.98(1H,s), 9.08(1H,s), diastereomer mixture |
| I-201 | 0.83(3H,d), 1.05(3H,d), 2.84(3H,s), 2.59-2.99(3H,m), |
| | 3.57-3.73(2H,m), 5.77(1H,d), 7.60(1H,d), 8.73(1H,d), |
| | 9.00(1H,s) |
| I-203 | 0.78(3H,d), 0.92-1.78(5H,m), 1.07(3H,d), 2.53-2.66(1H,m), |
| | 2.76(3H,s), 4.03-4.11(2H,m), 5.25-5.35(1H,m), 7.58(1H,d), |
| | 8.71(1H,d), 8.90(1H,bs) |
| I-206 | 0.87(3H,d), 1.14(3H,d), 2.69-2.79(1H,m), 2.88(3H,s), 5.85 |
| | (1H,d), 6.39(1H,s), 7.24-7.36(5H,m), 7.63(1H,d), 8.75(1H,d), |
| | 9.03(1H,s) |
| I-208 | 0.83(3H,d), 1.05(3H,d), 2.10(3H,s), 2.61-2.77(1H,m), 2.84 |
| | (3H,s), 5.78(1H,d), 7.60(1H,d), 8.73(1H,d), 9.01(1H,s) |
| I-210 | 0.83(3H,d), 1.03(3H,d), 2.08-2.18(2H,m), 2.37-2.76(3H,m), |
| | 2.82(3H,s), 3.57-3.70(2H,m), 5.82(1H,d), 7.60(1H,d), |
| | 8.72(1H,d), 8.99(1H,s) |
| I-211 | 0.82(3H,d), 1.03(3H,d), 2.31-2.50(4H,m), 2.58-2.70(1H,m), |
| | 2.82(3H,s), 4.94-5.09(1H,dd,1H,dd), 5.78-5.91(1H,d,1H,m), |
| | 7.590(1H,d), 8.71(1H,d), 9.00(1H,s) |
| I-213 | 0.85(3H,d), 1.08(3H,d), 1.91(3H,s), 2.65-2.77(1H,m), 2.82 |
| | (3H,s), 4.91(1H,s), 5.13(1H,s), 5.821(1H,d), 7.61(1H,d), |
| | 8.74(1H,d), 9.00(1H,s) |
| I-214 | 0.80-1.77(16H,m,16H,m), 2.57-2.68(2H,m,2H,m), 2.83(3H,s), |
| | 2.85(3H,s), 5.85-5.91(1H,d,1H,d), 7.59(1H,d,1H,d), 8.71 |
| | (1H,d,1H,d), 8.99(1H, s ,1H,s), diastereomer mixture |
| I-215 | 0.78-1.83(3H,d,3H,d,3H,t,3H,t,3H,d,3H,d,3H,d,3H,d, |
| | 2H,m,2H,m), 2.46-2.74(1H,m,1H,m,1H,m,1H,m), 2.84(3H,s), |
| | 2.86(3H,s), 5.86-5.94(1H,d,1H,d), 7.58-7.60(1H,d,1H,d), |
| | 8.70-8.72(1H,d,1H,d), 9.00(1H,s,1H,s), diastereomer mixture |
| I-217 | 0.82-0.86(3H,d,3H,d), 1.02-1.04(3H,d,3H,d), 1.11(3H,d), |
| | 1.17(3H,d), 2.15-3.05(2H,m,2H,m,1H,m,1H,m,1H,m,1H,m), |
| | 2.80(3H,s), 2.82(3H,s), 5.81-5.88(1H,d,1H,d), 7.60-7.62 |
| | (1H,d,1H,d), 8.73-8.74(1H,d,1H,d), 8.98(1H,s), 9.00(1H,s), |
| | diastereomer mixture |
| I-221 | 0.79(3H,d), 1.10(3H,d), 1.38(1H,d), 1.56(3H,s), 2.02(1H,d), |
| | 2.72-2.84(1H,m), 3.03(3H,s), 5.66(1H,d), 7.60(1H,d), |
| | 8.73(1H,d), 9.06(1H,s) |
| I-222 | 0.81(3H,d), 1.07(3H,d), 1.34(3H,s), 1.37(1H,d), 2.19(1H,d), |
| | 2.66-2.77(1H,m), 2.96(3H,s), 5.77(1H,d), 7.66(1H,d), |
| | 8.77(1H,d), 9.03(1H,s) |
| I-223 | 1.14(3H,d), 1.20(3H,d), 2.91(3H,s), 2.75-2.99(1H,m), |
| | 7.01-7.34(6H,m), 7.57(1H,d), 8.56(1H,s), 8.72(1H,d) |
| I-228 | 0.96-1.00(3H,d,3H,d), 2.16-2.37(3H,m), 2.86(3H,s), |
| | 7.00-7.33(6H,m), 7.57(1H,d), 8.55(1H,s), 8.72(1H,d) |
| I-231 | 0.74(3H,d), 1.26(3H,d), 2.72-2.83(1H,m), 3.09(3H,s), 6.10 |
| | (2H,bs), 7.14-7.38(5H,m), 7.63(1H,d), 8.76(1H,d), 8.99(1H,s) |
| I-232 | 0.74(3H,d), 1.10(3H,d), 2.36(3H,s), 2.58-2.61(1H,m), |
| | 3.17(3H,s), 5.42(1H,d), 6.71-7.07(5H,m), 7.59(1H,d), |
| | 8.68(1H,d), 8.83(1H,s) |
| I-233 | 0.74(3H,d), 1.06-1.10(6H,m), 2.30(3H,s), 2.52-2.63(1H,m), |
| | 3.55-3.73(2H,m), 5.43(1H,d), 6.66-7.05(5H,m), 7.60(1H,d), |
| | 8.68(1H,d), 8.80(1H,s) |
| I-234 | 0.80(3H,d), 1.01(3H,d), 2.16(3H,s), 2.64-2.69(1H,m), 2.88 |
| | (3H,s), 4.63(2H,m), 5.70(1H,d), 6.63(1H,d), 7.01(1H,dd), |
| | 7.06(1H,dd), 7.57(1H,d), 8.71(1H,d), 8.99(1H,s) |
| I-235 | 0.78(3H,d), 0.98(3H,d), 2.63-2.70(1H,m), 2.93(3H,s), 4.73 |
| | (2H,s), 5.69(1H,d), 6.85-7.31(4H,m), 7.56(1H,d), |
| | 8.70(1H,d), 8.99(1H,s) |
| I-236 | 0.79(3H,d), 0.99(3H,d), 2.65-2.68(1H,m), 2.91(3H,s), |
| | 4.71(2H,m), 5.67(1H,d), 6.82(1H,d), 7.09(1H,dd), 7.30(1H,d), |
| | 7.57(1H,d), 8.71(1H,d), 8.98(1H,s) |
| I-241 | 0.79(3H,d), 0.99(3H,d), 2.61-2.71(1H,m), 2.89(3H,s), 4.61 |
| | (2H,m), 5.68(1H,d), 6.79-6.93(4H,m), 7.55(1H,d), 8.72(1H,d), |
| | 8.99(1H,s) |
| I-244 | 0.78(3H,d), 1.00(3H,d), 2.63-2.70(1H,m), 2.88(3H,s), |
| | 4.59(2H,m), 5.68(1H,d), 6.74-6.83(4H,m), 7.56(1H,d), |
| | 8.70(1H,d), 9.00(1H,s) |
| I-247 | 0.77(3H,d), 1.00(3H,d), 2.64-2.67(1H,m), 2.85(3H,s), |
| | 4.62(2H,m), 5.68(1H,d), 6.75-7.12(4H,m), 7.56(1H,d), |
| | 8.70(1H,d), 8.98(1H,s) |
| I-248 | 0.77(3H,d), 0.99(3H,d), 2.67(1H,m), 2.86(3H,s), 4.61(2H,m), |
| | 5.66(1H,d), 6.79(2H,d), 7.15(2H,d), 7.57(1H,d), 8.71(1H,d), |
| | 8.99(1H,s) |
| I-249 | 0.78(3H,d), 1.00(3H,d), 1.16(3H,t), 2.53(2H,q), 2.57-2.70 |
| | (1H,m), 2.88(3H,s), 4.61(2H,m), 5.68(1H,d), 6.78(2H,d), |
| | 7.03(2H,d), 7.57(1H,d), 8.70(1H,d), 9.00(1H,s) |
| I-250 | 0.80(3H,d), 1.02(3H,d), 1.27(9H,s), 2.70(1H,m), 2.91(3H,s), |
| | 4.64(2H,m), 5.70(1H,d), 6.82(2H,d), 7.25(2H,d), 7.58(1H,d), |
| | 8.72(1H,d), 9.03(1H,s) |
| I-251 | 0.84(3H,d), 1.05(3H,d), 2.47(3H,s), 2.68-2.83(1H,m), 2.93 |
| | (3H,s), 4.67(2H,m), 5.72(1H,d), 6.85(2H,d), 7.21(2H,d), |
| | 7.62(1H,d), 8.76(1H,d), 9.05(1H,s) |
| I-253 | 0.80(3H,d), 1.03(3H,d), 2.65-2.69(1H,m), 2.87(3H,s), |
| | 4.76-4.88(2H,m), 5.69(1H,d), 7.33(1H,s), 7.54(1H,s), |
| | 7.60(1H,d), 8.74(1H,d), 8.99(1H,s) |
| I-254 | 0.80(3H,d), 1.01(3H,d), 2.64-2.70(1H,m), 2.78(3H,s), |
| | 4.70(2H,m), 5.72(1H,d), 7.06-7.36(4H,m), 7.58(1H,d), |
| | 8.72(1H,d), 8.99(1H,s) |
| I-255 | 0.74(3H,d), 0.95(3H,d), 2.60-2.65(1H,m), 2.82(3H,s), |
| | 4.57(2H,m), 5.62(1H,d), 6.68-7.26(4H,m), 7.52(1H,d), |
| | 8.66(1H,d), 8.94(1H,s) |
| I-256 | 0.78(3H,d), 1.01(3H,d), 2.61-2.68(1H,m), 2.88(3H,s), |
| | 3.71(2H,m), 5.69(1H,d), 7.15-7.41(5H,m), 7.56(1H,d), |
| | 8.71(1H,d), 8.98(1H,s) |
| I-257 | 0.71(3H,d), 1.06(6H,d), 1.15(3H,d), 2.23(3H,s), 2.49-2.55 |
| | (1H,m), 4.32(2H,m), 5.38(1H,d), 6.64-7.11(5H,m), 7.54(1H,d), |
| | 8.63(1H,d), 8.71(1H,s) |
| I-258 | 0.80(3H,d), 1.0(3H,d), 2.6-2.7(1H,m), 2.7(3H,s), 3.4(2H,m), |
| | 5.7(1H,d), 6.8-7.1(3H,m), 7.5(1H,d), 8.7(1H,d), 8.9(1H,s) |
| I-259 | 0.74(3H,d), 0.98-1.10(6H,m), 2.83(1H,m), 3.2-3.5(2H,m), |
| | 4.68(2H,m), 5.4(1H,d), 6.8-7.4(5H,m), 7.56(1H,d), 8.73 |
| | (1H,d), 8.96(1H,s) |
| I-260 | 0.70-1.44(8H,m,8H,m),2.35(1H,m,1H,m),2.76(3H,s),2.78(3H,s), |
| | 3.66(2H,m, 2H,m),5.80(1H,d),5.87(1H,d),7.14-7.28(5H,m,5H,m), |
| | 7.56(1H,m,1H,m),8.68(1H,m,1H,m),8.93(1H,s),8.95(1H,s), |
| | diastereomer mixture |
| I-262 | 0.73(3H,t), 0.86(3H,t), 1.07-1.38(4H,m), 2.28(1H,m), |
| | 2.75(3H,s), 3.65(2H,m), 6.03(1H,d), 7.17-7.28(5H,m), |
| | 7.56(1H,d), 8.68(1H,d), 8.94(1H,s) |
| I-263 | 0.74(3H,t), 0.86(3H,t), 1.07-1.43(4H,m), 2.35(1H,m), |
| | 2.86(3H,s), 4.64(2H,m), 5.95(1H,d), 6.86(2H,d), 6.92(1H,t), |
| | 7.21(2H,t), 7.56(1H,d), 8.70(1H,d), 8.98(1H,s) |
| I -266 | 0.82(3H,d), 0.89 (3H,t), 1.03(3H,d), 1.18-1.74(6H,m), |
| | 2.19-2.39(2H,m), 2.58-2.71(1H,m), 2.81(3H,s), 5.81(1H,d), |
| | 7.59(1H,d), 8.71(1H,d), 9.00(1H,s) |
| I-267 | 2.68(3H,s), 2.85(6H,s), 6.39(1H,s), 7.18-7.35(5H,m), |
| | 7.53(1H,d), 8.67(1H,d), 8.79(1H,s) |
| I-268 | 0.82(3H,d), 1.03(3H,d), 1.25(3H,t), 2.44-2.80(5H,m), |
| | 2.85(3H,s), 4.13(2H,q), 5.77(1H,d), 7.58(1H,d), 8.71(1H,d), |
| | 9.00(1H,s) |
| I-269 | 0.81-1.62(17H,m), 2.19-2.38(2H,m), 2.59-2.75(1H,m), |
| | 2.82(3H,s), 5.81(1H,d), 7.59(1H,d), 8.71(1H,d), 9.00(1H,s) |

Test Examples of the present invention are described below.

### Test Example I

Upland field soil was put into a 1/170,000 ha pot, and seed of various plants were sown. Then, when the plants reached predetermined leaf stages ((1) barnyardgrass : 1.3 to 2.8 leaf stages, (2) crabgrass : 1.0 to 2.8 leaf stages, (3) green foxtail : 1.3 to 2.6 leaf stages, (4) redroot pigweed : 0.1 to 1.2 leaf stages, (5) prickly sida : 0.1 to 1.2 leaf stages, (6) velvetleaf : 0.1 to 1.2 leaf stages, (7) tall morningglory : 0.1 to 1.5 leaf stages , (8) rice : 1.2 to 2.6 leaf stages, (9) wheat : 1.3 to 2.4 leaf stages, (10) corn , 2.1 to 3.2 leaf stages, (11) soybean : 0.1 to 0.4 leaf stage), a wettable powder or emulsifiable concentrate having the compound of the present invention formulated in accordance with a usual formulation method, was weighed so that the active ingredient would be a predetermined amount, and diluted with water in an amount of 500 L/ha. To the diluted solution, 0.1% (v/v) of agricultural spreader was added. The herbicide thus adjusted was applied by a small size sprayer for foliage treatment.

On the 18th to 23rd days after the application of the herbicide, the growth of the respective plants was visually observed, and the herbicidal effects were evaluated by the growth controlling degrees (%) ranging from 0 (equivalent to the non-treated plot) to 100 (complete kill), whereby the results shown in Table 3, were obtained.

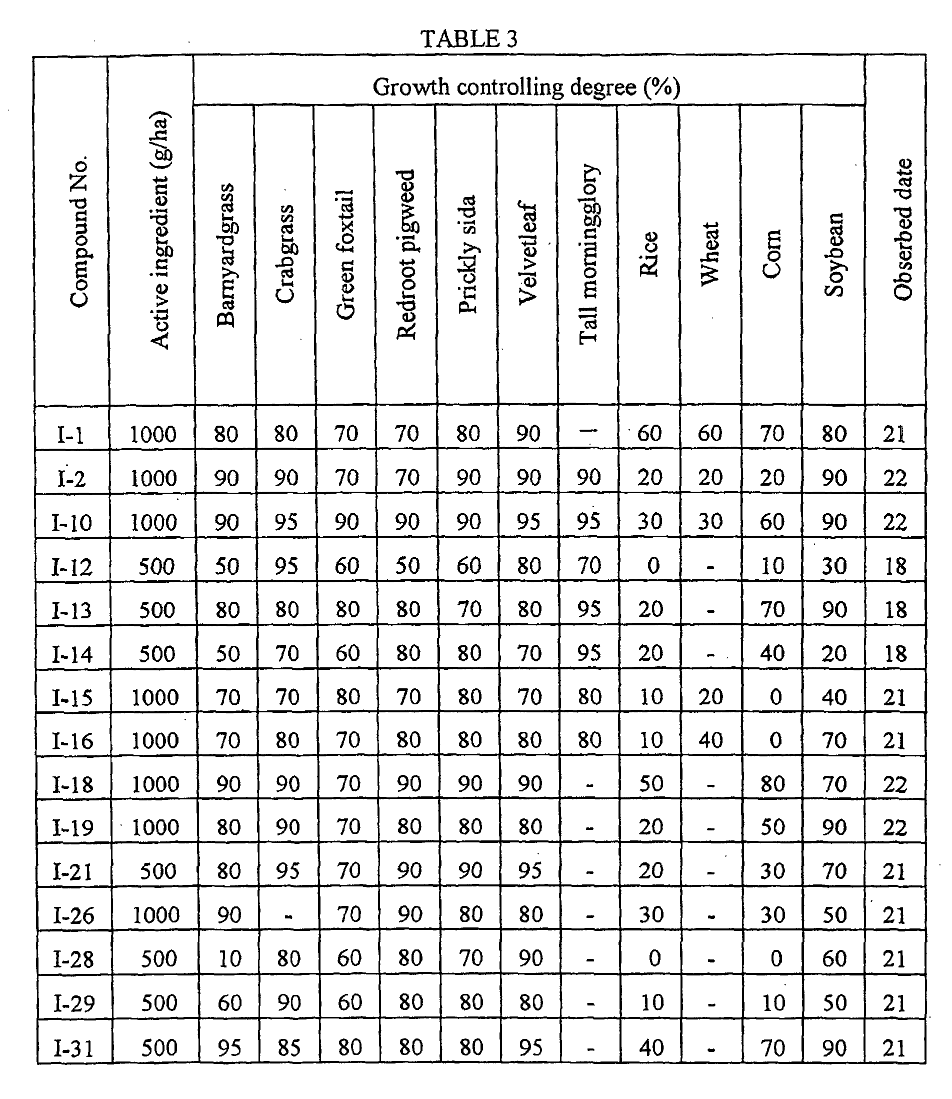

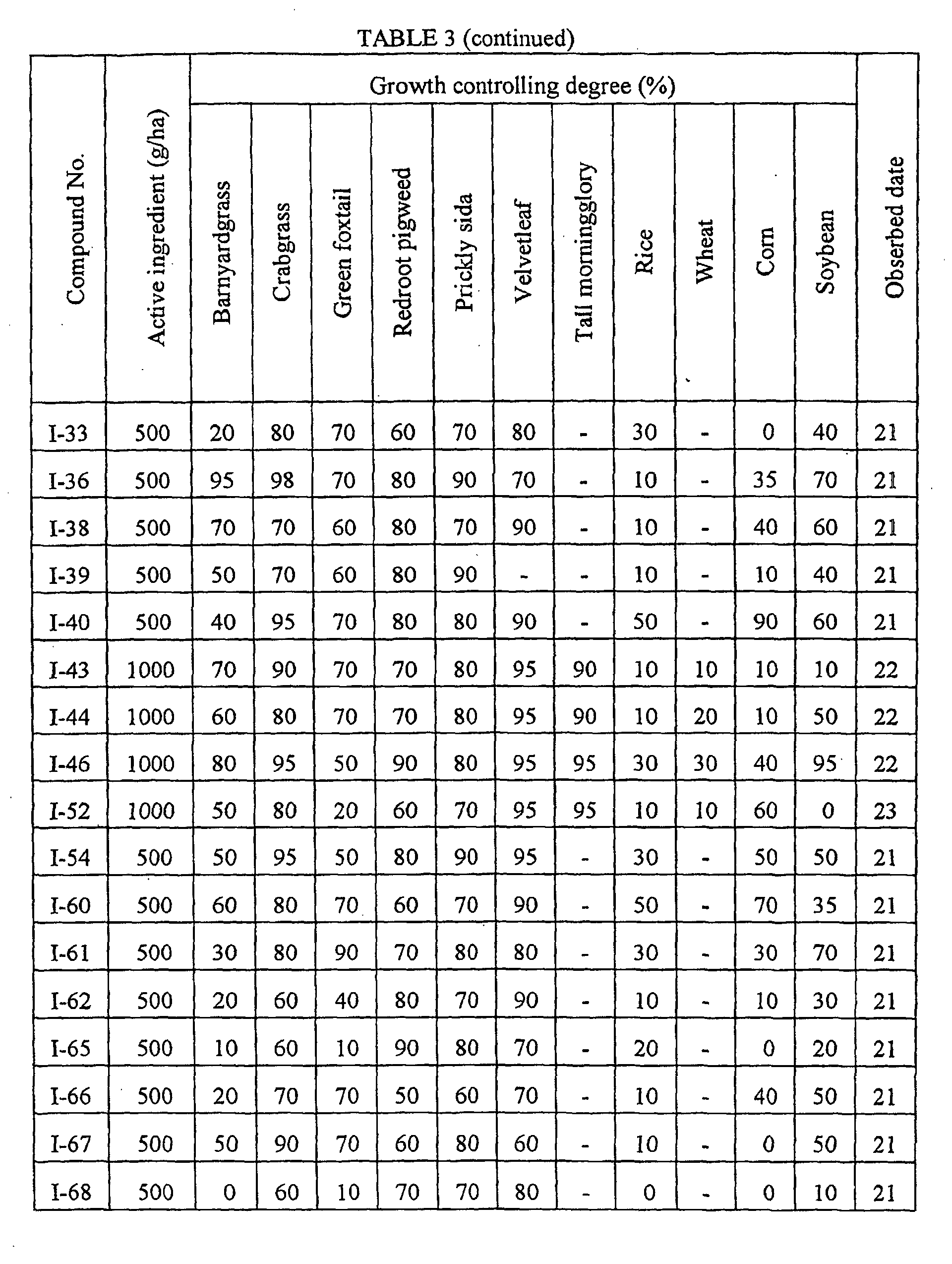

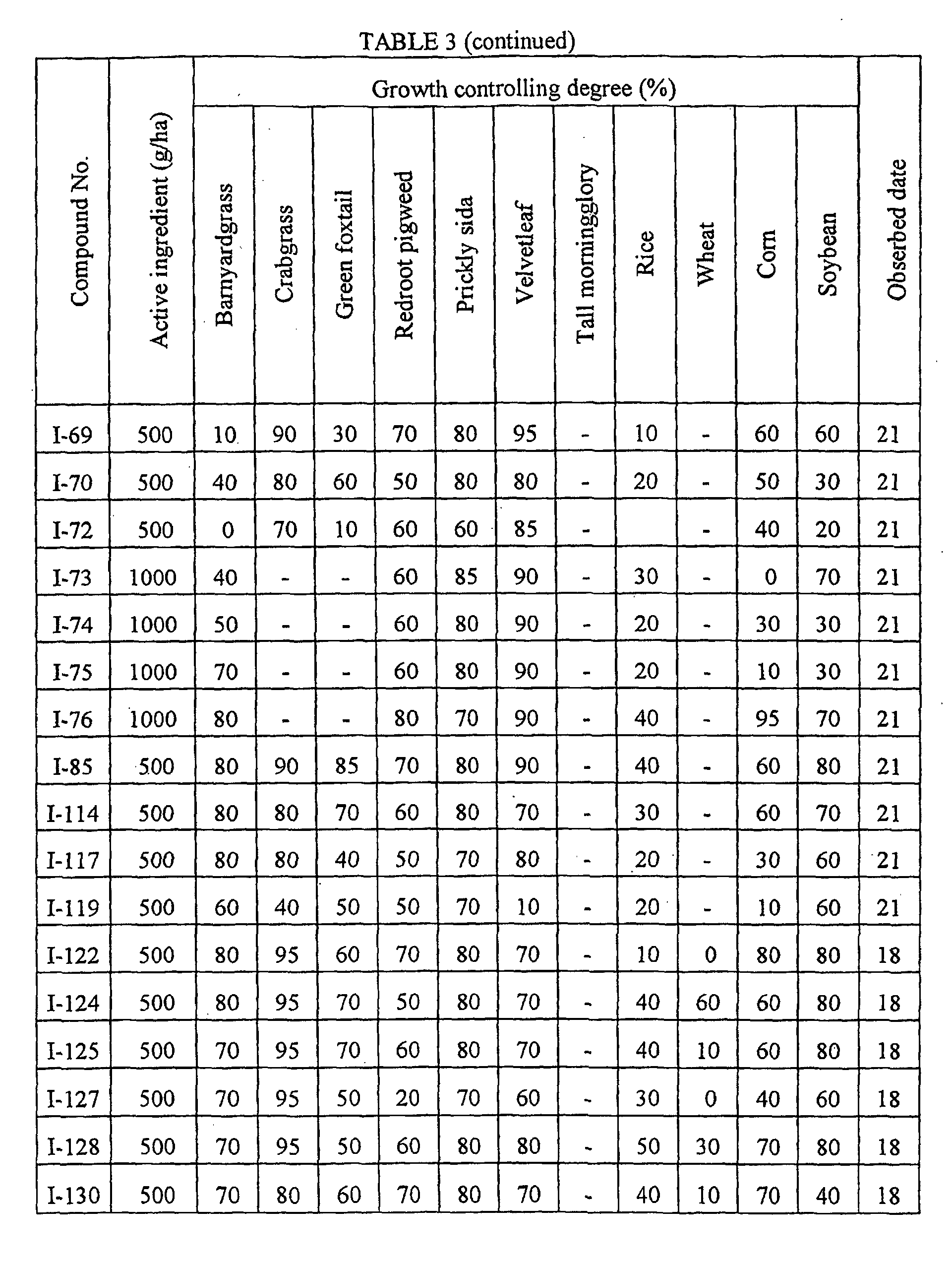

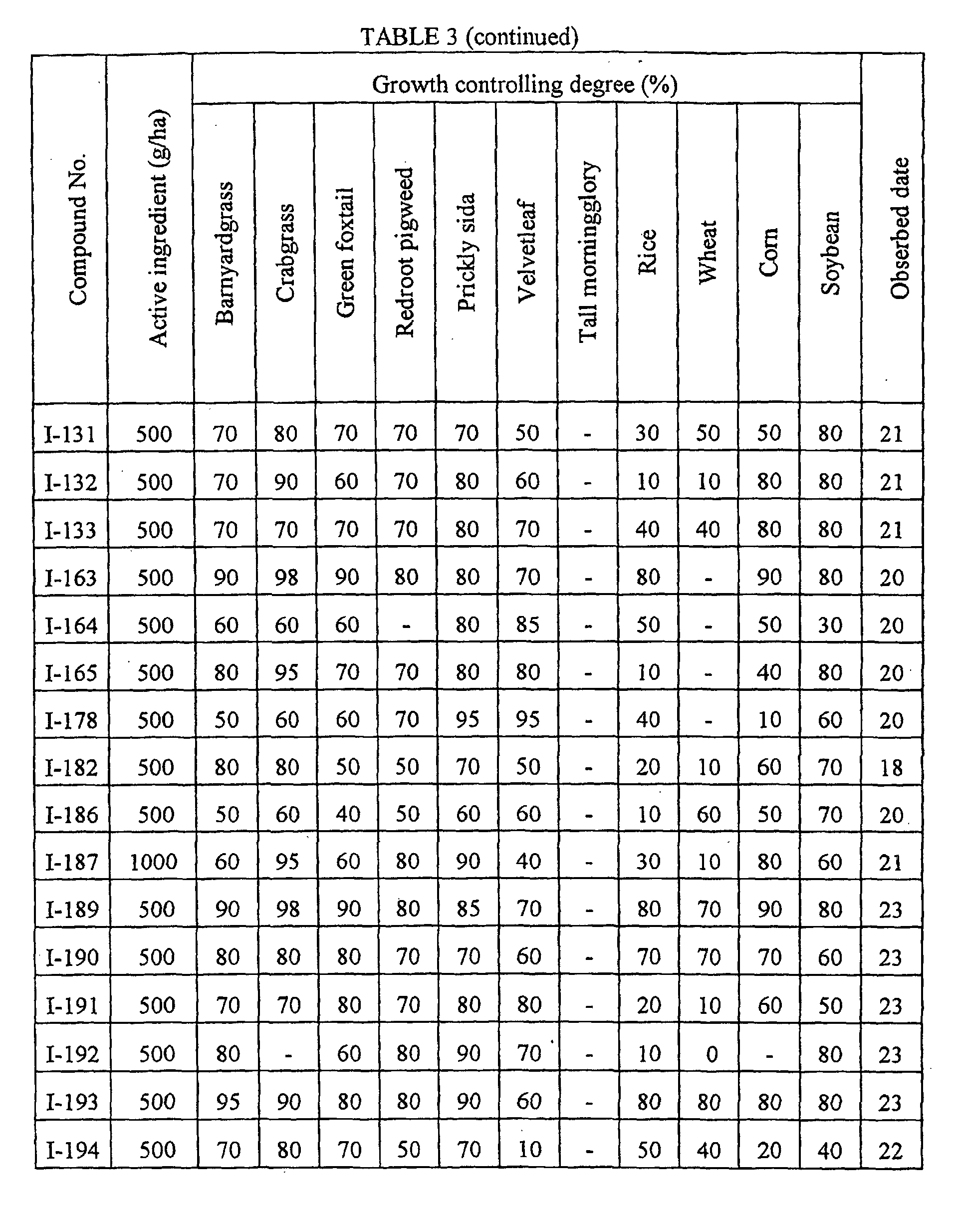

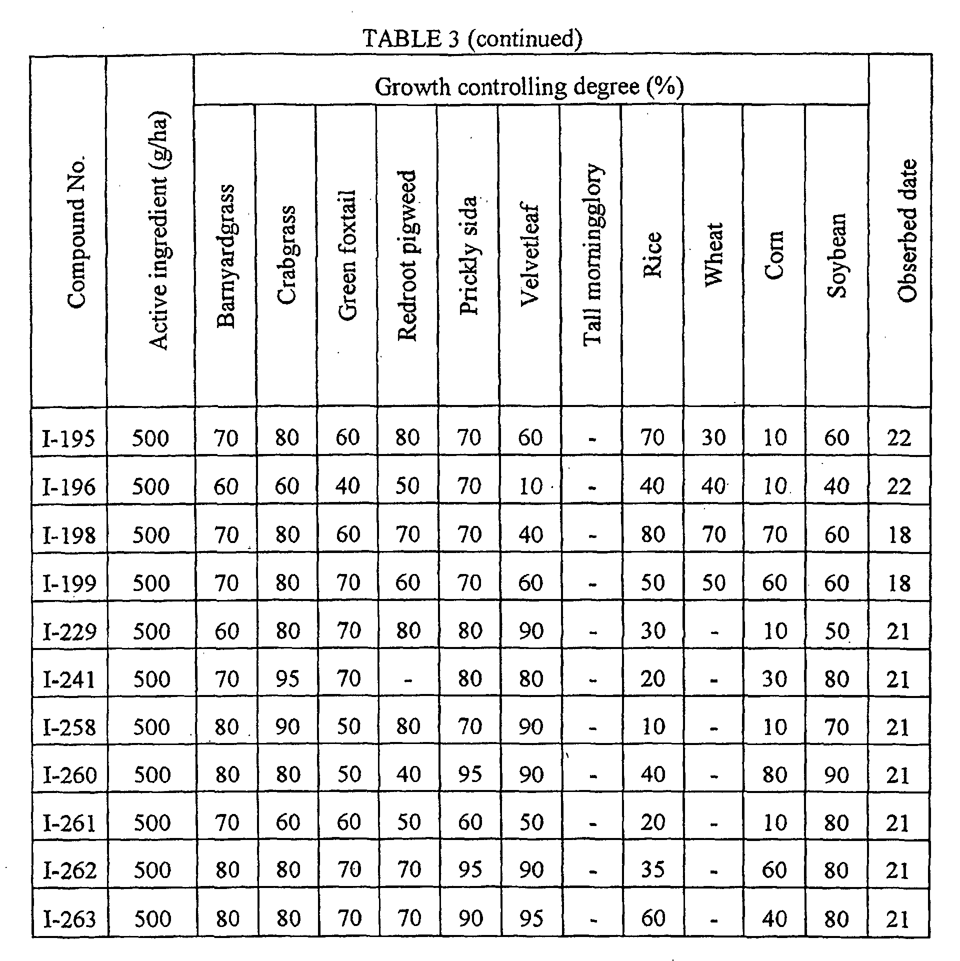

### Test Example 2

Upland field soil was put into a 1/170,000 ha pot, and seed of various plants (barnyardgrass, crabgrass, green foxtail, redroot pigweed, prickly sida, velvetleaf, rice, wheat, corn, soybean) were sown. On the 1st day after the sowing, a wettable powder or emulsifiable concentrate having the compound of the present invention formulated in accordance with a usual formulation method, was weighed so that the active ingredient would be a predetermined amount, and diluted with water in an amount of 1,500 L/ha. The herbicide thus adjusted was applied by a small size sprayer for soil surface treatment.

On the 20th to 28th days after the application of the herbicide, the growth of the respective plants was visually observed, and the herbicidal effects were evaluated by the growth controlling degrees (%) ranging from 0 (equivalent to the non-treated plot) to 100 (complete kill), whereby the results shown in Table 4, were obtained.

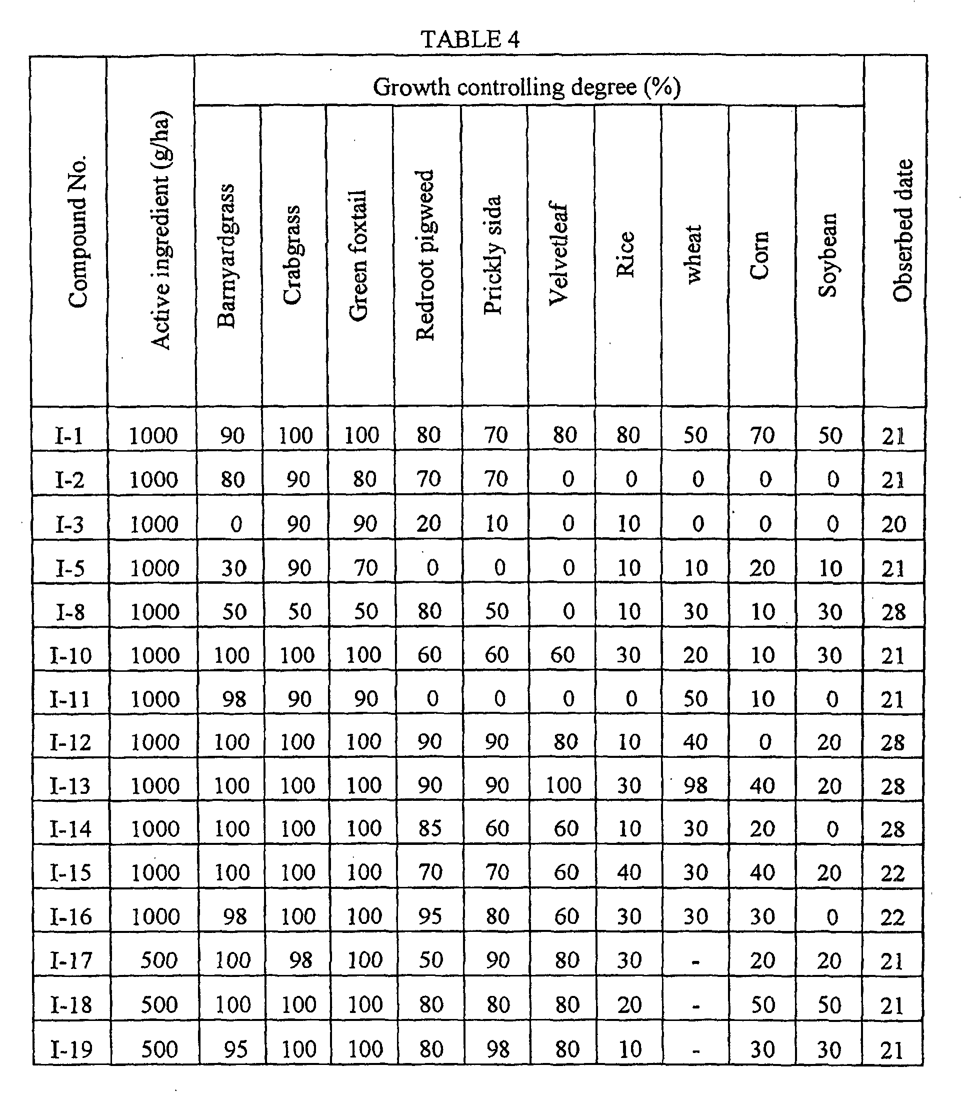

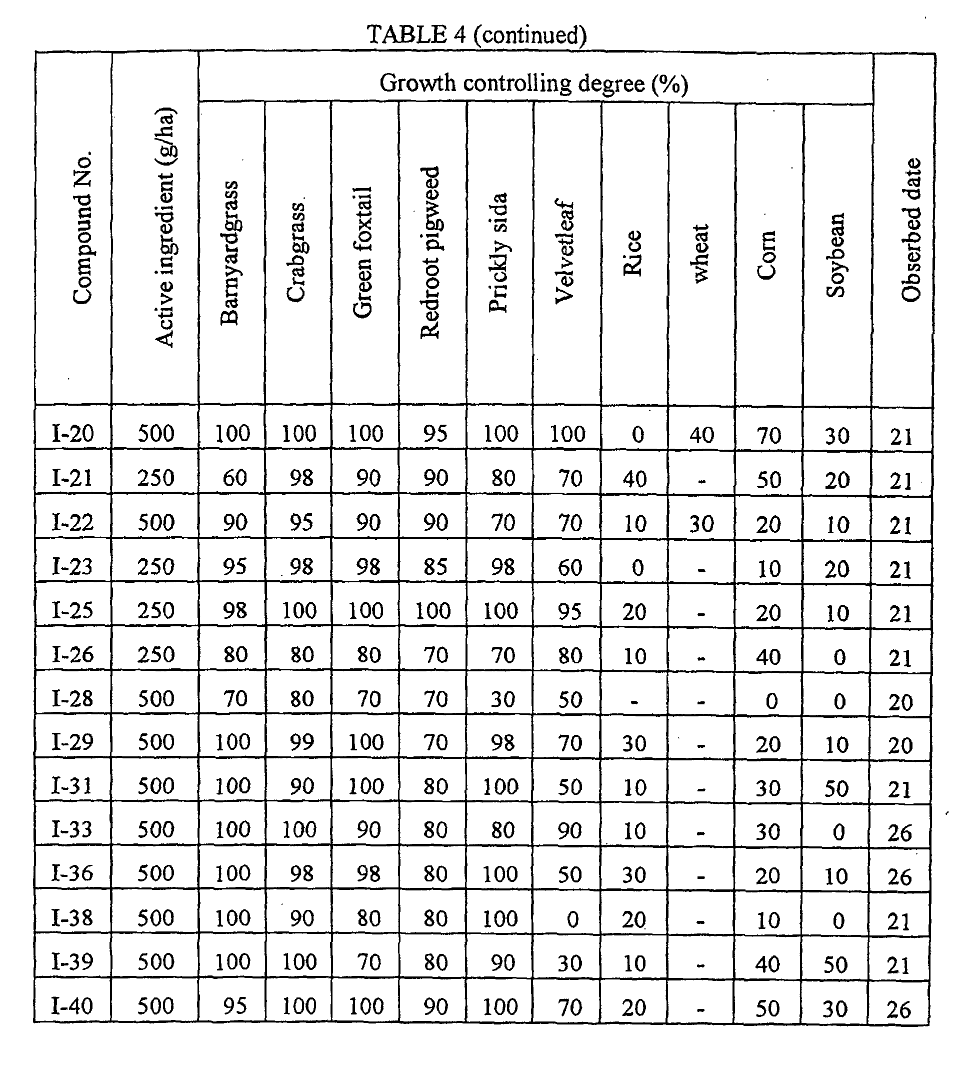

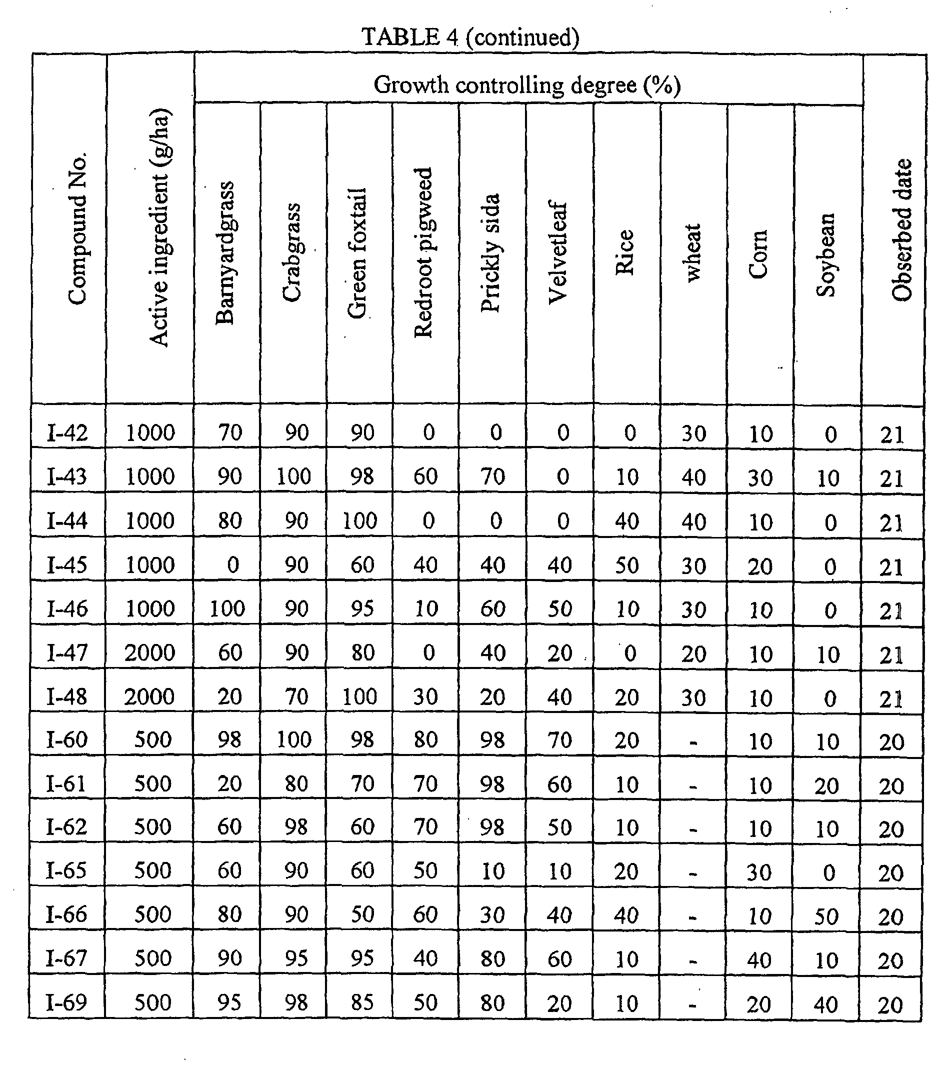

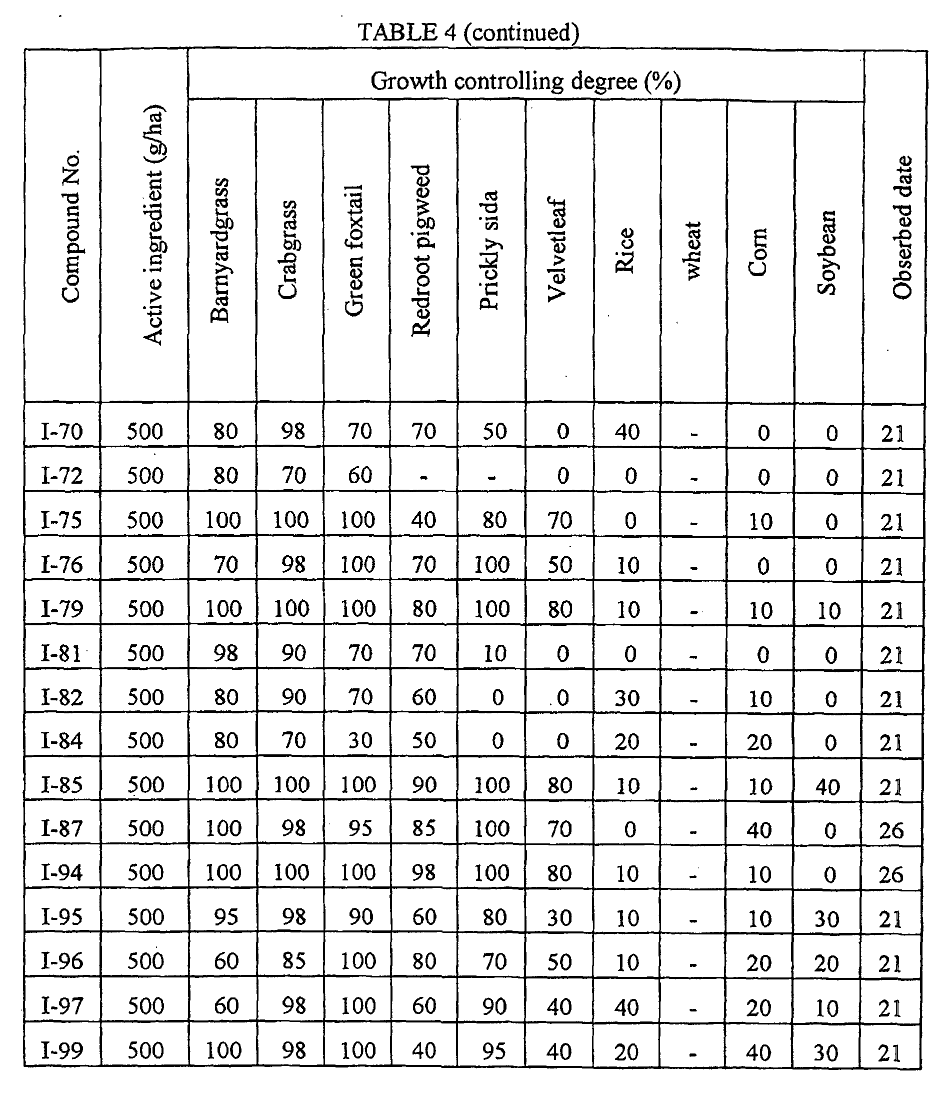

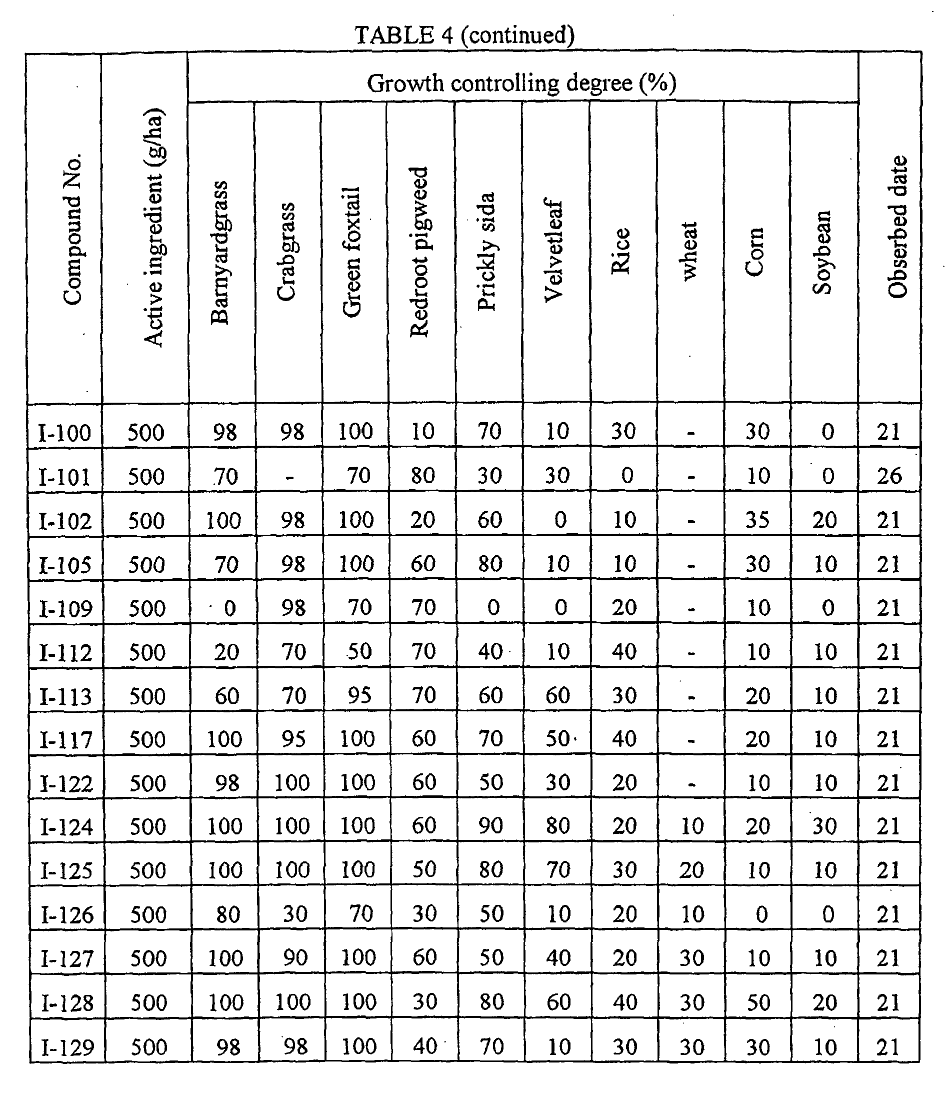

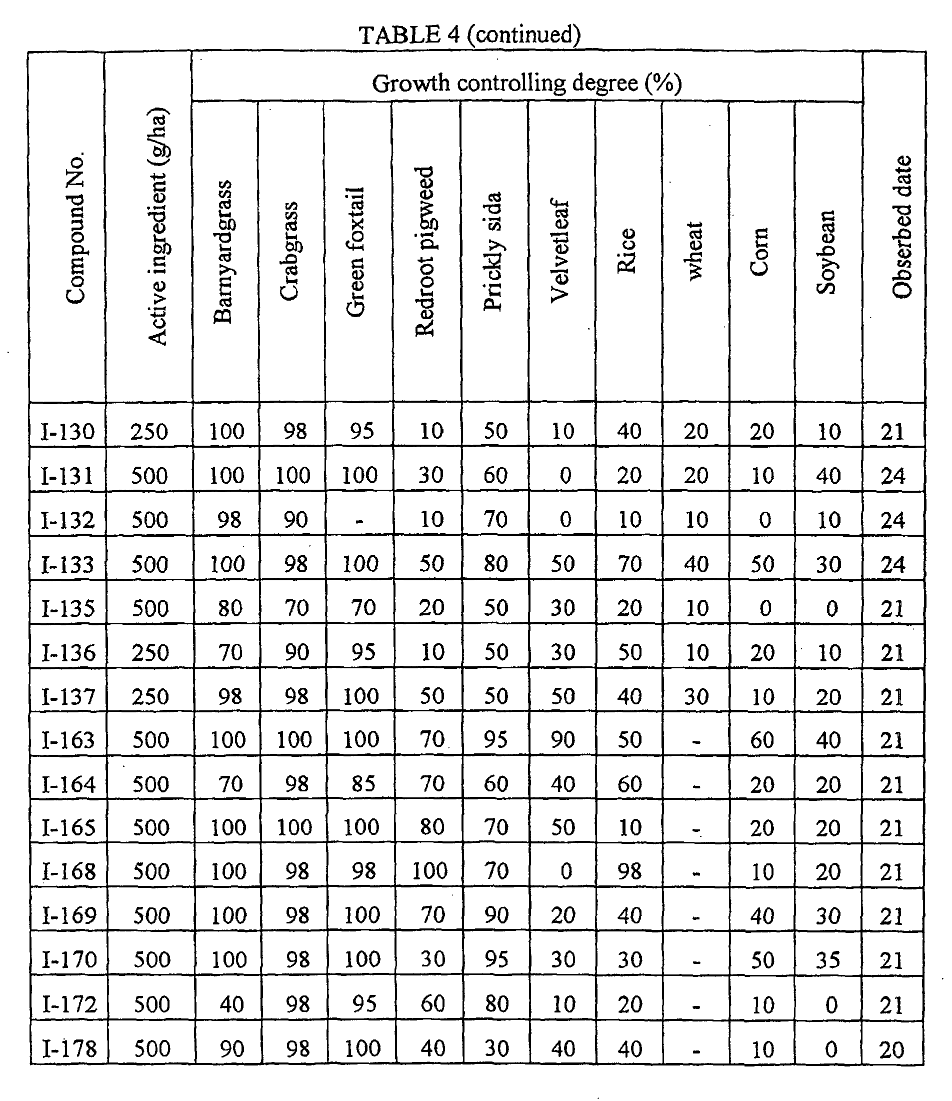

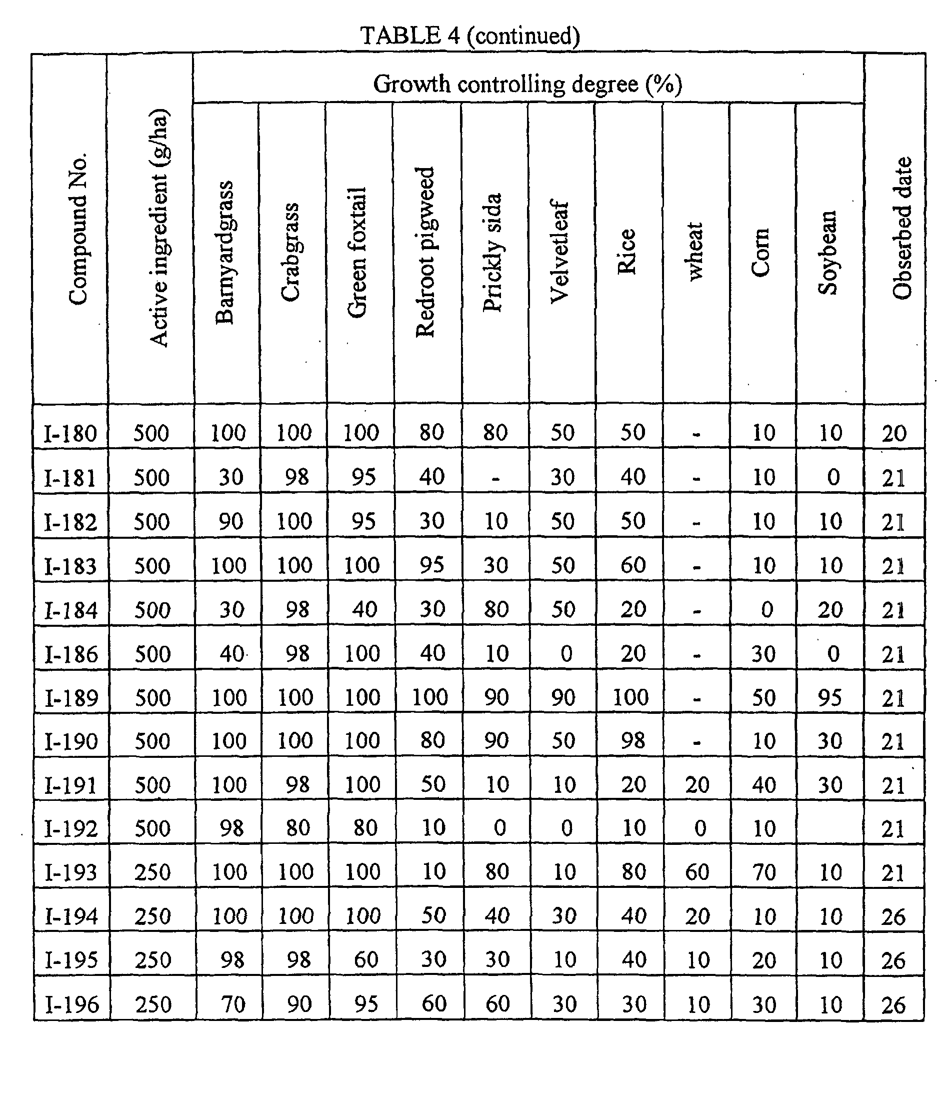

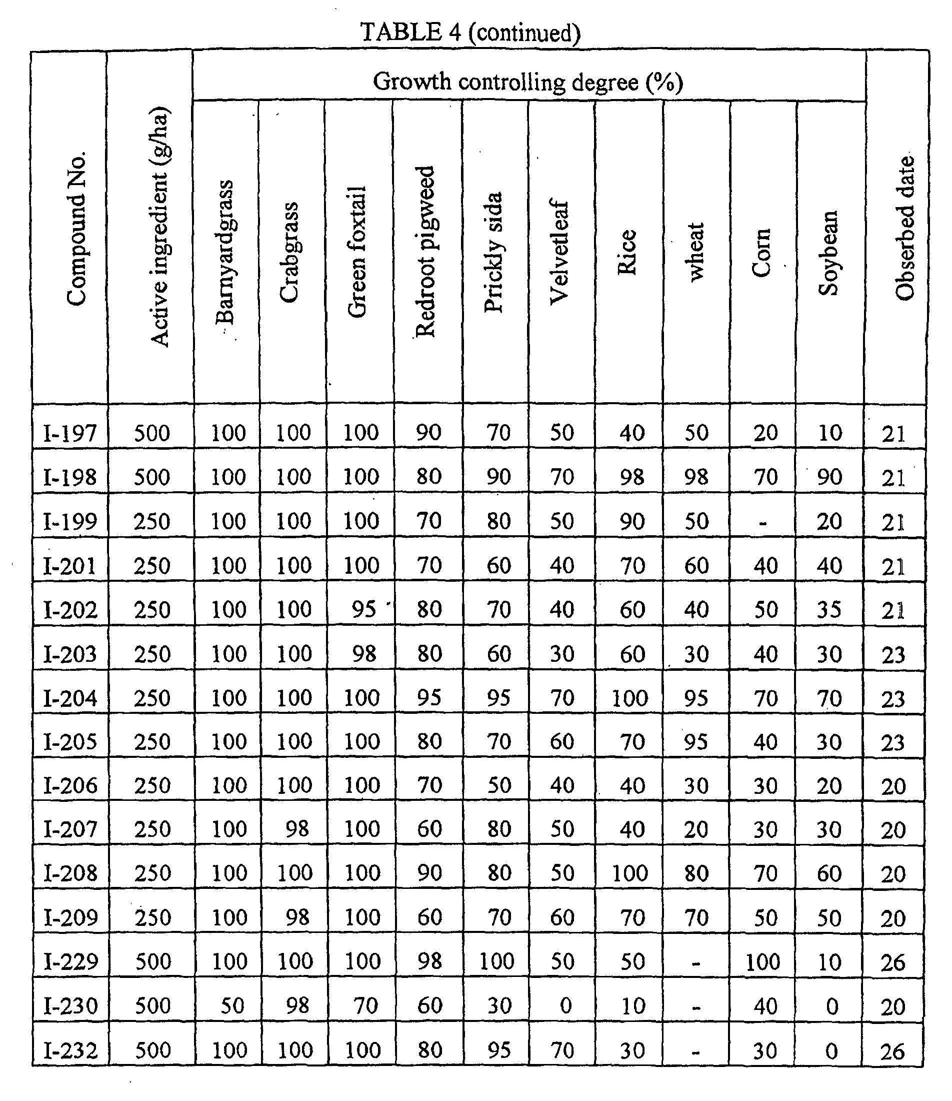

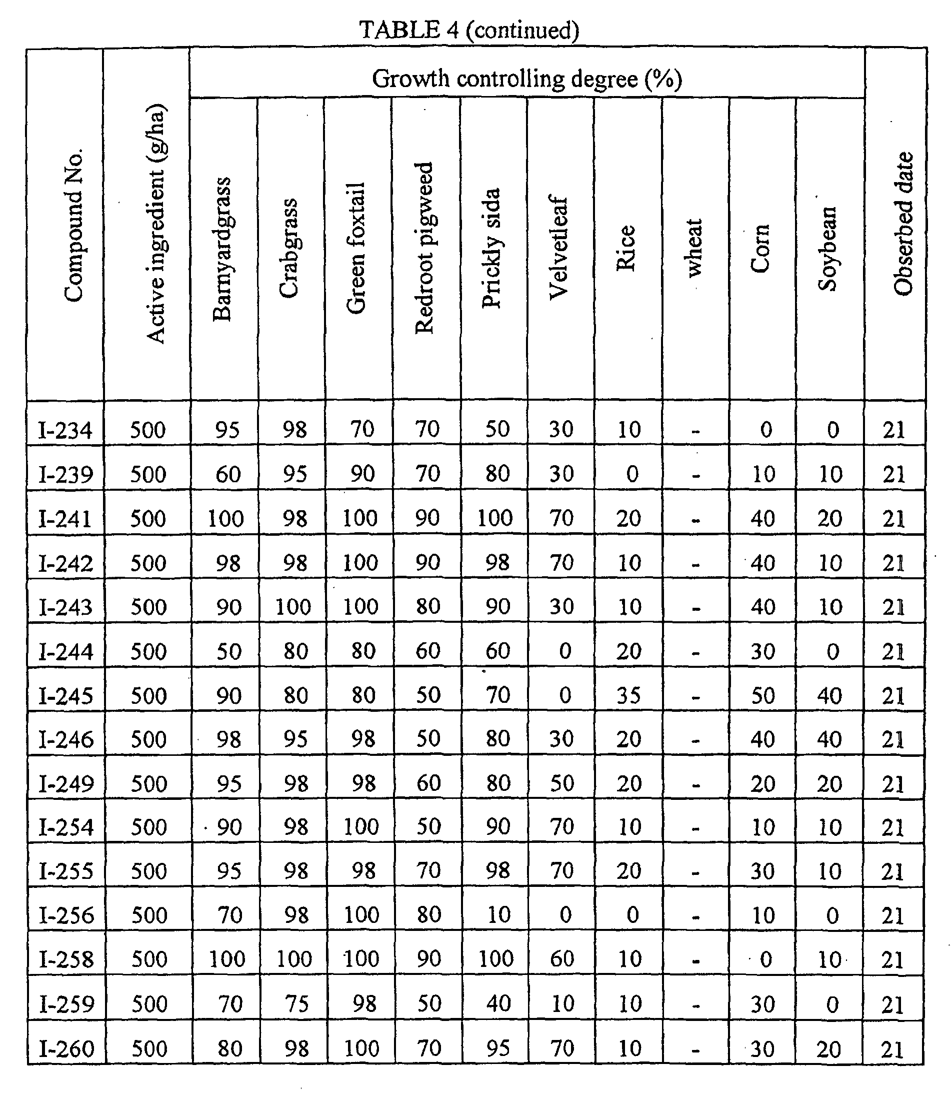

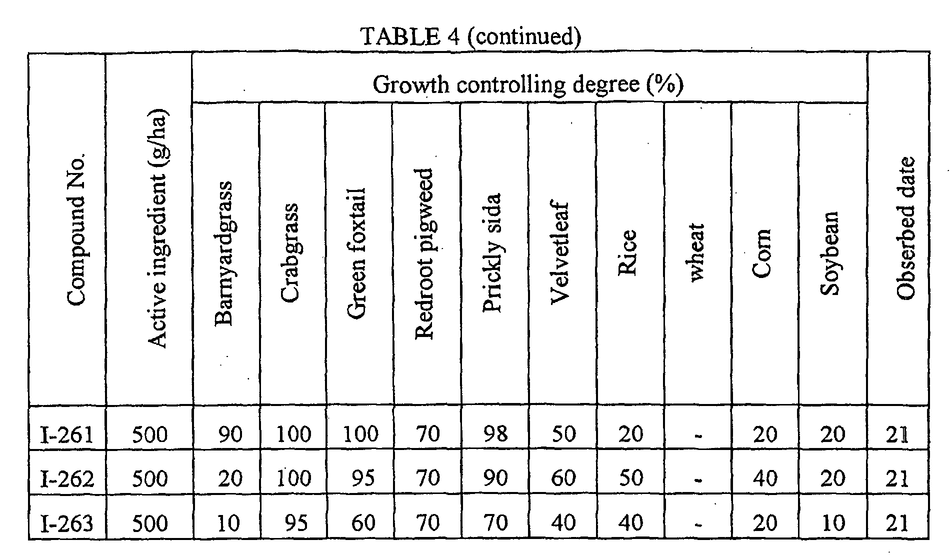

### Test Example 3

Paddy field soil was put into a 1/1,000,000 ha pot, and seed of barnyardgrass and japanese bulrush were sown and slightly covered with soil. Then, the pot was left to stand still in a greenhouse in a state where the depth of flooding water was from 0.5 to 1 cm, and one day or two days later, tubers of japanese ribbon wapato were planted. Thereafter, the depth of flooding water was maintained at a level of from 3 to 4 cm, and when barnyardgrass and japanese bulrush reached a 0.5 leaf stage and japanese ribbon wapato reached a primary leaf stage, an aqueous diluted solution of a wettable powder or emulsifiable concentrate having the compound of the present invention formulated in accordance with a usual formulation method, was uniformly applied under submerged conditions by a pipette so that the dose of the active ingredient would be at a predetermined level.

On the other hand, paddy field soil was put into a 1/1,000,000 ha pot and puddled and leveled, and the depth of flooding water was from 3 to 4 cm. Next day, rice (var. Nihonbare) of 2 leaf stage was transplanted in a depth of 3 cm. On the 4th day after the transplantation, the compound of the present invention was applied in the same manner as described above.

On the 14 days after the application of the herbicide, the growth of barnyard grass, japanese burlrush and japanese ribbon wapato was visually observed and on the 21 st day after the application of the herbicide, the growth of rice was visually observed, and the herbicidal effects were evaluated by the growth-controlling degrees (%) ranging from 0 (equivalent to the non-treated plot) to 100 (complete kill), whereby the results shown in Table 5 were obtained.

**TABLE 5**

| Comp. No. | Active ingredient g/ha | Growth controlling degree (%) | | | |
|---|---|---|---|---|---|
| | | Barnyardgrass | Japanese bulrush | Japanese ribbon wapato | Rice |
| I-1 | 500 | - | 98 | 95 | 60 |
| I-2 | 250 | 100 | 90 | 50 | 20 |
| I-3 | 1000 | 90 | 98 | 30 | 20 |
| I-5 | 500 | 60 | 90 | 50 | 20 |
| I-7 | 500 | 95 | 70 | 0 | 20 |
| I-8 | 500 | 90 | 90 | 10 | 0 |
| I-9 | 500 | 100 | 10 | 30 | 0 |
| I-10 | 500 | 100 | 98 | 10 | 10 |
| I-11 | 500 | 60 | 90 | 30 | 10 |
| I-12 | 500 | 100 | 100 | 70 | 30 |
| I-13 | 500 | 100 | 95 | 90 | 30 |
| I-14 | 500 | 100 | 98 | 30 | 10 |
| I-15 | 250 | 100 | 95 | 100 | 10 |
| I-16 | 250 | 100 | 90 | 30 | 10 |
| I-17 | 500 | - | 90 | 90 | 35 |
| I-18 | 500 | - | 90 | 90 | 10 |
| I-19 | 500 | - | 95 | 95 | 35 |
| I-20 | 500 | - | 90 | 90 | 40 |
| I-21 | 500 | - | 98 | 100 | 40 |
| I-22 | 500 | - | 95 | 95 | 0 |
| I-23 | 500 | - | 90 | 85 | 30 |
| I-24 | 500 | - | 95 | 100 | 40 |
| I-25 | 500 | - | 98 | 100 | 30 |
| I-26 | 500 | - | 90 | 10 | 20 |
| I-28 | 250 | 100 | 70 | 10 | 0 |
| I-29 | 250 | 100 | 70 | 10 | 10 |
| I-30 | 250 | 100 | 90 | 80 | 0 |
| I-31 | 250 | 100 | 95 | - | 20 |
| I-33 | 250 | 100 | 85 | 90 | 10 |
| I-35 | 250 | 100 | 95 | 70 | 0 |
| I-36 | 250 | 100 | 95 | 90 | 10 |
| I-37 | 250 | 100 | 50 | 10 | 0 |
| I-38 | 250 | 100 | 70 | 85 | 30 |
| I-39 | 250 | 100 | 95 | 85 | 10 |
| I-40 | 250 | 100 | 50 | 50 | 0 |
| I-42 | 500 | 70 | 95 | 10 | 30 |
| I-43 | 500 | 100 | 100 | 90 | 35 |
| I-44 | 500 | 90 | 95 | 100 | 30 |
| I-45 | 500 | 85 | 90 | 60 | 30 |
| I-46 | 500 | 100 | 100 | 90 | 10 |
| I-47 | 500 | 98 | 30 | 0 | 10 |
| I-48 | 500 | 100 | 30 | 0 | 10 |
| I-49 | 500 | 90 | 85 | - | 10 |
| I-50 | 500 | 95 | 95 | - | 10 |
| I-54 | 500 | 100 | 70 | 0 | 20 |
| I-56 | 500 | 95 | 70 | 90 | 20 |
| I-58 | 500 | 100 | 40 | 0 | 35 |
| I-59 | 500 | 90 | 10 | 60 | 10 |
| I-60 | 250 | 100 | 70 | 95 | 30 |
| I-61 | 250 | 95 | 0 | 20 | 10 |
| I-62 | 250 | 98 | 30 | 0 | 20 |
| I-65 | 125 | 98 | 90 | 60 | 0 |
| I-66 | 125 | 98 | 90 | 10 | 10 |
| I-67 | 125 | 100 | 98 | 60 | 0 |
| I-68 | 125 | 95 | 70 | 50 | 10 |
| I-69 | 125 | 95 | 70 | 40 | 0 |
| I-70 | 125 | 80 | 70 | 40 | 10 |
| I-71 | 125 | 90 | 90 | 40 | 0 |
| I-72 | 125 | 90 | 70 | 50 | 0 |
| I-73 | 125 | 98 | 90 | 20 | 0 |
| I-75 | 250 | 100 | 70 | 40 | 0 |
| I-76 | 250 | 100 | 100 | 60 | 10 |
| I-77 | 250 | 98 | 95 | 40 | 0 |
| I-78 | 250 | 100 | 70 | 40 | 20 |
| I-79 | 250 | 100 | 70 | 40 | 20 |
| I-80 | 250 | 98 | 90 | 20 | 20 |
| I-81 | 250 | 98 | 95 | 0 | 10 |
| I-82 | 250 | 98 | 98 | 0 | 30 |
| I-83 | 250 | 98 | 98 | 20 | 10 |
| I-84 | 250 | 98 | 98 | 20 | 0 |
| I-85 | 250 | 100 | 98 | 70 | 0 |
| I-87 | 250 | 100 | 90 | 70 | 0 |
| I-88 | 250 | 100 | 70 | 50 | 0 |
| I-89 | 250 | 100 | 95 | 40 | 0 |
| I-90 | 250 | 60 | 40 | 60 | 0 |
| I-91 | 250 | 90 | 20 | 20 | 0 |
| I-93 | 500 | 60 | 95 | 30 | 0 |
| I-94 | 250 | 100 | 98 | 90 | 0 |
| I-95 | 250 | 100 | 98 | 20 | 0 |
| I-96 | 250 | 100 | 100 | 0 | 30 |
| I-97 | 250 | 100 | 80 | 0 | 0 |
| I-98 | 250 | 100 | 80 | 40 | 10 |
| I-99 | 250 | 100 | 98 | 90 | 0 |
| I-100 | 250 | 100 | 95 | 30 | 10 |
| I-101 | 250 | 100 | 98 | 40 | 10 |
| I-102 | 250 | 98 | 100 | 60 | 10 |
| I-105 | 250 | 100 | 80 | - | 20 |
| I-108 | 250 | 98 | 100 | 60 | 10 |
| I-109 | 125 | 95 | 90 | 60 | 0 |
| I-110 | 250 | 98 | 90 | 20 | 0 |
| I-111 | 250 | 90 | 10 | - | 0 |
| I-113 | 250 | 95 | 30 | - | 0 |
| I-114 | 250 | 100 | 95 | 95 | 30 |
| I-115 | 500 | 70 | 40 | 20 | 20 |
| I-116 | 500 | 100 | 95 | 50 | 10 |
| I-117 | 250 | 95 | 98 | 0 | 0 |
| I-119 | 500 | 98 | 100 | - | 20 |
| I-122 | 500 | 98 | 100 | - | 10 |
| I-123 | 500 | 70 | 10 | - | 10 |
| I-124 | 250 | 100 | 70 | - | 0 |
| I-125 | 250 | 100 | 70 | - | 10 |
| I-127 | 250 | 100 | 70 | - | 0 |
| I-128 | 250 | 100 | 90 | - | 0 |
| I-129 | 250 | 100 | 90 | - | 0 |
| I-130 | 125 | 100 | 70 | - | 10 |
| I-131 | 250 | 100 | 95 | - | 10 |
| I-132 | 250 | 100 | 80 | - | 20 |
| I-133 | 250 | 100 | 95 | - | 10 |
| I-134 | 250 | 70 | 30 | - | 10 |
| I-135 | 500 | 98 | 100 | - | 10 |
| I-136 | 250 | 100 | 70 | - | 0 |
| I-137 | 250 | 100 | 98 | - | 0 |
| I-138 | 250 | 98 | 100 | - | 10 |
| I-139 | 250 | 98 | 98 | - | 10 |
| I-140 | 250 | 98 | 98 | - | 0 |
| I-141 | 250 | 100 | 98 | - | 20 |
| I-142 | 250 | 100 | 60 | - | 10 |
| I-143 | 250 | 98 | 98 | - | 10 |
| I-144 | 250 | 100 | 98 | - | 20 |
| I-145 | 250 | 100 | 0 | - | 20 |
| I-146 | 250 | 98 | 80 | 0 | 20 |
| I-163 | 250 | 100 | 99 | 90 | 60 |
| I-164 | 250 | 100 | 98 | 0 | 10 |
| I-165 | 250 | 98 | 98 | 60 | 10 |
| I-166 | 250 | 100 | 95 | 0 | 0 |
| I-169 | 250 | 100 | 50 | 0 | 10 |
| I-170 | 250 | 100 | 100 | 70 | 0 |
| I-171 | 250 | 60 | 98 | 0 | 0 |
| I-172 | 250 | 100 | 98 | 95 | 0 |
| I-173 | 250 | 90 | 95 | 30 | 0 |
| I-174 | 250 | 10 | 95 | 0 | 0 |
| I-176 | 250 | 98 | 98 | 0 | 0 |
| I-177 | 250 | 60 | 98 | 20 | 20 |
| I-178 | 500 | 98 | 95 | 40 | 0 |
| I-179 | 500 | 90 | 90 | 30 | 10 |
| I-180 | 500 | 98 | 100 | 60 | 30 |
| I-181 | 500 | 95 | 98 | 60 | 10 |
| I-182 | 500 | 100 | 98 | 80 | 0 |
| I-183 | 500 | 100 | 100 | 70 | 10 |
| I-184 | 500 | 70 | 90 | - | 10 |
| I-186 | 500 | 98 | 95 | - | 10 |
| I-187 | 500 | 100 | 10 | 20 | 0 |
| I-188 | 500 | 100 | 0 | 0 | 0 |
| I-189 | 500 | 100 | 98 | 95 | 95 |
| I-190 | 500 | 100 | 98 | 90 | 95 |
| I-191 | 500 | 100 | 90 | - | 20 |
| I-192 | 500 | 100 | 90 | - | 30 |
| I-193 | 250 | 100 | 95 | - | 30 |
| I-194 | 250 | 98 | 98 | 30 | 30 |
| I-195 | 250 | 70 | 100 | 80 | 20 |
| I-196 | 250 | 98 | 100 | 50 | 10 |
| I-197 | 250 | 100 | 98 | - | 0 |
| I-198 | 250 | 100 | 100 | - | 60 |
| I-199 | 250 | 100 | 98 | - | 40 |
| I-201 | 250 | 98 | 98 | - | 10 |
| I-202 | 250 | 98 | 98 | - | 10 |
| I-203 | 250 | 100 | 95 | - | 10 |
| I-204 | 250 | 100 | 98 | - | 60 |
| I-205 | 250 | 100 | 98 | - | 20 |
| I-206 | 250 | 100 | 98 | - | 10 |
| I-207 | 250 | 98 | 100 | - | 10 |
| I-208 | 250 | 98 | 98 | - | 70 |
| I-209 | 250 | 98 | 100 | - | 35 |
| I-211 | 250 | 100 | 98 | - | 50 |
| I-212 | 250 | 100 | 98 | 80 | 30 |
| I-229 | 500 | 100 | 95 | 90 | 0 |
| I-232 | 500 | 100 | 90 | 70 | 60 |
| I-233 | 500 | 95 | 90 | 40 | 10 |
| I-234 | 250 | 100 | 90 | 40 | 0 |
| I-235 | 250 | 100 | 90 | 60 | 0 |
| I-236 | 250 | 95 | 80 | 70 | 0 |
| I-237 | 250 | 95 | 95 | 40 | 0 |
| I-238 | 250 | 95 | 95 | 60 | 20 |
| I-239 | 250 | 98 | 98 | 70 | 0 |
| I-240 | 250 | 100 | 100 | 50 | 0 |
| I-241 | 250 | 100 | 95 | 80 | 0 |
| I-242 | 250 | 100 | 95 | 70 | 0 |
| I-243 | 250 | 100 | 98 | 70 | 0 |
| I-244 | 250 | 100 | 95 | 50 | 0 |
| I-245 | 250 | 100 | 95 | 70 | 10 |
| I-246 | 250 | 100 | 98 | 70 | 10 |
| I-247 | 250 | 100 | 95 | 0 | 0 |
| I-248 | 250 | 98 | 98 | 20 | 0 |
| I-249 | 250 | 98 | 98 | 0 | 0 |
| I-252 | 250 | 95 | 98 | 0 | 0 |
| I-253 | 250 | 90 | 20 | 0 | 10 |
| I-254 | 250 | 100 | 98 | - | 0 |
| I-255 | 250 | 100 | 95 | 20 | 0 |
| I-256 | 250 | 100 | 90 | 20 | 0 |
| I-258 | 250 | 100 | 98 | 70 | 30 |
| I-259 | 250 | 98 | 70 | 90 | 40 |
| I-260 | 250 | 100 | 98 | 60 | 10 |
| I-261 | 250 | 100 | 95 | 70 | 20 |
| I-262 | 250 | 98 | 95 | 30 | 0 |
| I-263 | 250 | 5 | 90 | 20 | 0 |

### Formulation Example 1

| | | |
|---|---|---|
| (1) | The compound of the present invention | 75 parts by weight |
| (2) | Geropon T-77 (tradename, manufactured by Rhone-Poulenc) | 14.5 parts by weight |
| (3) | NaCℓ | 10 parts by weight |
| (4) | Dextrin | 0.5 parts by weight |

The above components are placed in a high-speed mixing granulator, admixed with 20 wt% of water, granulated, and dried to form water-dispersible granules.

### Formulation Example 2

| | | |
|---|---|---|
| (1) | Kaolin | 78 parts by weight |
| (2) | Laveline FAN (tradename, manufactured by DAI-ICHI KOGYO SEIYAKU CO., LTD.) | 2 parts by weight |
| (3) | Sorpol 5039 (tradename, manufactured by TOHO Chemical Industry Co., Ltd.) | 5 parts by weight |
| (4) | Carplex (tradename, manufactured by Shionogi & Co., Ltd.) | 15 parts by weight |

The mixture of the above components (1) to (4) and the compound of the present invention are mixed in a weight ratio of 9:1 to obtain a wettable powder.

### Formulation Example 3

| | | |
|---|---|---|
| (1) | Hi-Filler No. 10 (tradename, manufactured by Matsumura Sangyo Co., Ltd.) | 33 parts by weight |
| (2) | Sorpol 5050 (tradename, manufactured by TOHO Chemical Industry Co., Ltd.) | 3 parts by weight |
| (3) | Sorpol 5073 (tradename, manufactured by TOHO Chemical Industry Co., Ltd.) | 4 parts by weight |
| (4) | The compound of the present invention | 60 parts by weight |

The above components (1) to (4) are mixed to obtain a wettable powder.

### Formulation Example 4

| | | |
|---|---|---|
| (1) | The compound of the present invention | 4 parts by weight |
| (2) | Bentonite | 30 parts by weight |
| (3) | Calcium carbonate | 61.5 parts by weight |
| (4) | Toxanon GR-31A (tradename, manufactured by Sanyo Chemical Industries Co., Ltd.) | 3 parts by weight |
| (5) | Calcium lignin sulfonate | 1.5 parts by weight |

Pulverized component (1) and components (2) and (3) are preliminarily mixed, and then components (4) and (5) and water are mixed thereto. The mixture is extruded and granulated, followed by drying and size-adjusting to obtain granules.

### Formulation Example 5

| | | |
|---|---|---|
| (1) | The compound of the present invention | 30 parts by weight |
| (2) | Zieclite (tradename, manufactured by Zieclite Co., Ltd.) | 60 parts by weight |
| (3) | New Kalgen WG-1 (tradename, manufactured by TAKEMOTO OIL & FAT CO., LTD.) | 5 parts by weight |
| (4) | New Kalgen FS-7 (tradename, manufactured by TAKEMOTO OIL & FAT CO., LTD.) | 5 parts by weight |

Components (1), (2) and (3) are mixed and passed through a pulverizer, and then component (4) is added thereto. The mixture is kneaded and then extruded and granulated, followed by drying and size-adjusting to obtain water-dispersible granules.

### Formulation Example 6

| | | |
|---|---|---|
| (1) | The compound of the present invention | 28 parts by weight |
| (2) | Soprophor FL (tradename, manufactured by Rhone-Poulenc) | 2 parts by weight |
| (3) | Sorpol 355 (tradename, manufactured by TOHO Chemical Industry Co., Ltd.) | 1 parts by weight |
| (4) | IP solvent 1620 (tradename, manufactured by Idemitsu Petrochemical Co., Ltd.) | 32 parts by weight |
| (5) | Ethylene glycol | 6 parts by weight |
| (6) | Water | 31 parts by weight |

The above components (1) to (6) are mixed and pulverized by a wet-grinding machine (Dyno-mill) to obtain a water-based suspension concentrate.

While the invention has been described in detail and with reference to specific embodiments thereof, it will be apparent to one skill in the art that various changes and modifications can be made therein without departing from the spirit and scope thereof. All references cited herein are incorporated in their entirety.

This application is based on Japanese application No. 2002-125603 filed on April 26, 2002, the entire contents of which being incorporated hereinto by reference.

### INDUSTRIAL APPLICABILITY

The present invention provides novel pyridine compounds which are useful as active ingredients of herbicides.

## Claims

1. A pyridine compound represented by formula (I): 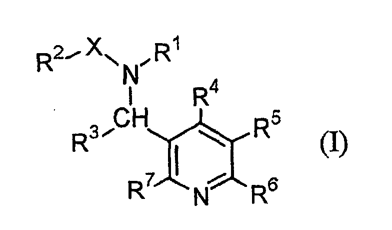 wherein R¹ represents hydrogen or alkyl which may be substituted; R² represents alkyl which may be substituted, alkenyl which may be substituted, alkynyl which may be substituted, cycloalkyl which may be substituted, cycloalkenyl which may be substituted, alkoxy which may be substituted, alkylthio which may be substituted, mono- or di-alkylamino which may be substituted, phenylamino which may be substituted, cyclic alkylamino which may be substituted, aryl which may be substituted, or a cyclic ether group which may be substituted; R³ represents alkyl which may be substituted, cycloalkyl which may be substituted, aryl which may be substituted, or heteroaryl which may be substituted; R⁴ represents hydrogen, alkyl, haloalkyl, halogen, -OR⁸, or -SR⁸; R⁵, R⁶ and R⁷ each represents hydrogen, halogen, or alkyl; R⁸ represents alkyl which may be substituted, alkenyl which may be substituted, alkynyl which may be substituted, or cycloalkyl which may be substituted; and X represents CO, CS, or SO₂, and wherein R⁴ is not chlorodifluoromethyl, bromodifluoromethyl, nor iododifluoromethyl, or
a salt thereof.

2. The pyridine compound according to claim 1, wherein the substituent for the alkyl which may be substituted, the alkenyl which may be substituted, the alkynyl which may be substituted, the cycloalkyl which may be substituted, the cycloalkenyl which may be substituted, the alkoxy which may be substituted, the alkylthio which may be substituted, the mono- or di-alkylamino which may be substituted, the cyclic alkylamino which may be substituted, or the cyclic ether group which may be substituted, which are contained in R¹, R², R³ or R⁸, is at least one selected from the group consisting of halogen, alkyl, alkoxy, alkylthio, dialkylamino, trimethylsiliyl, cycloalkyl, cycloalkenyl, alkylcarbonyl, alkoxycarbonyl, cyclic ether, aryl which may be further substituted, aryloxy which may be further substituted, arylthio which may be further substituted, and heteroaryl which may be further substituted; and wherein the substituent for the phenylamino which may be substituted, the aryl which may be substituted, or the heteroaryl which may be substituted, which are contained in R² or R³, is selected from the group consisting of halogen, alkyl, haloalkyl, alkoxy, alkylthio, alkoxycarbonyl, nitro, cyano, trimethylsilyl, alkoxyimino, and phenyl which may be further substituted, or
a salt thereof.

3. The pyridine compound according to claim 1, wherein the substituent for the alkyl which may be substituted, the alkenyl which may be substituted, the alkynyl which may be substituted, the cycloalkyl which may be substituted, the cycloalkenyl which may be substituted, the alkoxy which may be substituted, the alkylthio which may be substituted, the mono- or di-alkylamino which may be substituted, the cyclic alkylamino which may be substituted, or the cyclic ether group which may be substituted, which are contained in R¹, R², R³ or R⁸ is at least one selected from the group consisting of halogen; alkyl, alkoxy, alkylthio, dialkylamino, trimethylsiliyl, cycloalkyl, cycloalkenyl, alkylcarbonyl, alkoxycarbonyl, cyclic ether, aryl which may be further substituted, aryloxy which may be further substituted, arylthio which may be further substituted, and heteroaryl which may be further substituted, wherein the substituent for the aryl which may be further substituted, the aryloxy which may be further substituted, the arylthio which may be further substituted, or the heteroaryl which may be further substituted is selected from the group consisting of halogen, alkyl, haloalkyl, alkoxy, alkylthio, alkoxycarbonyl, nitro, cyano, trimethylsilyl, alkoxyimino, and phenyl; and the substituent for the phenylamino which may be substituted, the aryl which may be substituted, or the heteroaryl which may be substituted, which are contained in R² or R³ is selected from the group consisting of halogen, alkyl, haloalkyl, alkoxy, alkylthio, alkoxycarbonyl, nitro, cyano, trimethylsilyl, alkoxyimino, and phenyl which may be further substituted, wherein the substituent for the phenyl may be further substituted is selected from the group consisting of halogen, alkyl, haloalkyl, alkoxy, alkylthio, alkoxycarbonyl, nitro, cyano, trimethylsilyl, alkoxyimino, and phenyl, or
a salt thereof.

4. The pyridine compound according to claim 1, wherein the cyclic alkylamino is aziridinyl, azetidinyl, pyrrolidinyl, or piperidinyl; the aryl is phenyl, naphthyl, or indanyl; the cyclic ether is epoxy, tetrahydrofuryl, 1,3-dioxolanyl, or 1,3-dioxanyl; and the heteroaryl is a 5-membered or 6-membered aryl containing 1 to 3 heteroatoms selected from oxygen, sulfur and nitrogen, or the aryl condensed with a benzene ring, or
a salt thereof.

5. The pyridine compound according to claim 1, wherein R⁴ is hydrogen, alkyl, trifluoromethyl, halogen, -OR⁸ or -SR⁸, or
a salt thereof.

6. A pyridine compound represented by formula (Ia): 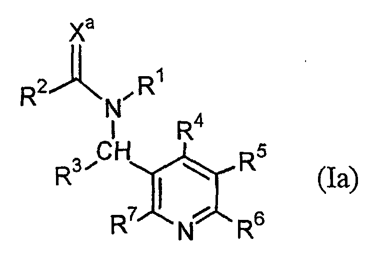 wherein R¹ represents hydrogen or alkyl which may be substituted; R² represents alkyl which may be substituted, alkenyl which may be substituted, alkynyl which may be substituted, cycloalkyl which may be substituted, alkoxy which may be substituted, alkylthio which may be substituted, mono- or di-alkylamino which may be substituted, phenylamino which may be substituted, or cyclic alkylamino which may be substituted; R³ represents alkyl which may be substituted, cycloalkyl which may be substituted, aryl which may be substituted, or heteroaryl which may be substituted; R⁴ represents alkyl, haloalkyl, -OR⁸, or -SR⁸; R⁵, R⁶ and R⁷ each represents hydrogen, halogen, or alkyl; R⁸ represents alkyl which may be substituted, alkenyl which may be substituted, alkynyl which may be substituted, or cycloalkyl which may be substituted; and X^{a} represents oxygen or sulfur, and wherein R⁴ is not chlorodifluoromethyl, bromodifluoromethyl, nor iododifluoromethyl, or
a salt thereof

7. A process for producing a pyridine compound represented by formula (I): 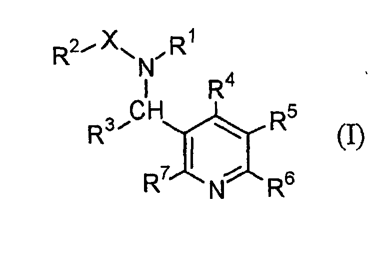 wherein R¹ represents hydrogen or alkyl which may be substituted; R² represents alkyl which may be substituted, alkenyl which may be substituted, alkynyl which may be substituted, cycloalkyl which may be substituted, cycloalkenyl which may be substituted, alkoxy which may be substituted, alkylthio which may be substituted, mono- or di-alkylamino which may be substituted, phenylamino which may be substituted, cyclic alkylamino which may be substituted, aryl which may be substituted, or a cyclic ether group which may be substituted; R³ represents alkyl which may be substituted, cycloalkyl which may be substituted, aryl which may be substituted, or heteroaryl which may be substituted; R⁴ represents hydrogen, alkyl, haloalkyl, halogen, -OR⁸ or -SR⁸; R⁵, R⁶ and R⁷ each represents hydrogen, halogen, or alkyl; R⁸ represents alkyl which may be substituted, alkenyl which may be substituted, alkynyl which may be substituted, or cycloalkyl which may be substituted; and X represents CO, CS, or SO₂, and wherein R⁴ is not chlorodifluoromethyl, bromodifluoromethyl, nor iododifluoromethyl, or a salt thereof,
which comprises:
(1) reacting an amine derivative represented by formula (II): 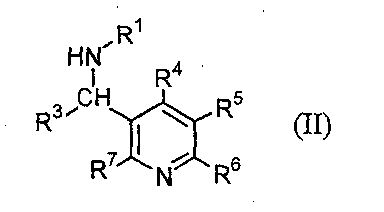 wherein R¹, R³, R⁴, R⁵, R⁶ and R⁷ have the same meanings as described above,
with a carboxylic acid derivative represented by formula (III-1):
R²-COY¹ (III-1)
wherein R² has the same meaning as described above; and Y¹ represents OH, chlorine, or R²COO,
(2) reacting the amine derivative represented by formula (II) described above with a sulfonic acid derivative represented by formula (III-2):
R²-SO₂Y² (III-2)
wherein R² has the same meaning as described above; and Y² represents chlorine or R²SO₂O,
(3) reacting a pyridine compound represented by formula (I-1): 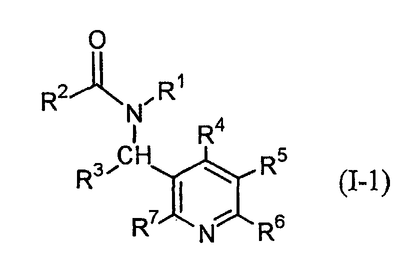 wherein R¹, R², R³, R⁴, R⁵, R⁶ and R⁷ have the same meanings as described above,
with a sulfurizing agent,
(4) reacting an amine derivative represented by formula (II-1): 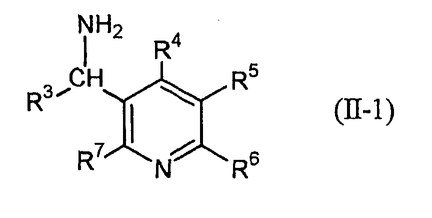 wherein R³, R⁴, R⁵, R⁶ and R⁷ have the same meanings as described above,
with a compound represented by the formula:
R²-X-Y¹
wherein Y¹ represents OH, chlorine, or bromine; and R² and X have the same meanings as described above,
(5) reacting a pyridine compound represented by formula (I-5): 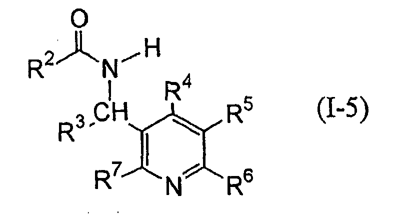 wherein R², R³, R⁴, R⁵, R⁶ and R⁷ have the same meanings as described above,
with a compound represented by the formula:
R^{1a}-Y⁴
wherein R^{1a} represents alkyl which may be substituted; Y⁴ represents chlorine, bromine, iodine, -OSO₂R⁹, or -OSO₃R⁹; and R⁹ represents C₁-C₆ alkyl or phenyl which may be substituted with C₁-C₆ alkyl, or
(6) reacting a pyridine compound represented by formula (I-6): 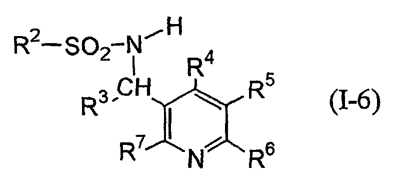 wherein R², R³, R⁴, R⁵, R⁶ and R⁷ have the same meanings as described above,
with a compound represented by the formula:
R^{1a}-Y⁴
wherein R^{1a} and Y⁴ have the same meanings as described above.

8. A herbicide which comprises, as an active ingredient, the pyridine compound according to any one of claims 1 to 6 or a salt thereof

9. A method for controlling a noxious weed, which comprises applying an effective amount of the pyridine compound according to any one of claims 1 to 6 or a salt thereof.

10. A mixed herbicide composition, which comprises at least one of the pyridine compound according to any one of claims 1 to 6 or a salt thereof, and at least one other herbicide(s).
